# EUROPEAN PATENT APPLICATION

(11) **EP 4 206 179 A1**
(43) Date of publication of application: **05.07.2023**
(21) Application number: 21858428.2
(22) Date of filing: 19.02.2021
(51) Int. Cl.: C07C 211/54, C07C 211/57, C07D 307/91, C07D 333/76, H01L 51/00, H01L 51/50

(54) **HETEROCYCLIC COMPOUND AND ORGANIC LIGHT-EMITTING DEVICE COMPRISING SAME**

(30) Priority: 21.08.2020 KR 20200105342
(71) Applicant: LT Materials Co., Ltd., Yongin-si, Gyeonggi-do 17118 (KR)
(72) Inventor: LEE, Gi-Back, Yongin-si, Gyeonggi-do 17118 (KR); LEE, Nam-Jin, Yongin-si, Gyeonggi-do 17118 (KR); JEONG, Won-Jang, Yongin-si, Gyeonggi-do 17118 (KR); KIM, Dong-Jun, Yongin-si, Gyeonggi-do 17118 (KR); HAM, Hyo-Kyun, Yongin-si, Gyeonggi-do 17118 (KR)
(74) Representative: Goddar, Heinz J.
(86) International application number: PCT/KR2021/002104
(87) International publication number: WO 2022/039340

(57) **Abstract**

The present specification relates to a heterocyclic compound represented by Chemical Formula 1 and an organic light emitting device including the same.

## Description

### [Technical Field]

This application claims priority to and the benefit of Korean Patent Application No. 10-2020-0105342 filed in the Korean Intellectual Property Office on August 21, 2020, the entire contents of which are incorporated herein by reference.

The present specification relates to a heterocyclic compound and an organic light emitting device including the same.

### [Background Art]

An organic electroluminescence device is a kind of self-emitting type display device, and has an advantage in that the viewing angle is wide, the contrast is excellent, and the response speed is fast.

An organic light emitting device has a structure in which an organic thin film is disposed between two electrodes. When a voltage is applied to an organic light emitting device having the structure, electrons and holes injected from the two electrodes combine with each other in an organic thin film to make a pair, and then, emit light while being extinguished. The organic thin film may be composed of a single layer or multiple layers, if necessary.

A material for the organic thin film may have a light emitting function, if necessary. For example, as the material for the organic thin film, it is also possible to use a compound, which may itself constitute a light emitting layer alone, or it is also possible to use a compound, which may serve as a host or a dopant of a host-dopant-based light emitting layer. In addition, as a material for the organic thin film, it is also possible to use a compound, which may perform a function such as hole injection, hole transport, electron blocking, hole blocking, electron transport or electron injection.

In order to improve the performance, service life, or efficiency of the organic light emitting device, there is a continuous need for developing a material for an organic thin film.

It is necessary to perform studies on an organic light emitting device including a compound having a chemical structure, which may satisfy conditions required for a material which is available for the organic light emitting device, for example, appropriate energy levels, electrochemical stability, thermal stability, and the like, and may perform various functions required for the organic light emitting device according to the substituent.

### <Related Art Document>

US Patent No. 4,356,429

### [Detailed Description of the Invention]

### [Technical Problem]

The present application relates to a heterocyclic compound and an organic light emitting device including the same.

### [Technical Solution]

In an exemplary embodiment of the present application, provided is a heterocyclic compound represented by the following Chemical Formula 1. 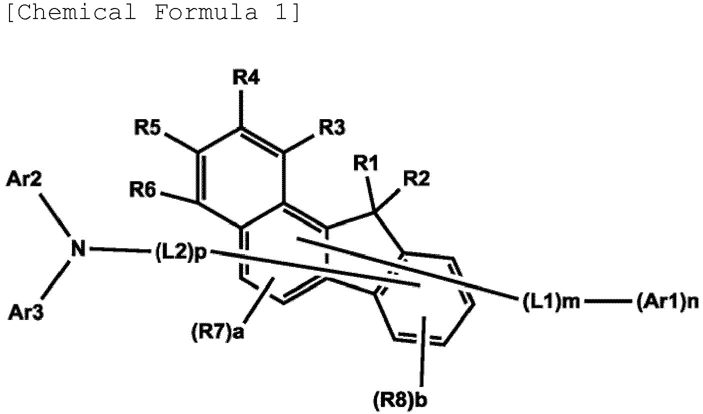 In Chemical Formula 1,
R1 and R2 are the same as or different from each other, and are each independently a substituted or unsubstituted C1 to C60 alkyl group; a substituted or unsubstituted C6 to C60 aryl group; or a substituted or unsubstituted C2 to C60 heteroaryl group,
R3 to R8 are the same as or different from each other, and are each independently selected from the group consisting of hydrogen; deuterium; a halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)RR'; -SiRR'R" and -NRR', or two or more adjacent groups are bonded to each other to form a substituted or unsubstituted C6 to C60 aliphatic or aromatic hydrocarbon ring, or a substituted or unsubstituted C2 to C60 aliphatic or aromatic hetero ring,
L1 and L2 are the same as or different from each other, and are each independently a direct bond; a substituted or unsubstituted C6 to C60 arylene group; or a substituted or unsubstituted C2 to C60 heteroarylene group,
Ar1 to Ar3 are the same as or different from each other, and are each independently a substituted or unsubstituted C1 to C60 alkyl group; a substituted or unsubstituted C6 to C60 aryl group; or a substituted or unsubstituted C2 to C60 heteroaryl group,
p and m are an integer from 0 to 4,
n is an integer from 1 to 6,
a is an integer of 0 or 1,
b is an integer from 0 to 2, and
R, R' and R" are the same as or different from each other, and are each independently a substituted or unsubstituted C1 to C60 alkyl group; a substituted or unsubstituted C6 to C60 aryl group; or a substituted or unsubstituted C2 to C60 heteroaryl group.

Further, according to an exemplary embodiment of the present application, provided is an organic light emitting device including: a first electrode; a second electrode provided to face the first electrode; and an organic material layer having one or more layers provided between the first electrode and the second electrode, in which one or more layers of the organic material layer include the heterocyclic compound represented by Chemical Formula 1.

### [Advantageous Effects]

The compound described in the present specification can be used as a material for the organic material layer of the organic light emitting device. The compound can serve as a hole injection material, a hole transport material, a light emitting material, an electron transport material, an electron injection material, an electron blocking material, a hole blocking material, and the like in an organic light emitting device. In particular, the compound can be used as a hole transport material or electron blocking material for an organic light emitting device.

In particular, the heterocyclic compound according to the present application has an amine-based substituent and a substituent of -(L1)m-(Ar1)n in benzofluorene which is a core structure. When an amine derivative is used as a hole transport layer, the unshared electron pair of the amine can improve the flow of holes and improve the hole transfer capacity of the hole transport layer, and when the amine derivative is used as an electron blocking layer, deterioration of the hole transport material caused by electrons penetrating into the hole transport layer can be suppressed, and in addition, by bonding a substituent whose hole characteristics are enhanced, the band gap and T1 (energy level value of the triplet state) values are adjusted to improve the ability to transport holes, and the heterocyclic compound of the present application has characteristics capable of improving the thermal stability of the compound by increasing the planarity and glass transition temperature of the amine derivative. When the heterocyclic compound represented by Chemical Formula 1 is used as a material for the hole transport layer or the electron blocking layer of the organic light emitting device, the driving voltage of the device can be lowered, the light efficiency can be improved, and the service life characteristics of the device can be improved.

### [Brief Description of Drawings]

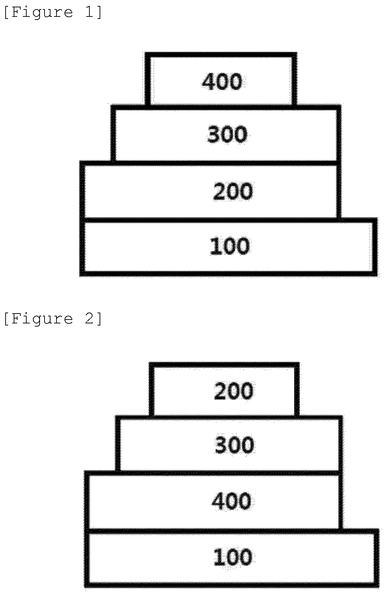

FIGS. 1 to 3 each are views schematically illustrating a stacking structure of an organic light emitting device according to an exemplary embodiment of the present application.

### <Explanation of Reference Numerals and Symbols>

- 100:: Substrate
- 200:: Positive electrode
- 300:: Organic material layer
- 301:: Hole injection layer
- 302:: Hole transport layer
- 303:: Light emitting layer
- 304:: Hole blocking layer
- 305:: Electron transport layer
- 306:: Electron injection layer
- 400:: Negative electrode

### [Best Mode]

Hereinafter, the present application will be described in detail.

In the present specification, the halogen may be fluorine, chlorine, bromine or iodine.

In the present specification, the alkyl group includes a straight-chain or branched-chain having 1 to 60 carbon atoms, and may be additionally substituted with another substituent. The number of carbon atoms of the alkyl group may be 1 to 60, specifically 1 to 40, and more specifically 1 to 20. Specific examples thereof include a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethyl-butyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, a cyclopentylmethyl group, a cyclohexylmethyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 2-methylpentyl group, a 4-methylhexyl group, a 5-methylhexyl group, and the like, but are not limited thereto.

In the present specification, the alkenyl group includes a straight-chain or branched-chain having 2 to 60 carbon atoms, and may be additionally substituted with another substituent. The number of carbon atoms of the alkenyl group may be 2 to 60, specifically 2 to 40, and more specifically 2 to 20. Specific examples thereof include a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group, and the like, but are not limited thereto.

In the present specification, the alkynyl group includes a straight-chain or branched-chain having 2 to 60 carbon atoms, and may be additionally substituted with another substituent. The number of carbon atoms of the alkynyl group may be 2 to 60, specifically 2 to 40, and more specifically 2 to 20.

In the present specification, an alkoxy group may be straight-chained, branched, or cyclic. The number of carbon atoms of the alkoxy group is not particularly limited, but is preferably 1 to 20. Specific examples thereof include methoxy, ethoxy, n-propoxy, isopropoxy, i-propyloxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy, and the like, but are not limited thereto.

In the present specification, the cycloalkyl group includes a monocycle or polycycle having 3 to 60 carbon atoms, and may be additionally substituted with another substituent. Here, the polycycle means a group in which a cycloalkyl group is directly linked to or fused with another cyclic group. Here, another cyclic group may also be a cycloalkyl group, but may also be another kind of cyclic group, for example, a heterocycloalkyl group, an aryl group, a heteroaryl group, and the like. The number of carbon atoms of the cycloalkyl group may be 3 to 60, specifically 3 to 40, and more specifically 5 to 20. Specific examples thereof include a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group, and the like, but are not limited thereto.

In the present specification, the heterocycloalkyl group includes O, S, Se, N, or Si as a heteroatom, includes a monocycle or polycycle having 2 to 60 carbon atoms, and may be additionally substituted with another substituent. Here, the polycycle means a group in which a heterocycloalkyl group is directly linked to or fused with another cyclic group. Here, another cyclic group may also be a heterocycloalkyl group, but may also be another kind of cyclic group, for example, a cycloalkyl group, an aryl group, a heteroaryl group, and the like. The number of carbon atoms of the heterocycloalkyl group may be 2 to 60, specifically 2 to 40, and more specifically 3 to 20.

In the present specification, the aryl group includes a monocycle or polycycle having 6 to 60 carbon atoms, and may be additionally substituted with another substituent. Here, the polycycle means a group in which an aryl group is directly linked to or fused with another cyclic group. Here, another cyclic group may also be an aryl group, but may also be another kind of cyclic group, for example, a cycloalkyl group, a heterocycloalkyl group, a heteroaryl group, and the like. The number of carbon atoms of the aryl group may be 6 to 60, specifically 6 to 40, and more specifically 6 to 25. Specific examples of the aryl group include a phenyl group, a biphenyl group, a triphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, an indenyl group, an acenaphthylenyl group, a 2,3-dihydro-1H-indenyl group, a fused cyclic group thereof, and the like, but are not limited thereto.

In the present specification, a fluorenyl group may be substituted, and adjacent substituents may be bonded to each other to form a ring.

When the fluorenyl group is substituted, the substituent may be represented by the following structures, but is not limited thereto.

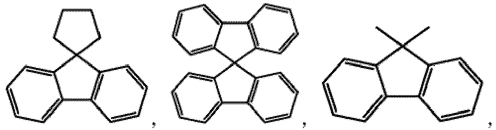

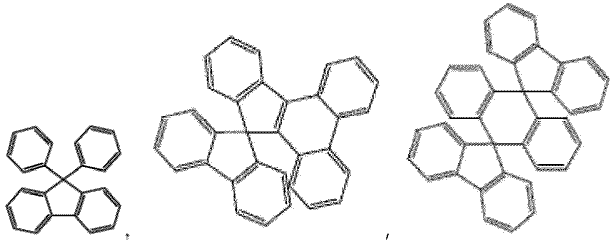

In the present specification, the heteroaryl group includes S, O, Se, N, or Si as a heteroatom, includes a monocycle or polycycle having 2 to 60 carbon atoms, and may be additionally substituted with another substituent. Here, the polycycle means a group in which a heteroaryl group is directly linked to or fused with another cyclic group.

Here, another cyclic group may also be a heteroaryl group, but may also be another kind of cyclic group, for example, a cycloalkyl group, a heterocycloalkyl group, an aryl group, and the like. The number of carbon atoms of the heteroaryl group may be 2 to 60, specifically 2 to 40, and more specifically 3 to 25. Specific examples of the heteroaryl group include a pyridyl group, a pyrrolyl group, a pyrimidyl group, a pyridazinyl group, a furanyl group, a thiophene group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a dioxynyl group, a triazinyl group, a tetrazinyl group, a quinolyl group, an isoquinolyl group, a quinazolinyl group, an isoquinazolinyl group, a quinozolilyl group, a naphthyridyl group, an acridinyl group, a phenanthridinyl group, an imidazopyridinyl group, a diaza naphthalenyl group, a triazaindene group, an indolyl group, an indolizinyl group, a benzothiazolyl group, a benzoxazolyl group, a benzimidazolyl group, a benzothiophene group, a benzofuran group, a dibenzothiophene group, a dibenzofuran group, a carbazolyl group, a benzocarbazolyl group, a dibenzocarbazolyl group, a phenazinyl group, a dibenzosilole group, spirobi (dibenzosilole), a dihydrophenazinyl group, a phenoxazinyl group, a phenanthridyl group, an imidazopyridinyl group, a thienyl group, an indolo[2,3-a]carbazolyl group, an indolo[2,3-b]carbazolyl group, an indolinyl group, a 10,11-dihydro-dibenzo[b,f]azepin group, a 9,10-dihydroacridinyl group, a phenanthrazinyl group, a phenothiathiazinyl group, a phthalazinyl group, a naphthylidinyl group, a phenanthrolinyl group, a benzo[c][1,2,5]thiadiazolyl group, a 5,10-dihydrodibenzo[b,e][1,4]azasilinyl, a pyrazolo[1,5-c]quinazolinyl group, a pyrido[1,2-b]indazolyl group, a pyrido[1,2-a]imidazo[1,2-e]indolinyl group, a 5,11-dihydroindeno[1,2-b]carbazolyl group, and the like, but are not limited thereto.

In the present specification, the amine group may be selected from the group consisting of a monoalkylamine group; a monoarylamine group; a monoheteroarylamine group; -NH₂; a dialkylamine group; a diarylamine group; a diheteroarylamine group; an alkylarylamine group; an alkylheteroarylamine group; and an arylheteroarylamine group, and the number of carbon atoms thereof is not particularly limited, but is preferably 1 to 30. Specific examples of the amine group include a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group, and the like, but are not limited thereto.

In the present specification, an arylene group means a group having two bonding positions in an aryl group, that is, a divalent group. The above-described description on the aryl group may be applied to the arylene group, except for a divalent group. Further, a heteroarylene group means that there are two bonding positions in a heteroaryl group, that is, a divalent group. The above-described description on the heteroaryl group may be applied to the heteroarylene group, except for a divalent heteroarylene group.

In the present specification, a phosphine oxide group is represented by -P(=O)R101R102, and R101 and R102 are the same as or different from each other, and may be each independently a substituent composed of at least one of hydrogen; deuterium; a halogen group; an alkyl group; an alkenyl group; an alkoxy group; a cycloalkyl group; an aryl group; and a heterocyclic group. Specific examples of the phosphine oxide group include a diphenylphosphine oxide group, dinaphthylphosphine oxide, and the like, but are not limited thereto.

In the present specification, a silyl group includes Si and is a substituent to which the Si atom is directly linked as a radical, and is represented by -SiR104R105R106, and R104 to R106 are the same as or different from each other, and may be each independently a substituent composed of at least one of hydrogen; deuterium; a halogen group; an alkyl group; an alkenyl group; an alkoxy group; a cycloalkyl group; an aryl group; and a heterocyclic group. Specific examples of the silyl group include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group, and the like, but are not limited thereto.

In the present specification, the "adjacent" group may mean a substituent substituted with an atom directly linked to an atom in which the corresponding substituent is substituted, a substituent disposed to be sterically closest to the corresponding substituent, or another substituent substituted with an atom in which the corresponding substituent is substituted. For example, two substituents substituted at the ortho position in a benzene ring and two substituents substituted with the same carbon in an aliphatic ring may be interpreted as groups which are "adjacent" to each other.

Structures exemplified by the above-described cycloalkyl group, cycloheteroalkyl group, aryl group and heteroaryl group may be applied, except that an aliphatic or aromatic hydrocarbon ring or hetero ring which adjacent groups may form is not a monovalent group.

In the present specification, the term "substitution" means that a hydrogen atom bonded to a carbon atom of a compound is changed into another substituent, and a position to be substituted is not limited as long as the position is a position at which the hydrogen atom is substituted, that is, a position at which the substituent may be substituted, and when two or more substituents are substituted, the two or more substituents may be the same as or different from each other.

In the present specification, "substituted or unsubstituted" means being unsubstituted or substituted with one or more substituents selected from the group consisting of a C1 to C60 straight-chained or branched alkyl; a C2 to C60 straight-chained or branched alkenyl; a C2 to C60 straight-chained or branched alkynyl; a C3 to C60 monocyclic or polycyclic cycloalkyl; a C2 to C60 monocyclic or polycyclic heterocycloalkyl; a C6 to C60 monocyclic or polycyclic aryl; a C2 to C60 monocyclic or polycyclic heteroaryl; -SiRR'R"; -P(=O)RR'; a C1 to C20 alkylamine; a C6 to C60 monocyclic or polycyclic arylamine; and a C2 to C60 monocyclic or polycyclic heteroarylamine, or being unsubstituted or substituted with a substituent to which two or more substituents selected among the exemplified substituents are linked, and

R, R' and R" are the same as or different from each other, and are each independently a substituted or unsubstituted C1 to C60 alkyl group; a substituted or unsubstituted C6 to C60 aryl group; or a substituted or unsubstituted C2 to C60 heteroaryl group.

In the present specification, "when a substituent is not indicated in the structure of a chemical formula or compound" means that a hydrogen atom is bonded to a carbon atom. However, since deuterium (²H) is an isotope of hydrogen, some hydrogen atoms may be deuterium.

In an exemplary embodiment of the present application, "when a substituent is not indicated in the structure of a chemical formula or compound" may mean that all the positions that may be reached by the substituent are hydrogen or deuterium. That is, deuterium is an isotope of hydrogen, and some hydrogen atoms may be deuterium which is an isotope, and in this case, the content of deuterium may be 0% to 100%.

In an exemplary embodiment of the present application, in "the case where a substituent is not indicated in the structure of a chemical formula or compound", when the content of deuterium is 0%, the content of hydrogen is 100%, and all the substituents do not explicitly exclude deuterium such as hydrogen, hydrogen and deuterium may be mixed and used in the compound.

In an exemplary embodiment of the present application, deuterium is one of the isotopes of hydrogen, is an element that has a deuteron composed of one proton and one neutron as a nucleus, and may be represented by hydrogen-2, and the element symbol may also be expressed as D or 2H.

In an exemplary embodiment of the present application, the isotope means an atom with the same atomic number (Z), but different mass numbers (A), and the isotope may also be interpreted as an element which has the same number of protons, but different number of neutrons.

In an exemplary embodiment of the present application, when the total number of substituents of a basic compound is defined as T1 and the number of specific substituents among the substituents is defined as T2, the content T% of the specific substituent may be defined as T2/T1×100 = T%.

That is, in an example, the deuterium content of 20% in a phenyl group represented by 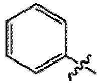 may be represented by 20% when the total number of substituents that the phenyl group can have is 5 (T1 in the formula) and the number of deuteriums among the substituents is 1 (T2 in the formula). That is, a deuterium content of 20% in the phenyl group may be represented by the following structural formula.

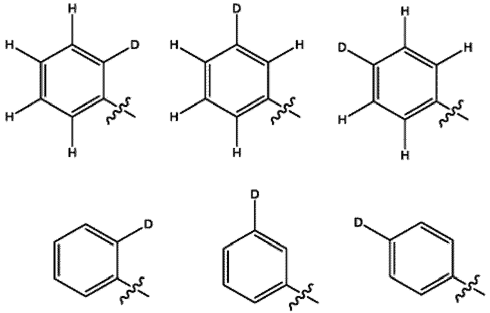

Further, in an exemplary embodiment of the present application, "a phenyl group having a deuterium content of 0%" may mean a phenyl group that does not include a deuterium atom, that is, has five hydrogen atoms.

In an exemplary embodiment of the present application, provided is the compound represented by Chemical Formula 1.

The heterocyclic compound represented by Chemical Formula 1 of the present invention has an amine-based substituent and a -(L1)m-(Ar1)n substituent having hole characteristics, and when the heterocyclic compound is used as a hole transport layer of an organic light emitting device later, the unshared electron pair of the amine substituent may improve the flow of holes to improve the hole transfer capacity of the hole transport layer, and when the heterocyclic compound is used as an electron blocking layer, deterioration of the hole transport material caused by electrons penetrating into the hole transport layer may be suppressed.

In addition, the -(L1)m-(Ar1)n substituent in whose hole characteristics are enhanced is bonded to increase the planarity and glass transition temperature of the amine derivative, thereby improving the thermal stability of the heterocyclic compound. Therefore, the heterocyclic compound also has a feature of improving the service life of the organic light emitting device including the heterocyclic compound.

Further, since the bandgap and T1 values are adjusted to improve the hole transfer capacity and improve the stability of the molecule, the driving voltage of the device may be lowered, the light efficiency of the device may be improved, and service life characteristics of the device may be improved by the thermal stability of the compound.

In an exemplary embodiment of the present application, Chemical Formula 1 may be represented by the following Chemical Formula 2 or 3. 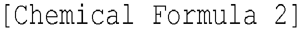 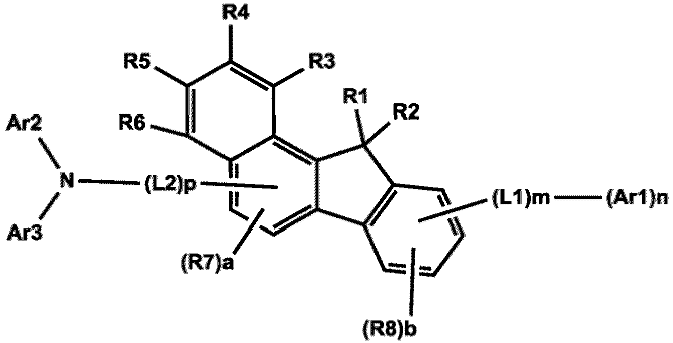 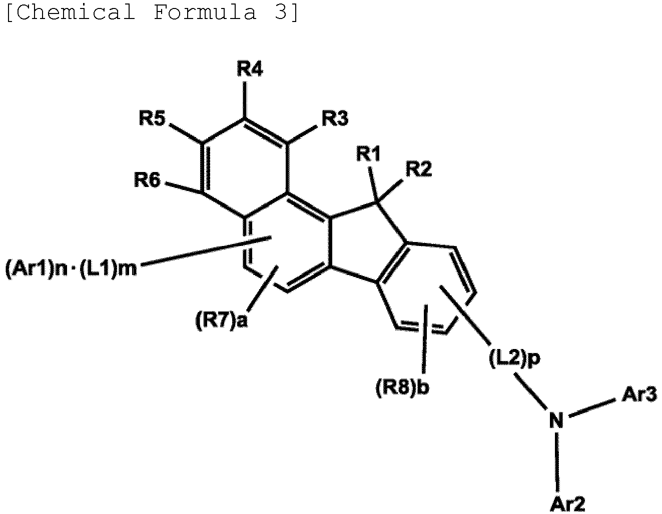 In Chemical Formulae 2 and 3,
the definitions of R1 to R8, L1, L2, Ar1 to Ar3, p, m, n, a and b are the same as the definitions in Chemical Formula 1.

In an exemplary embodiment of the present application, Chemical Formula 2 may be represented by the following Chemical Formula 2-1 or Chemical Formula 2-2. 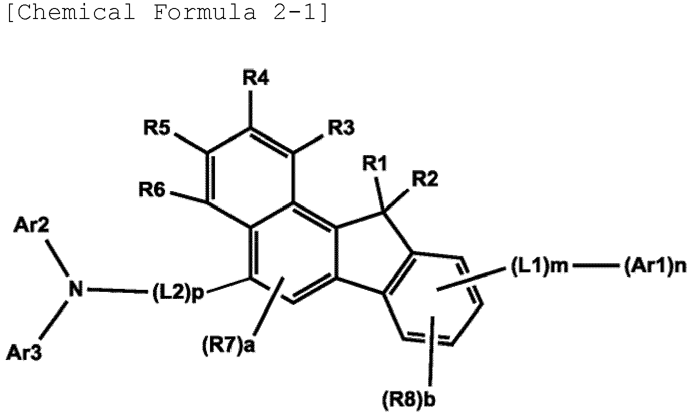 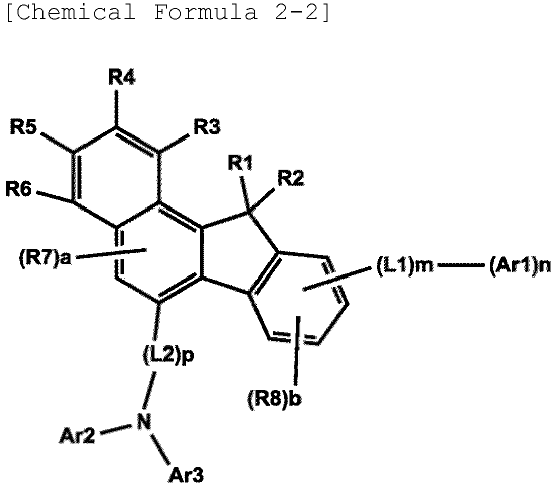 In Chemical Formulae 2-1 and 2-2,
the definitions of R1 to R8, L1, L2, Ar1 to Ar3, p, m, n, a and b are the same as the definitions in Chemical Formula 2.

In an exemplary embodiment of the present application, Chemical Formula 3 may be represented by the following Chemical Formula 3-1 or Chemical Formula 3-2. In Chemical Formulae 3-1 and 3-2, the definitions of R1 to R8, L1, L2, Ar1 to Ar3, p, m, n, a and b are the same as the definitions in Chemical Formula 3.

In an exemplary embodiment of the present application, Chemical Formula 2-1 may be represented by any one of the following Chemical Formulae 2-1-1 to 2-1-4. 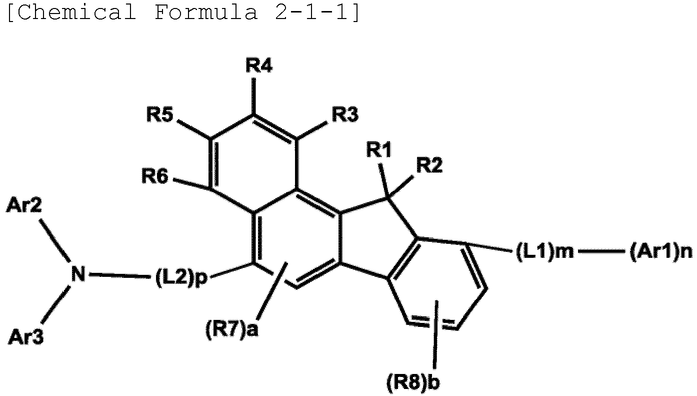 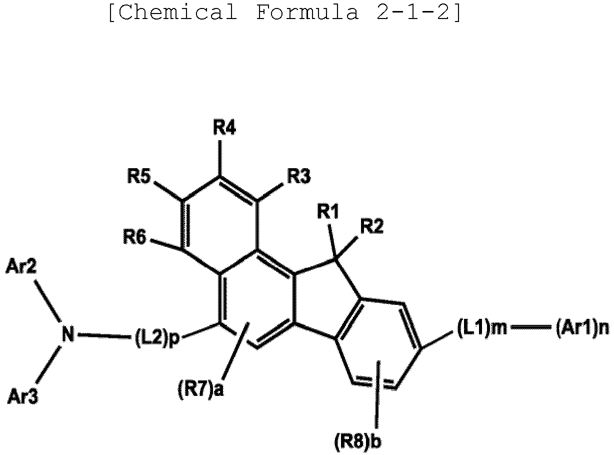 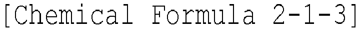 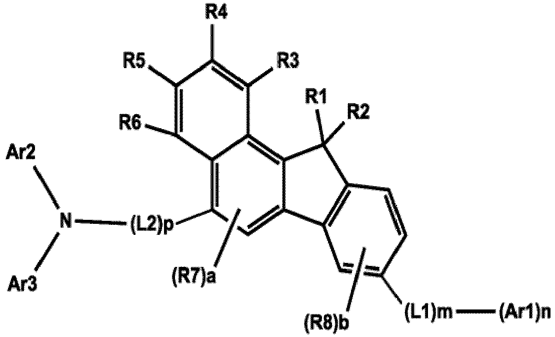 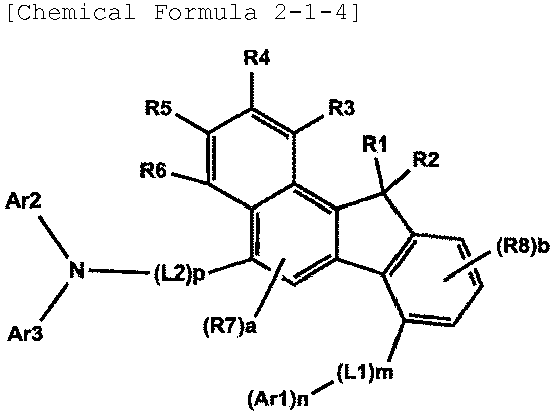 In Chemical Formulae 2-1-1 to 2-1-4,
the definitions of R1 to R8, L1, L2, Ar1 to Ar3, p, m, n, a and b are the same as the definitions in Chemical Formula 2-1.

In an exemplary embodiment of the present application, Chemical Formula 2-2 may be represented by any one of the following Chemical Formulae 2-2-1 to 2-2-4. 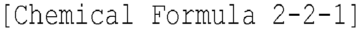 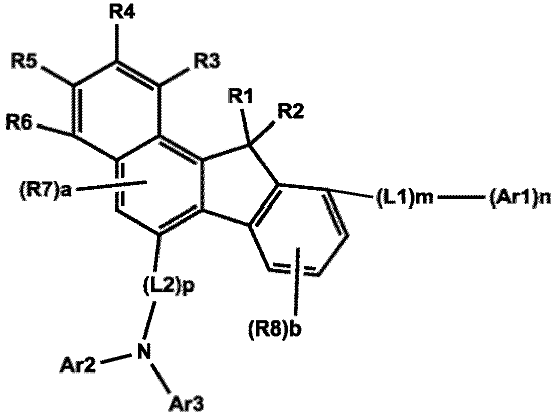 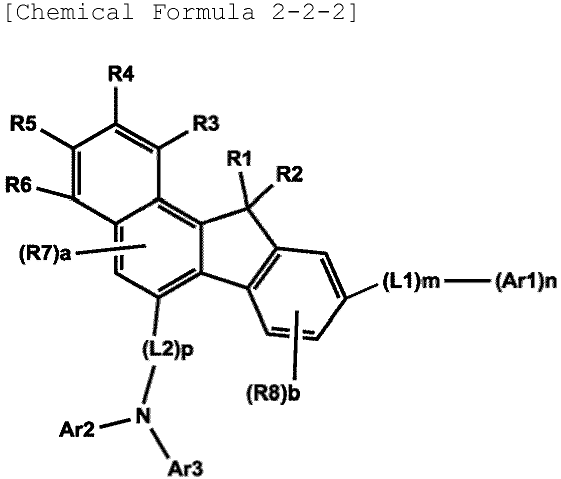 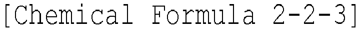 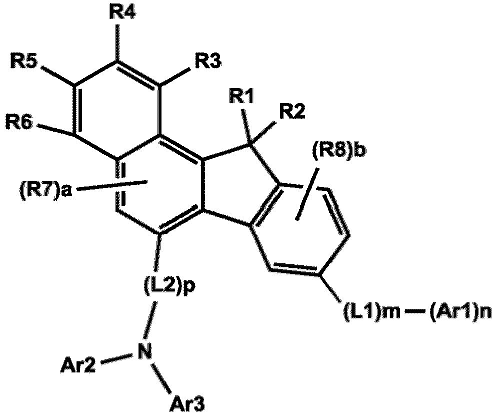 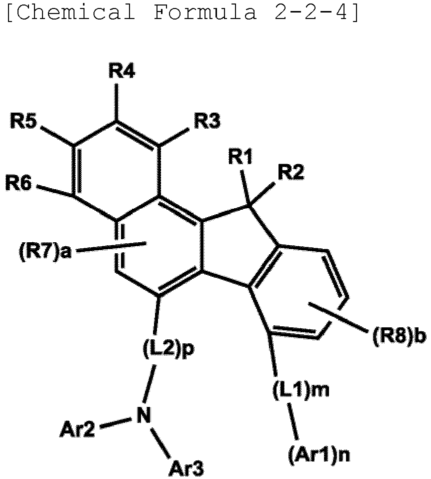 In Chemical Formulae 2-2-1 to 2-2-4,
the definitions of R1 to R8, L1, L2, Ar1 to Ar3, p, m, n, a and b are the same as the definitions in Chemical Formula 2-2.

In an exemplary embodiment of the present application, Chemical Formula 3-1 may be represented by any one of the following Chemical Formulae 3-1-1 to 3-1-4. 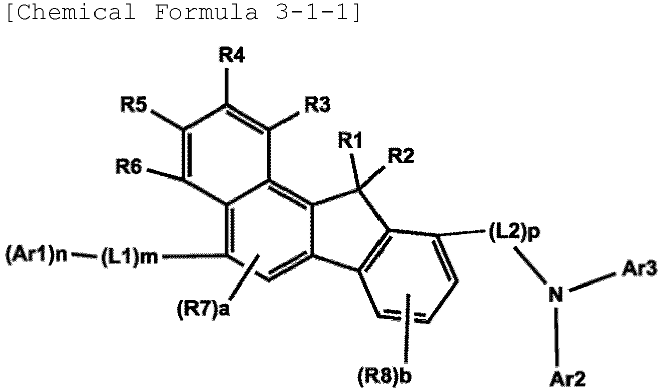 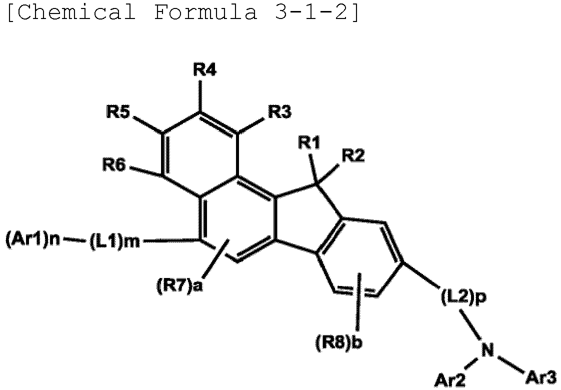 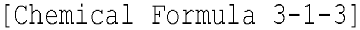 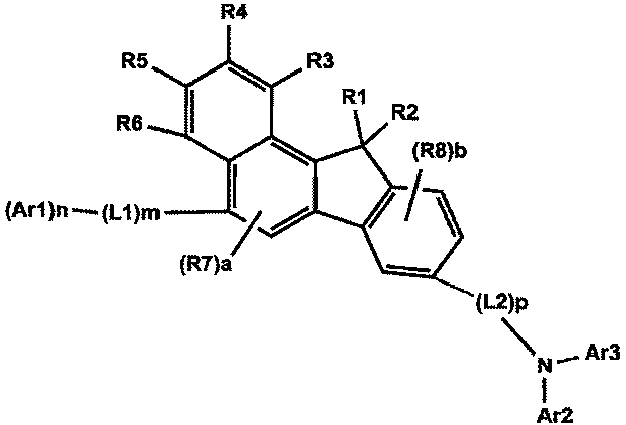 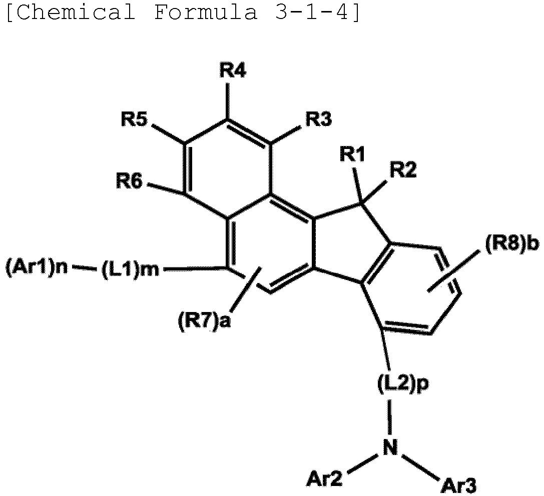 In Chemical Formulae 3-1-1 to 3-1-4,
the definitions of R1 to R8, L1, L2, Ar1 to Ar3, p, m, n, a and b are the same as the definitions in Chemical Formula 3-1.

In an exemplary embodiment of the present application, Chemical Formula 3-2 may be represented by any one of the following Chemical Formulae 3-2-1 to 3-2-4. 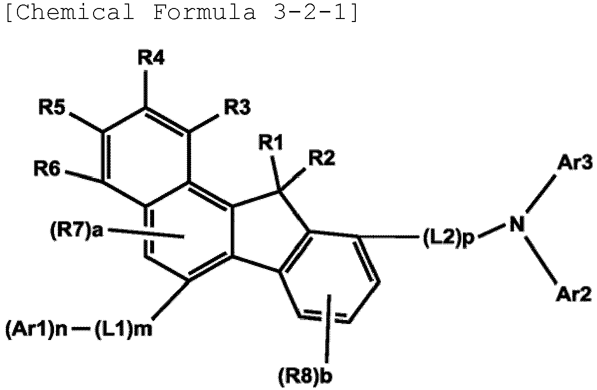 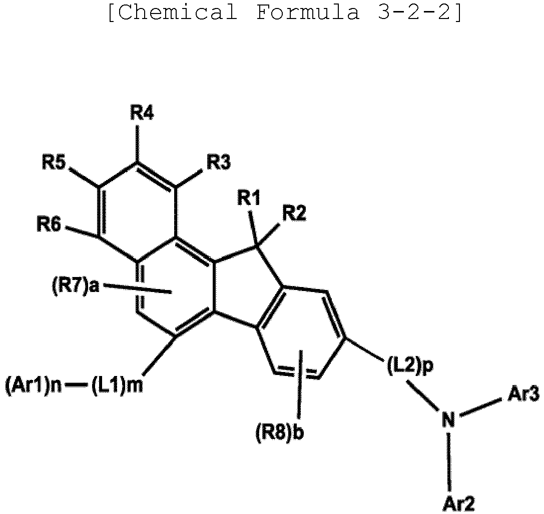 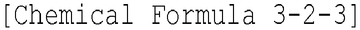 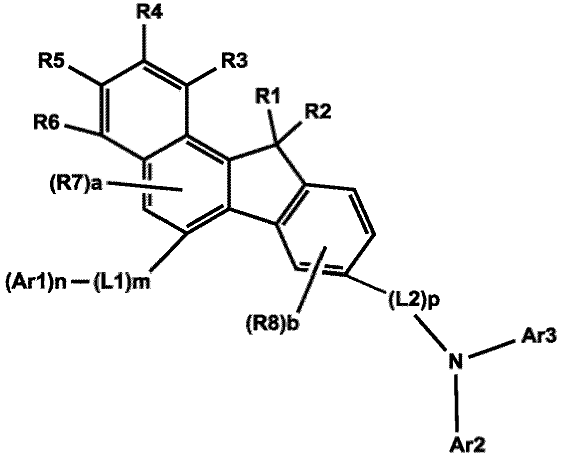 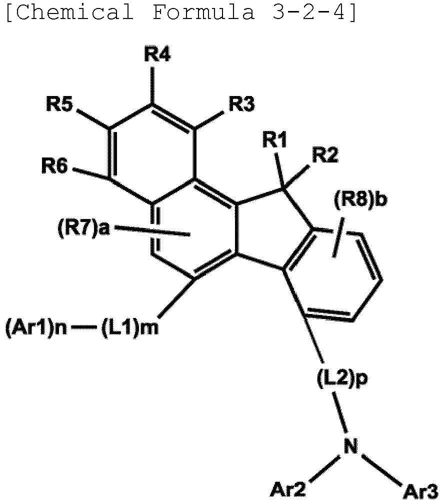 In Chemical Formulae 3-2-1 to 3-2-4,
the definitions of R1 to R8, L1, L2, Ar1 to Ar3, p, m, n, a and b are the same as the definitions in Chemical Formula 3-2.

In an exemplary embodiment of the present application, R1 and R2 are the same as or different from each other, and may be each independently a substituted or unsubstituted C1 to C60 alkyl group; a substituted or unsubstituted C6 to C60 aryl group; or a substituted or unsubstituted C2 to C60 heteroaryl group.

In another exemplary embodiment, R1 and R2 are the same as or different from each other, and may be each independently a substituted or unsubstituted C1 to C60 alkyl group; or a substituted or unsubstituted C6 to C60 aryl group.

In still another exemplary embodiment, R1 and R2 are the same as or different from each other, and may be each independently a substituted or unsubstituted C1 to C50 alkyl group; or a substituted or unsubstituted C6 to C50 aryl group.

In yet another exemplary embodiment, R1 and R2 are the same as or different from each other, and may be each independently a substituted or unsubstituted C1 to C40 alkyl group; or a substituted or unsubstituted C6 to C40 aryl group.

In still yet another exemplary embodiment, R1 and R2 are the same as or different from each other, and may be each independently a C1 to C40 alkyl group; or a C6 to C40 aryl group.

In a further exemplary embodiment, R1 and R2 are the same as or different from each other, and may be each independently a C1 to C20 alkyl group; or a C6 to C20 aryl group.

In another further exemplary embodiment, R1 and R2 are the same as or different from each other, and may be each independently a straight-chained C1 to C20 alkyl group; or a C6 to C20 monocyclic or polycyclic aryl group.

In still another further exemplary embodiment, R1 and R2 are the same as or different from each other, and may be each independently a methyl group; or a phenyl group.

In an exemplary embodiment of the present application, R3 to R8 are the same as or different from each other, and are each independently selected from the group consisting of hydrogen; deuterium; a halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)RR'; -SiRR'R" and -NRR', or two or more adjacent groups may be bonded to each other to form a substituted or unsubstituted C6 to C60 aliphatic or aromatic hydrocarbon ring, or a substituted or unsubstituted C2 to C60 aliphatic or aromatic hetero ring.

In another exemplary embodiment, R3 to R8 are the same as or different from each other, and may be each independently selected from the group consisting of hydrogen; a substituted or unsubstituted C1 to C60 alkyl group; a substituted or unsubstituted C6 to C60 aryl group; a substituted or unsubstituted C2 to C60 heteroaryl group; -P(=O)RR'; -SiRR'R" and -NRR'.

In still another exemplary embodiment, R3 to R8 are the same as or different from each other, and may be each independently hydrogen; a substituted or unsubstituted C1 to C60 alkyl group; a substituted or unsubstituted C6 to C60 aryl group; or a substituted or unsubstituted C2 to C60 heteroaryl group.

In yet another exemplary embodiment, R3 to R8 are the same as or different from each other, and may be each independently hydrogen; a substituted or unsubstituted C1 to C40 alkyl group; a substituted or unsubstituted C6 to C40 aryl group; or a substituted or unsubstituted C2 to C40 heteroaryl group.

In still yet another exemplary embodiment, R3 to R8 are the same as or different from each other, and may be each independently hydrogen; a C1 to C40 alkyl group; a C6 to C40 aryl group; or a C2 to C40 heteroaryl group.

In a further exemplary embodiment, R3 to R8 may be hydrogen.

In an exemplary embodiment of the present application, L1 and L2 are the same as or different from each other, and may be each independently a direct bond; a substituted or unsubstituted C6 to C60 arylene group; or a substituted or unsubstituted C2 to C60 heteroarylene group.

In another exemplary embodiment, L1 and L2 are the same as or different from each other, and may be each independently a direct bond; a substituted or unsubstituted C6 to C40 arylene group; or a substituted or unsubstituted C2 to C40 heteroarylene group.

In still another exemplary embodiment, L1 and L2 are the same as or different from each other, and may be each independently a direct bond; a substituted or unsubstituted C6 to C20 arylene group; or a substituted or unsubstituted C2 to C20 heteroarylene group.

In yet another exemplary embodiment, L1 and L2 are the same as or different from each other, and may be each independently a direct bond; a C6 to C20 arylene group; or a C2 to C20 heteroarylene group.

In still yet another exemplary embodiment, L1 and L2 are the same as or different from each other, and may be each independently a direct bond; or a C6 to C20 arylene group.

In a further exemplary embodiment, L1 and L2 are the same as or different from each other, and may be each independently a direct bond; or a C6 to C20 monocyclic or polycyclic arylene group.

In another further exemplary embodiment, L1 and L2 are the same as or different from each other, and may be each independently a direct bond; a C6 to C10 monocyclic arylene group; or a C10 to C20 polycyclic arylene group.

In still another further exemplary embodiment, L1 and L2 are the same as or different from each other, and may be each independently a direct bond; a phenylene group; a biphenylene group; or a naphthalene group.

In an exemplary embodiment of the present application, L1 may be a direct bond; or a phenylene group.

In an exemplary embodiment of the present application, L2 may be a direct bond; a phenylene group; or a biphenylene group.

In an exemplary embodiment of the present application, Ar1 may be a substituted or unsubstituted C1 to C60 alkyl group; a substituted or unsubstituted C6 to C60 aryl group; or a substituted or unsubstituted C2 to C60 heteroaryl group.

In another exemplary embodiment, Ar1 may be a substituted or unsubstituted C6 to C60 aryl group; or a substituted or unsubstituted C2 to C60 heteroaryl group.

In still another exemplary embodiment, Ar1 may be a C6 to C60 aryl group which is unsubstituted or substituted with one or more substituents selected from the group consisting of a C1 to C30 alkyl group, deuterium and a halogen group; or a C2 to C60 heteroaryl group.

In yet another exemplary embodiment, Ar1 may be a C6 to C40 aryl group which is unsubstituted or substituted with one or more substituents selected from the group consisting of a C1 to C30 alkyl group, deuterium and a halogen group; or a C2 to C40 heteroaryl group.

In still yet another exemplary embodiment, Ar1 may be a C6 to C40 aryl group which is unsubstituted or substituted with one or more substituents selected from the group consisting of a C1 to C30 alkyl group, deuterium and a halogen group; or a C2 to C40 heteroaryl group including at least one of N, O and S as a heteroatom.

In a further exemplary embodiment, Ar1 may be a C6 to C20 aryl group which is unsubstituted or substituted with one or more substituents selected from the group consisting of a C1 to C30 alkyl group, deuterium and a halogen group; or a C2 to C20 heteroaryl group including at least one of N, O and S as a heteroatom.

In another further exemplary embodiment, Ar1 may be a phenyl group substituted with deuterium; a phenyl group substituted with a fluoro group (-F); a phenyl group substituted with a t-butyl group; a phenyl group substituted with a methyl group; an unsubstituted phenyl group; a biphenyl group; a naphthyl group; a dimethylfluorenyl group; or a dibenzofuran group.

In an exemplary embodiment of the present application, Ar2 and Ar3 are the same as or different from each other, and may be each independently a substituted or unsubstituted C1 to C60 alkyl group; a substituted or unsubstituted C6 to C60 aryl group; or a substituted or unsubstituted C2 to C60 heteroaryl group.

In another exemplary embodiment, Ar2 and Ar3 are the same as or different from each other, and may be each independently a C6 to C60 aryl group which is unsubstituted or substituted with one or more substituents selected from the group consisting of a C1 to C30 alkyl group, a C2 to C40 heteroaryl group and a C6 to C40 aryl group; or a C2 to C60 heteroaryl group.

In still another exemplary embodiment, Ar2 and Ar3 are the same as or different from each other, and may be each independently a phenyl group substituted with a dimethylfluorenyl group; a phenyl group substituted with a dibenzofuran group; a phenyl group substituted with a dibenzothiophene group; a phenyl group substituted with a biphenyl group; a phenyl group substituted with a dimethylbenzo[a]fluorenyl group; an unsubstituted phenyl group; a biphenyl group; a naphthyl group; a terphenyl group; a dimethylfluorenyl group; a diphenylfluorenyl group; a spirobifluorenyl group; a dimethylbenzo[a]fluorenyl group; a dibenzofuran group; or a dibenzothiophene group.

In an exemplary embodiment of the present application, the dimethylbenzo[a]fluorenyl group may be represented by the following structural formula.

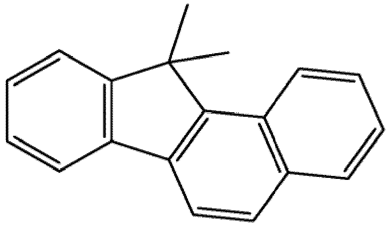

In an exemplary embodiment of the present application, the 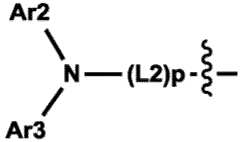 of Chemical Formula 1 may be represented by the following Chemical Formula A-1 or A-2. 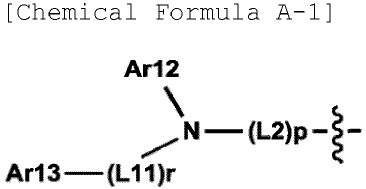 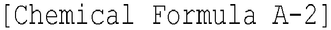 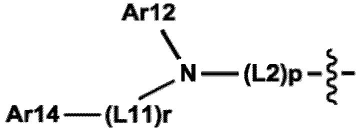 In Chemical Formulae A-1 and A-2,
the definitions of L2 and p are the same as the definitions in Chemical Formula 1, 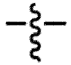 means a position linked to Chemical Formula 1,
L11 is a direct bond; or a substituted or unsubstituted C6 to C60 arylene group,
Ar12 and Ar13 are the same as or different from each other, and are each independently a substituted or unsubstituted C6 to C60 aryl group,
Ar14 is a substituted or unsubstituted C2 to C60 heteroaryl group, and
r is an integer from 0 to 3.

In an exemplary embodiment of the present application, L11 may be a direct bond; or a substituted or unsubstituted C6 to C60 arylene group.

In another exemplary embodiment, L11 may be a direct bond; or a substituted or unsubstituted C6 to C40 arylene group.

In still another exemplary embodiment, L11 may be a direct bond; or a substituted or unsubstituted C6 to C20 arylene group.

In yet another exemplary embodiment, L11 may be a direct bond; a phenylene group; or a biphenylene group.

In an exemplary embodiment of the present application, Ar12 and Ar13 are the same as or different from each other, and may be each independently a substituted or unsubstituted C6 to C60 aryl group.

In another exemplary embodiment, Ar12 and A13 are the same as or different from each other, and may be each independently a substituted or unsubstituted C6 to C40 aryl group.

In still another exemplary embodiment, Ar12 and A13 are the same as or different from each other, and may be each independently a substituted or unsubstituted C6 to C20 aryl group.

In yet another exemplary embodiment, Ar12 and Ar13 are the same as or different from each other, and may be each independently a phenyl group; a biphenyl group; a naphthyl group; a terphenyl group; a dimethylfluorenyl group; a diphenylfluorenyl group; a spirobifluorenyl group; or a dimethylbenzo[a]fluorenyl group.

In an exemplary embodiment of the present application, Ar14 may be a substituted or unsubstituted C2 to C60 heteroaryl group.

In another exemplary embodiment, Ar14 may be a substituted or unsubstituted C2 to C40 heteroaryl group.

In still another exemplary embodiment, Ar14 may be a C2 to C40 heteroaryl group.

In yet another exemplary embodiment, Ar14 may be a dibenzofuran group; or a dibenzothiophene group.

In an exemplary embodiment of the present application, R, R' and R" are the same as or different from each other, and may be each independently a substituted or unsubstituted C1 to C60 alkyl group; a substituted or unsubstituted C6 to C60 aryl group; or a substituted or unsubstituted C2 to C60 heteroaryl group.

In another exemplary embodiment, R, R' and R" are the same as or different from each other, and may be each independently a substituted or unsubstituted C6 to C60 aryl group.

In still another exemplary embodiment, R, R' and R" are the same as or different from each other, and may be each independently a substituted or unsubstituted C6 to C60 monocyclic or polycyclic aryl group.

In yet another exemplary embodiment, R, R' and R" are the same as or different from each other, and may be each independently a substituted or unsubstituted C6 to C40 monocyclic aryl group.

In still yet another exemplary embodiment, R, R' and R" are the same as or different from each other, and may be each independently a C6 to C20 monocyclic aryl group.

In a further exemplary embodiment, R, R' and R" may be a phenyl group.

In an exemplary embodiment of the present application, the definition of R31 is the same as the definition of R.

According to an exemplary embodiment of the present application, Chemical Formula 1 may be represented by any one of the following compounds, but is not limited thereto.

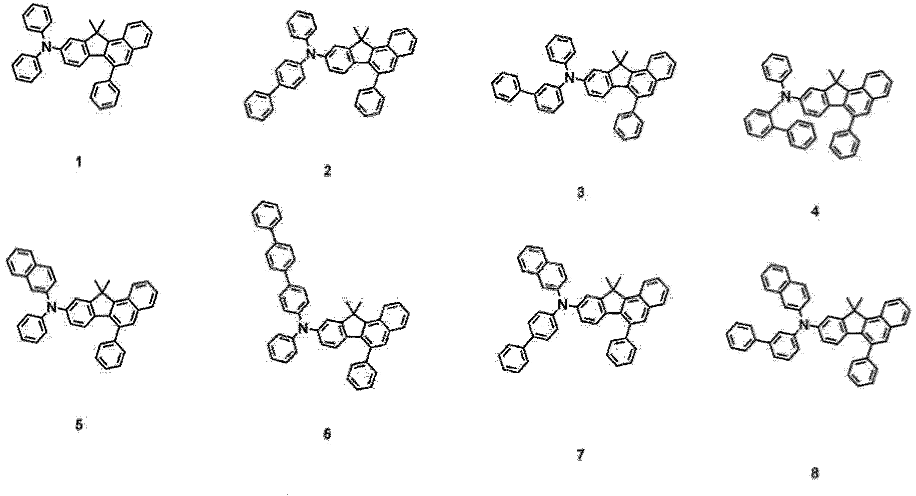

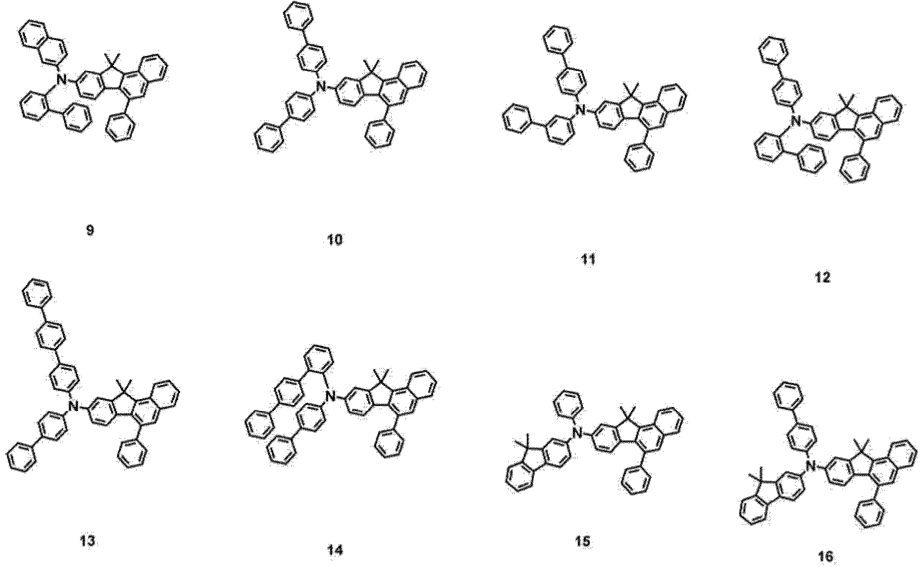

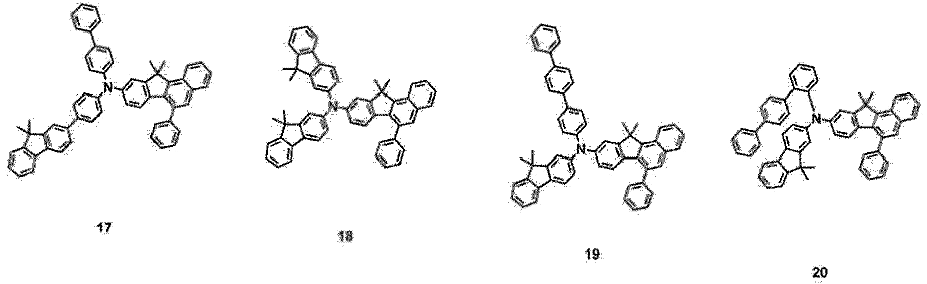

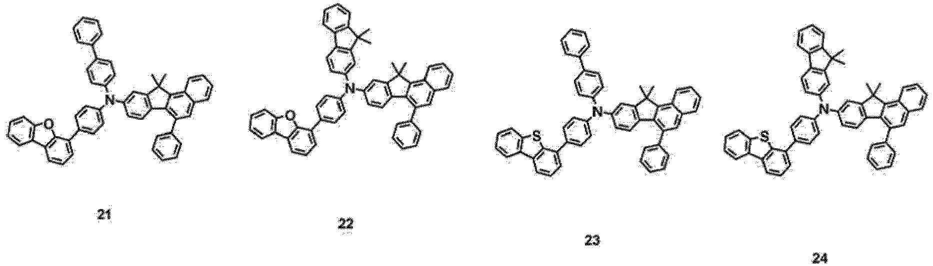

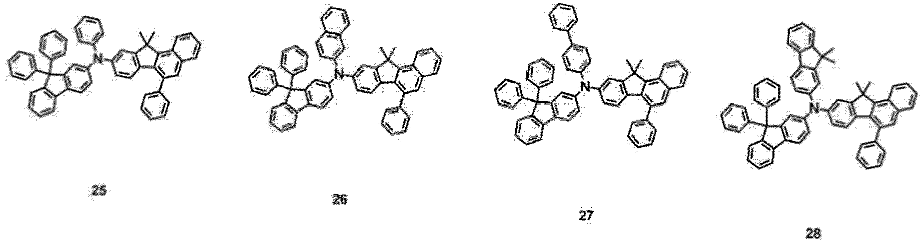

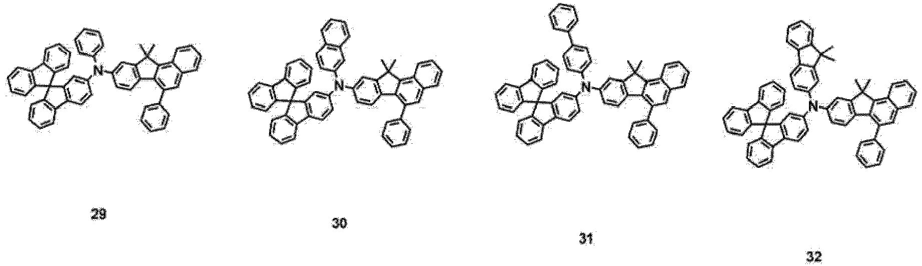

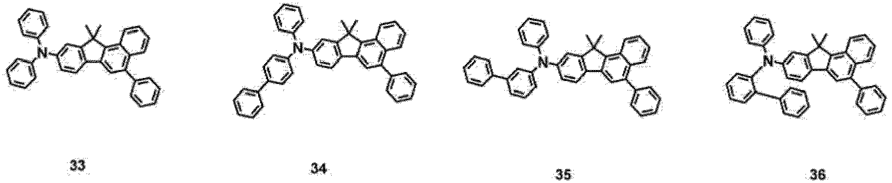

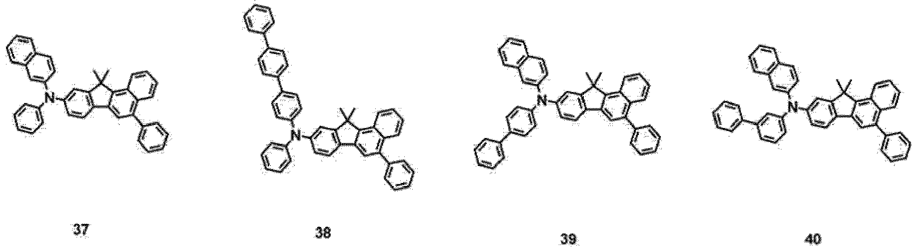

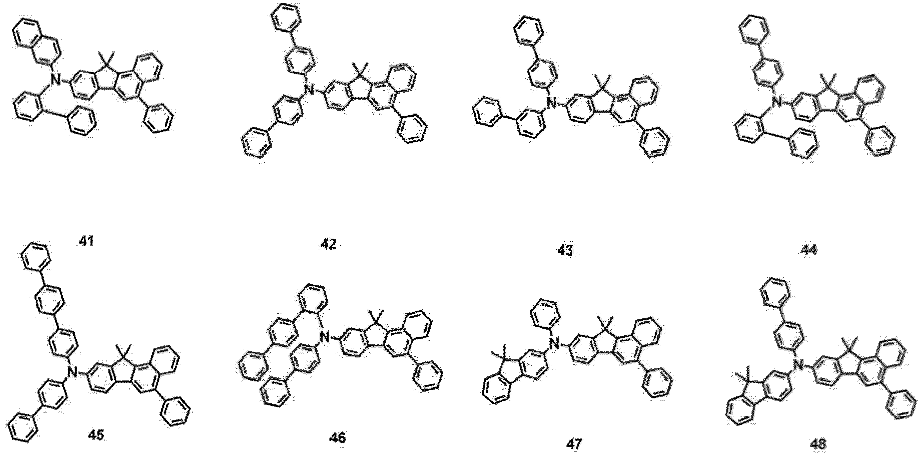

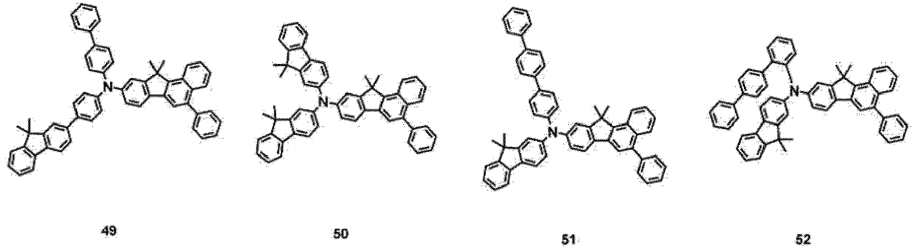

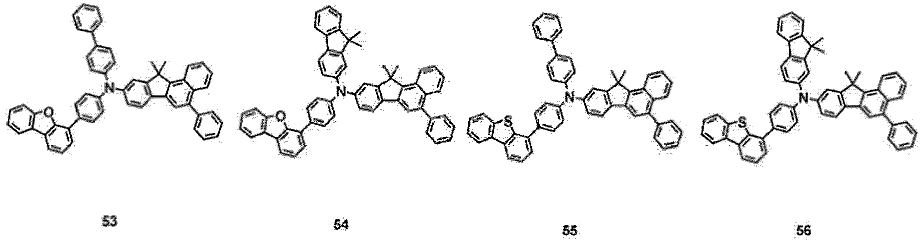

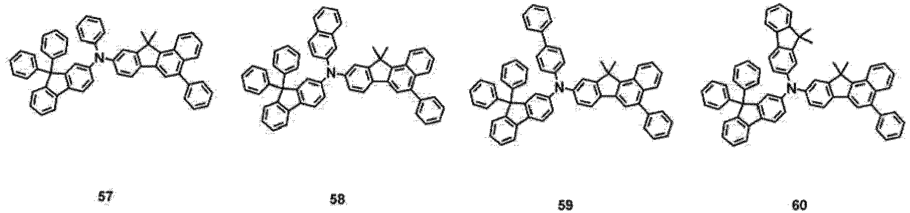

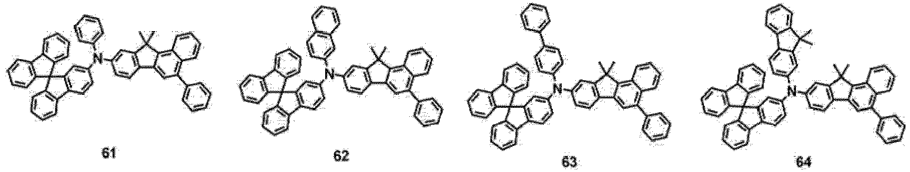

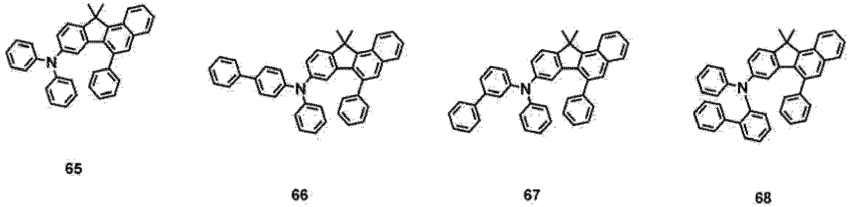

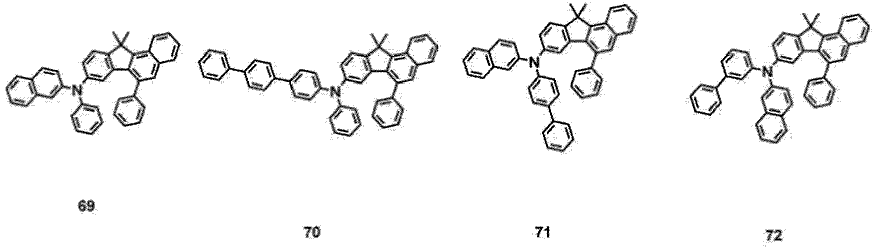

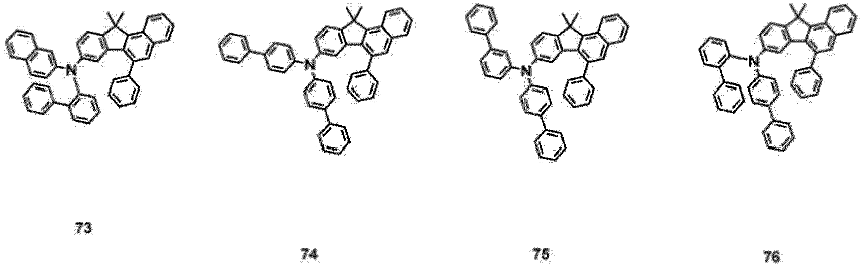

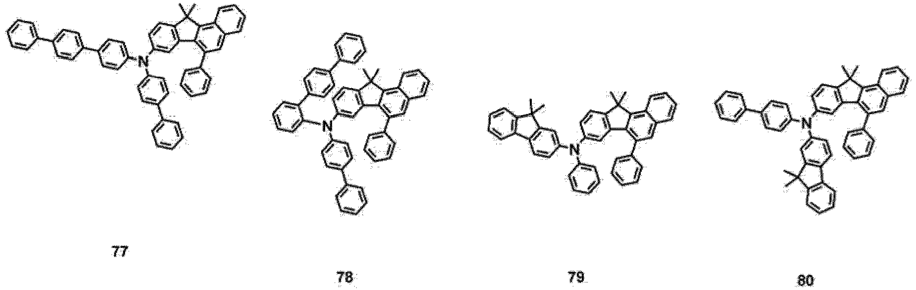

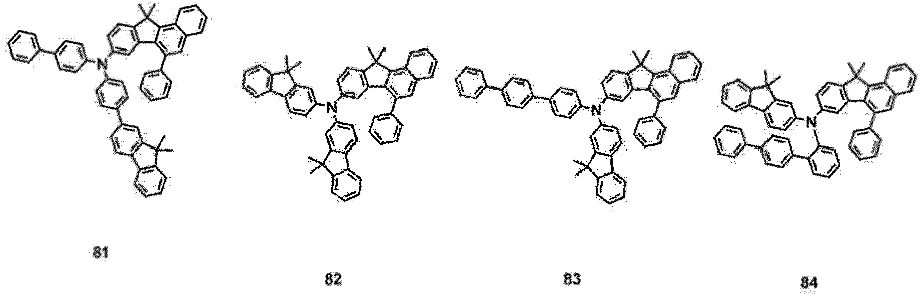

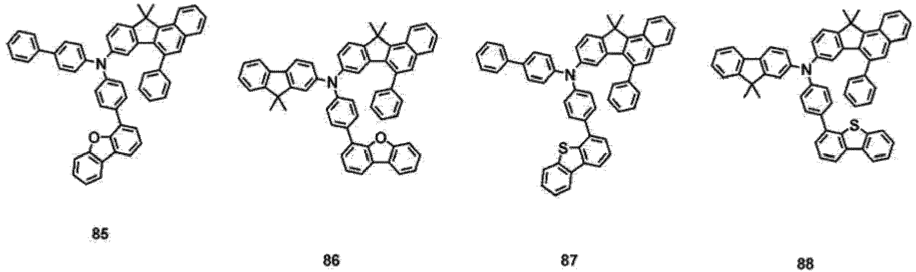

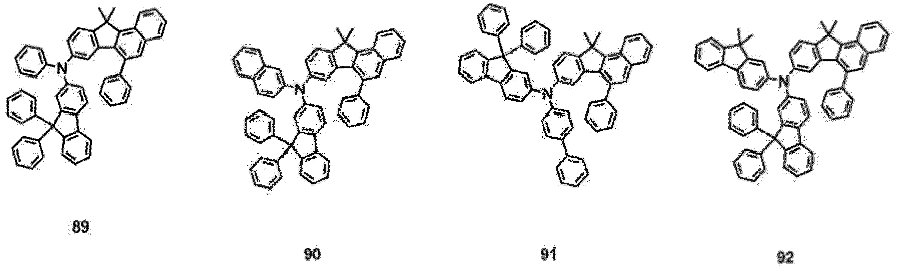

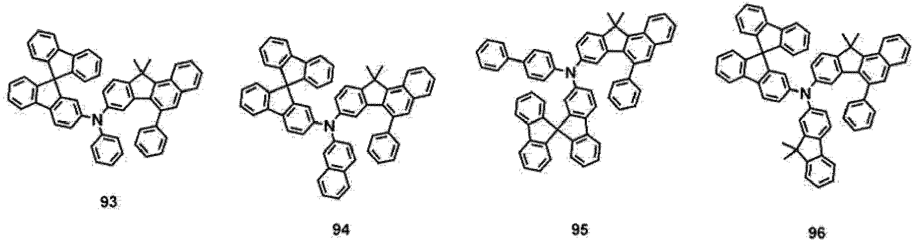

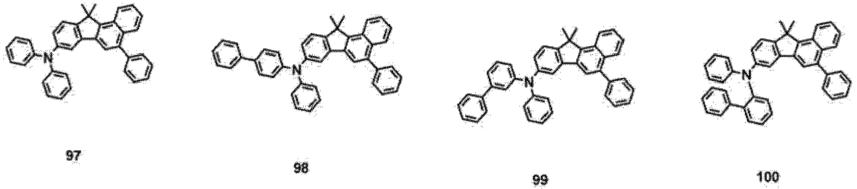

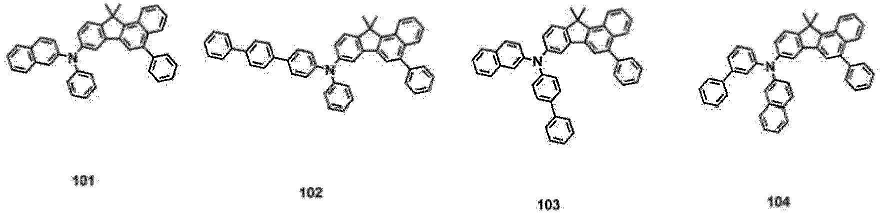

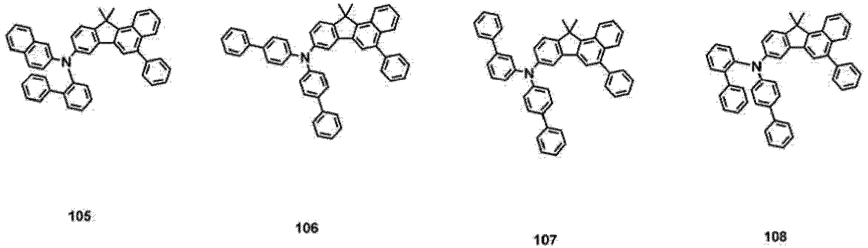

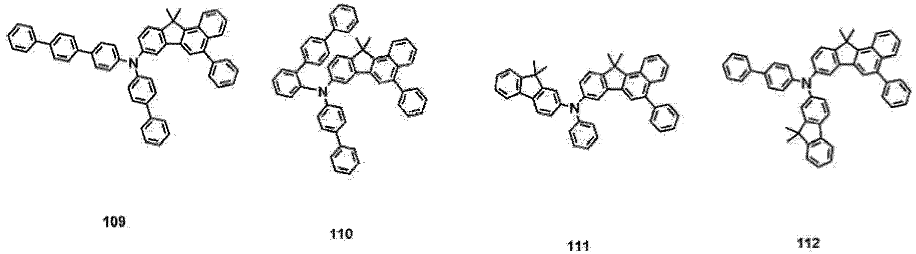

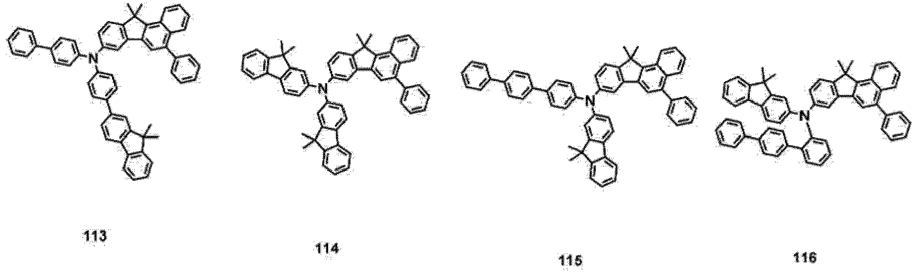

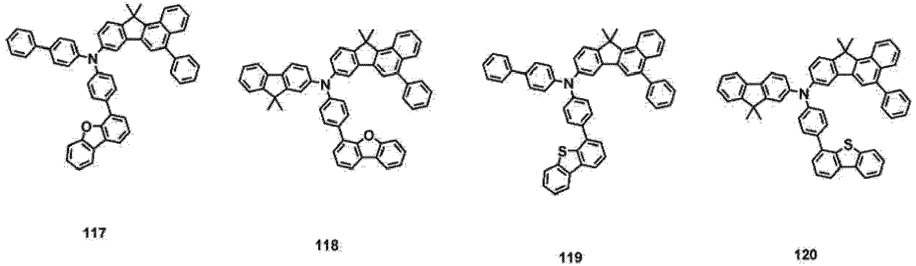

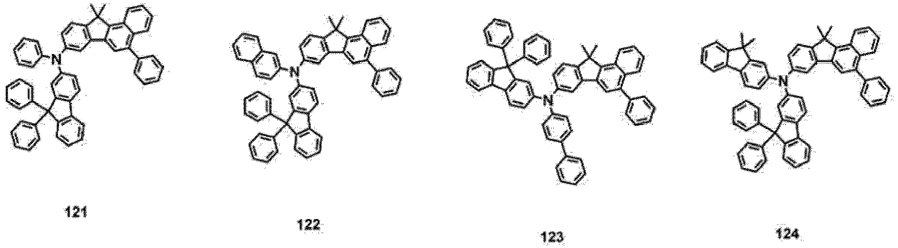

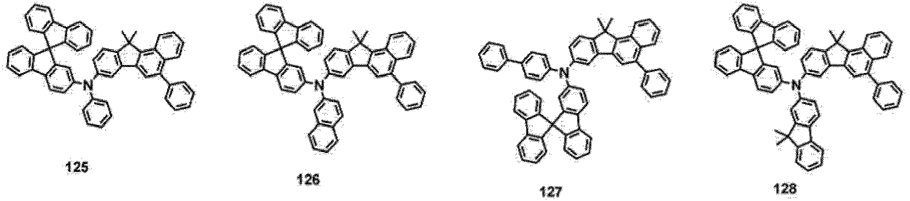

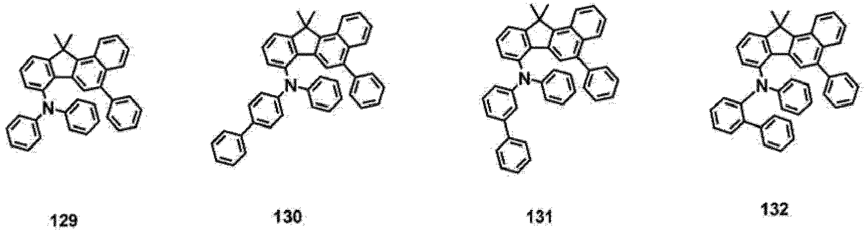

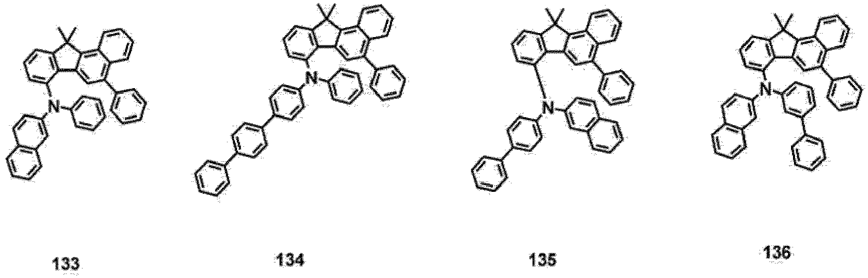

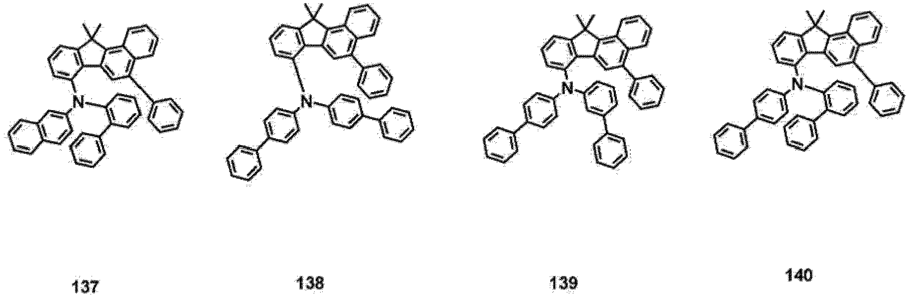

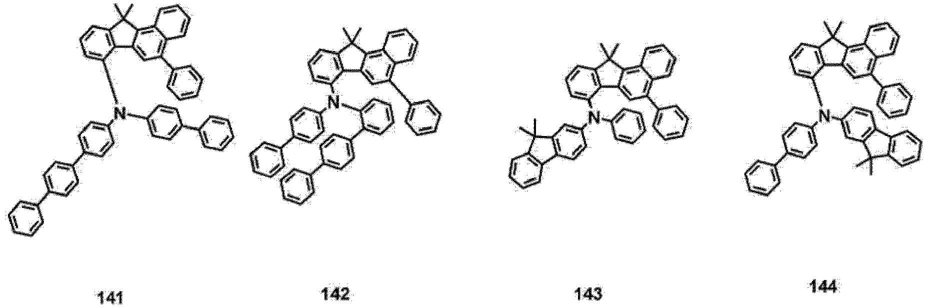

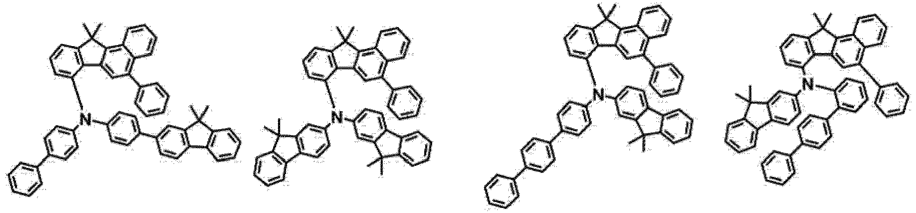

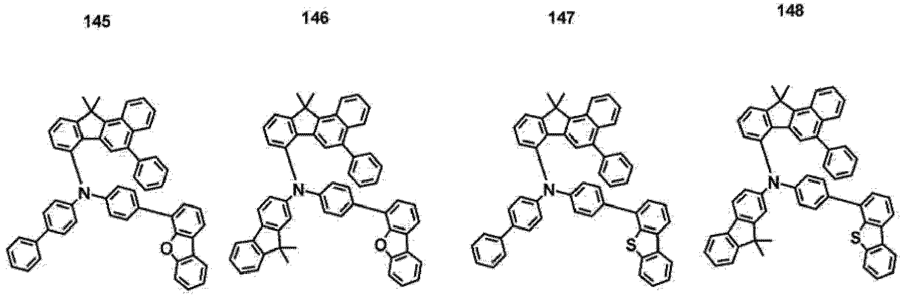

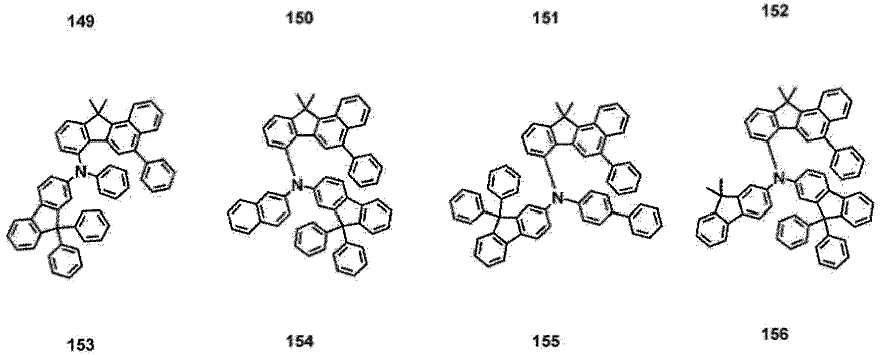

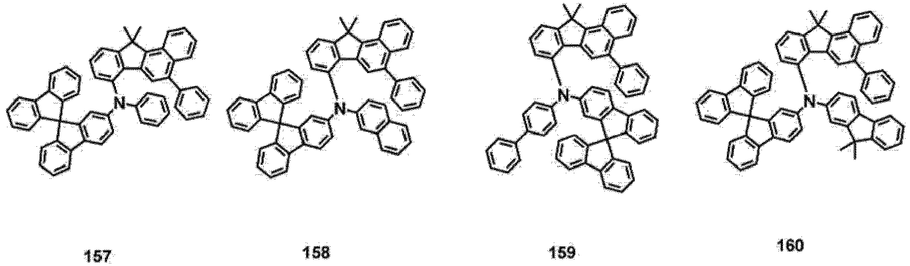

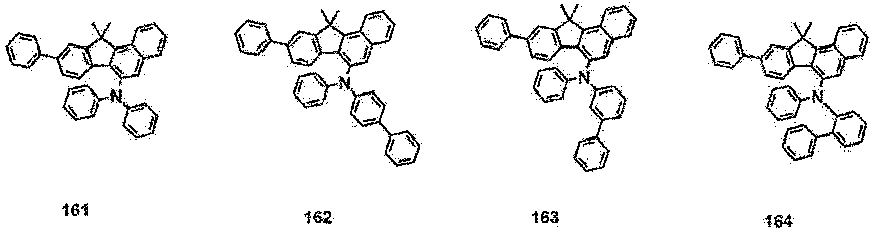

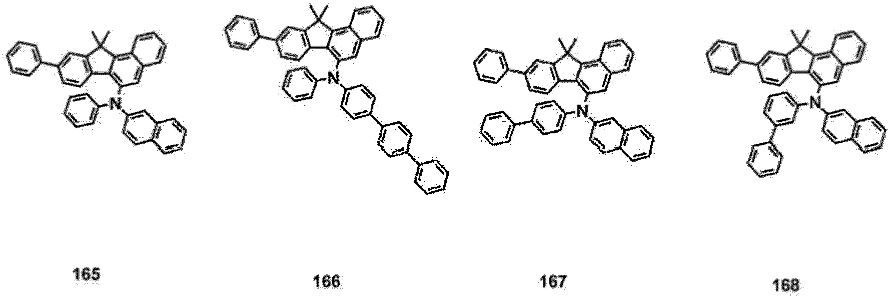

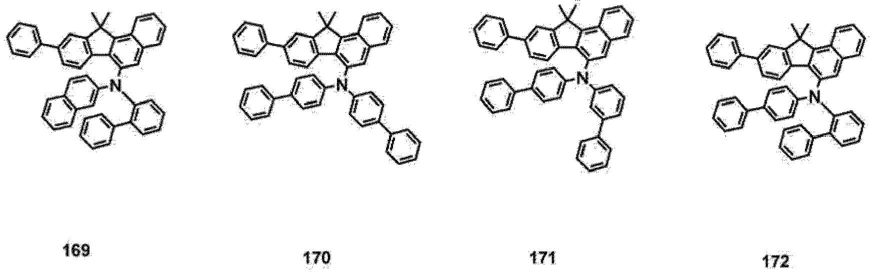

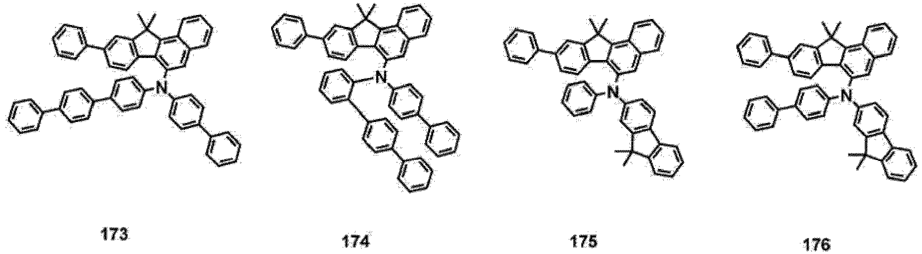

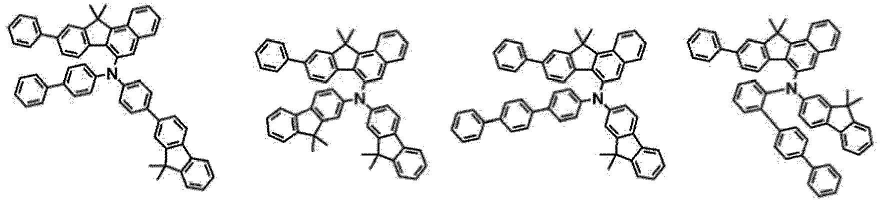

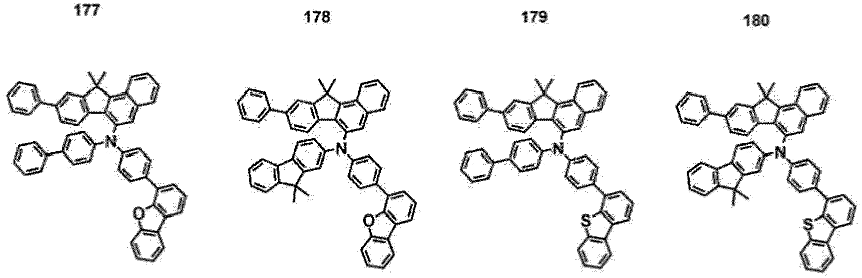

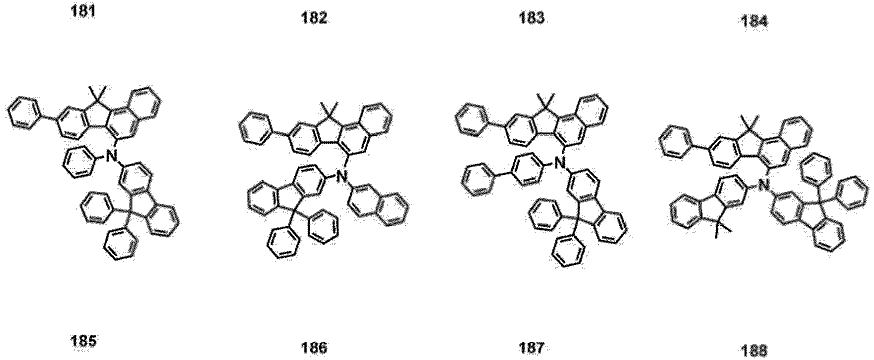

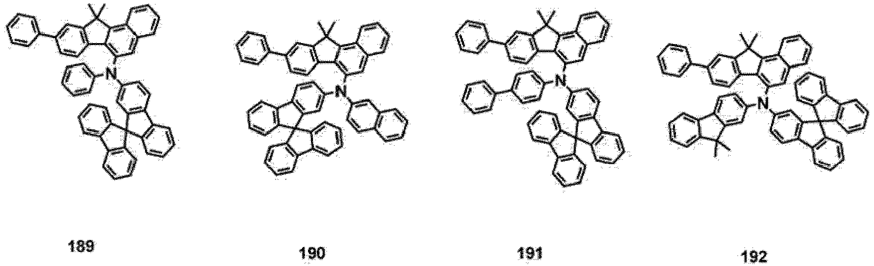

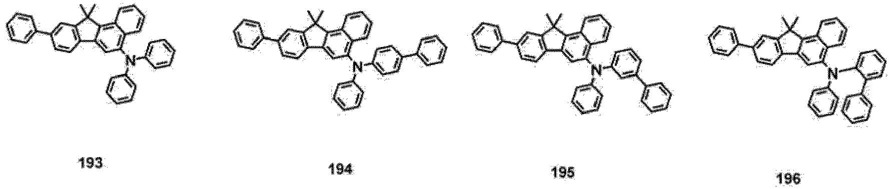

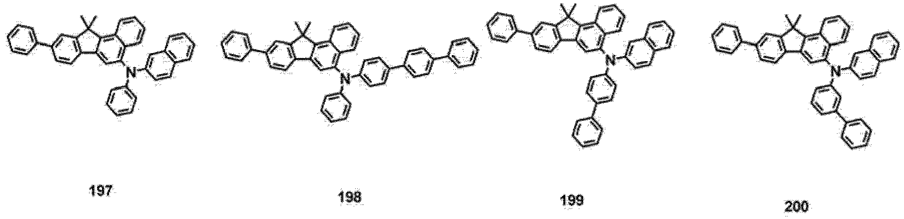

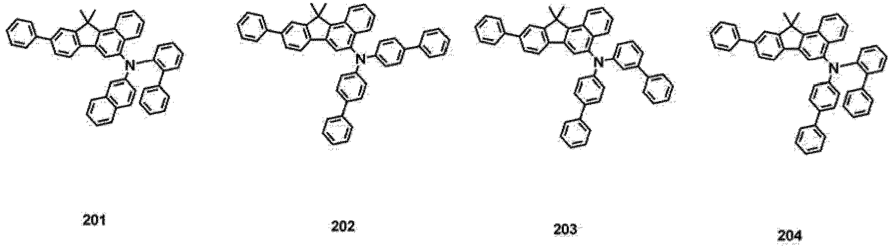

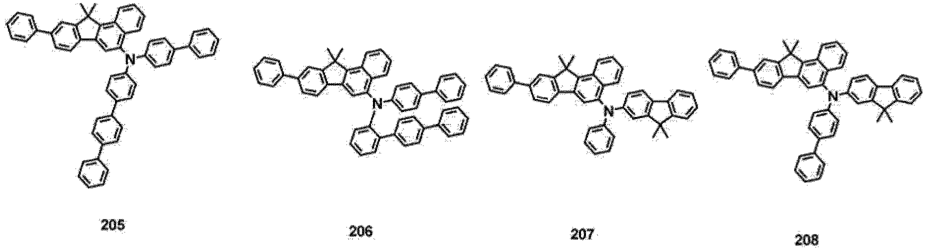

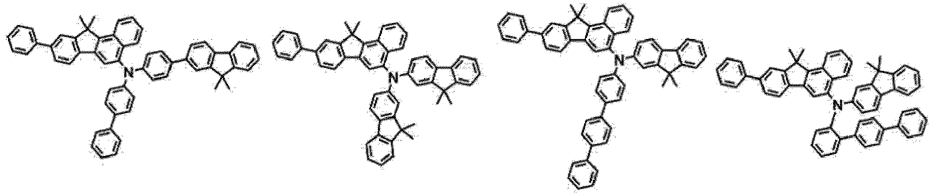

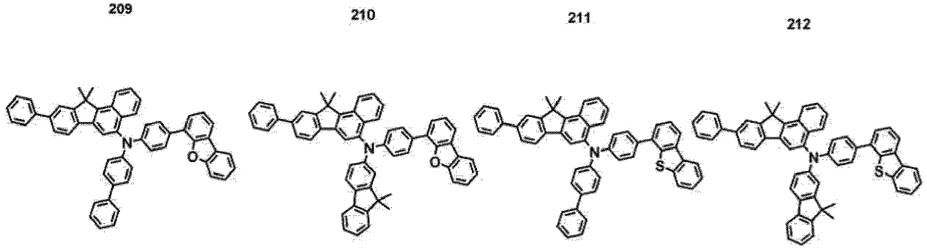

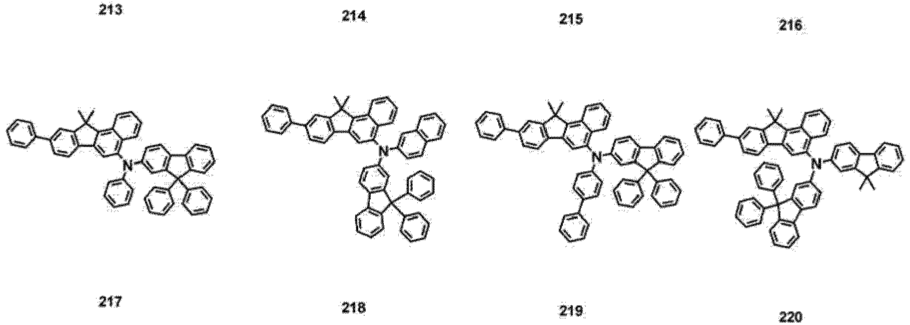

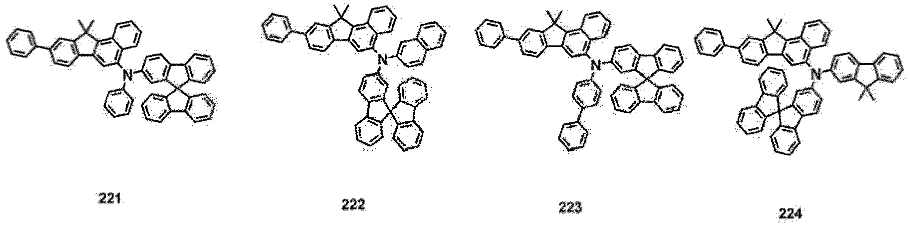

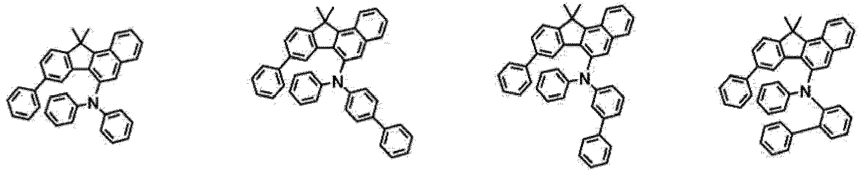

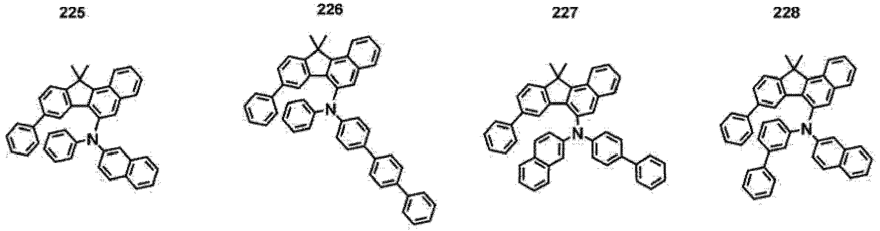

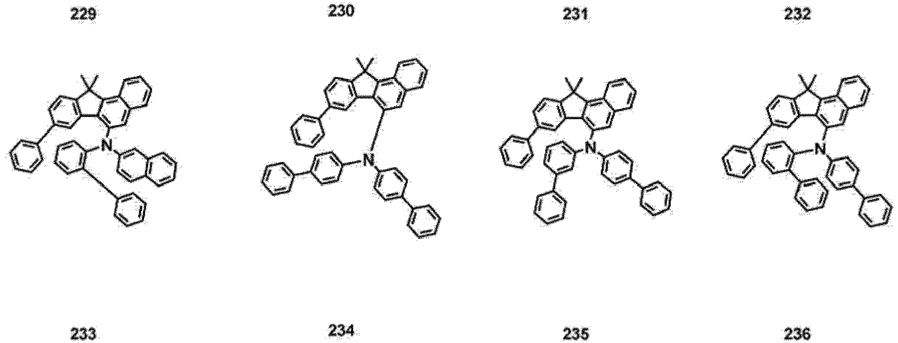

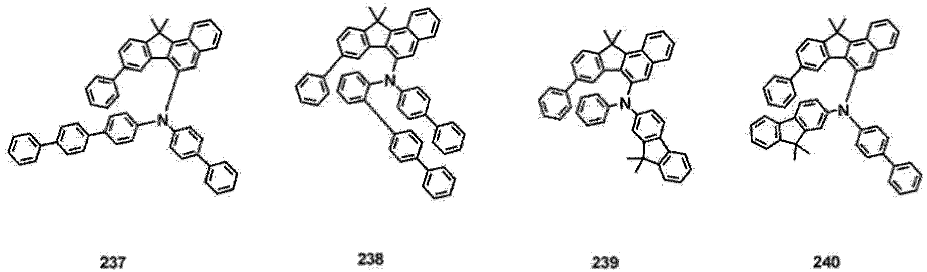

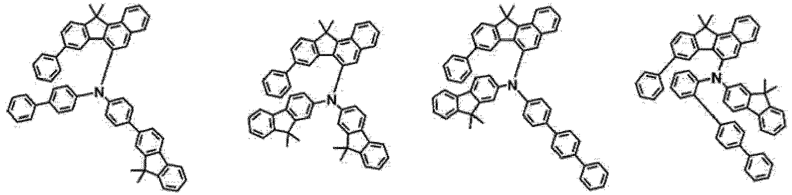

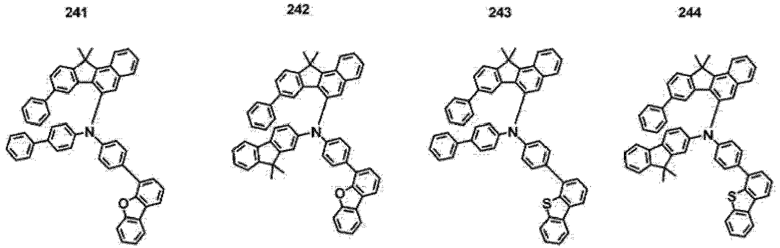

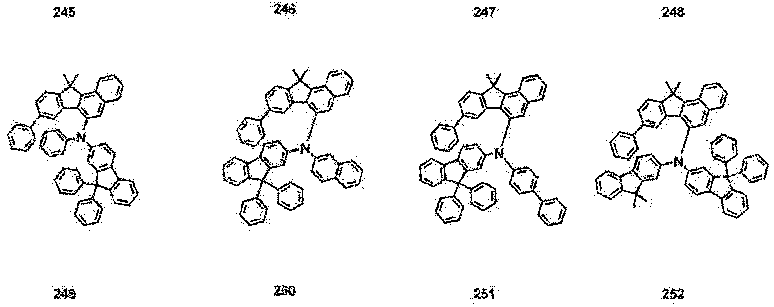

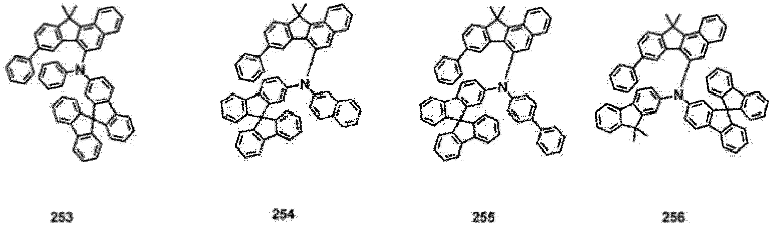

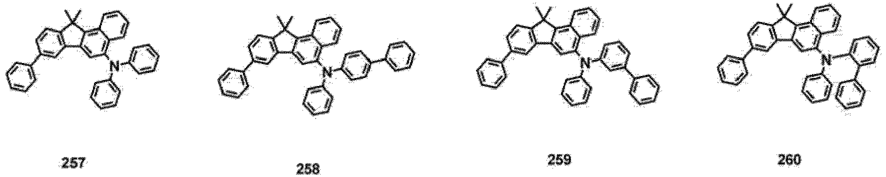

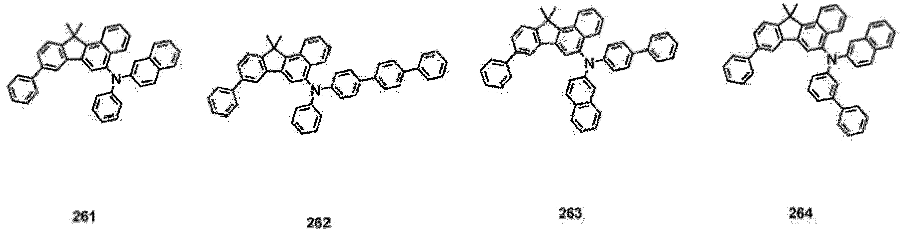

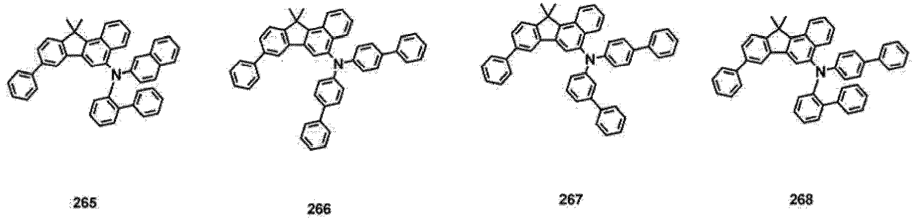

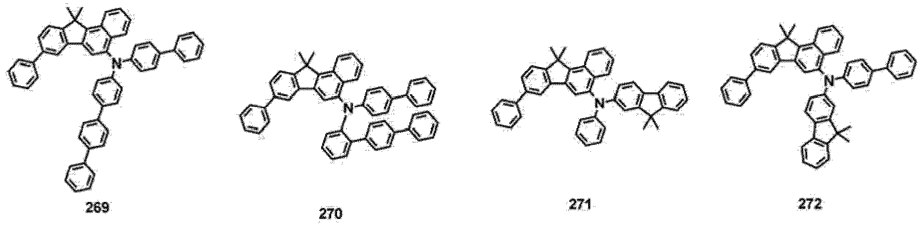

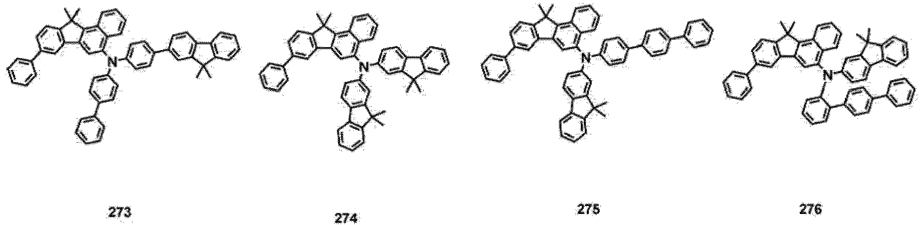

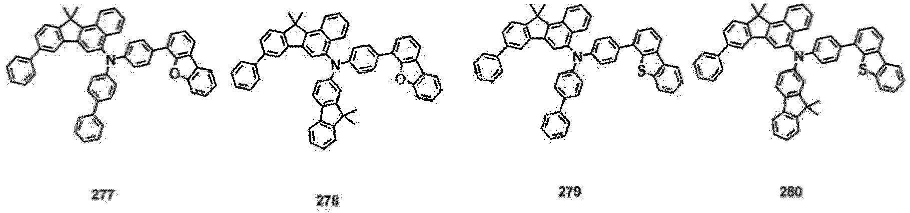

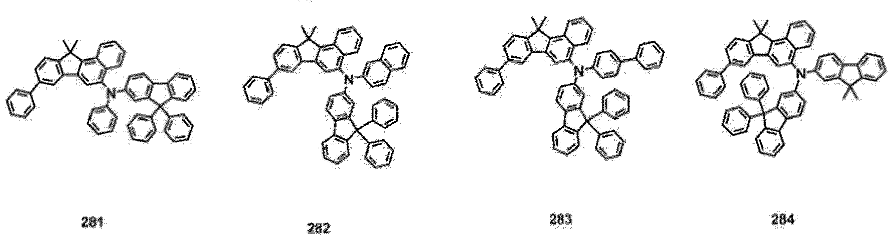

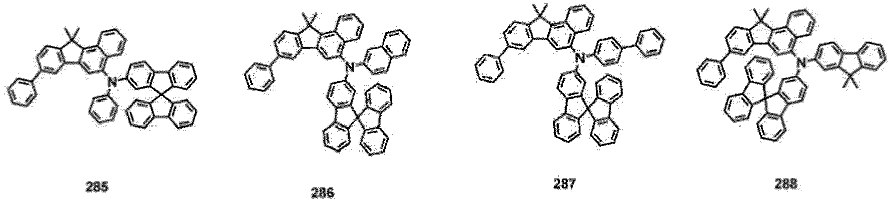

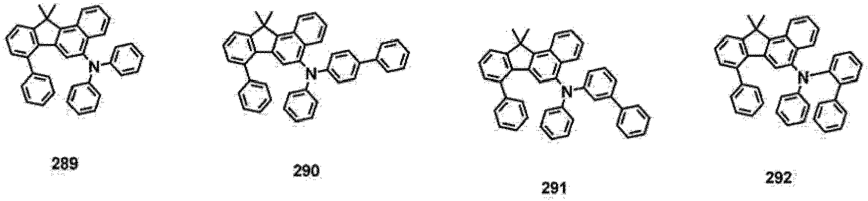

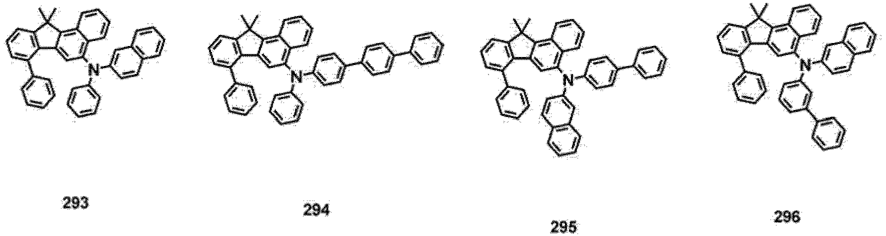

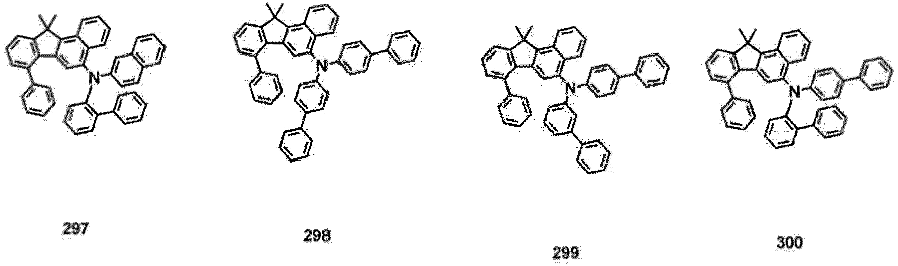

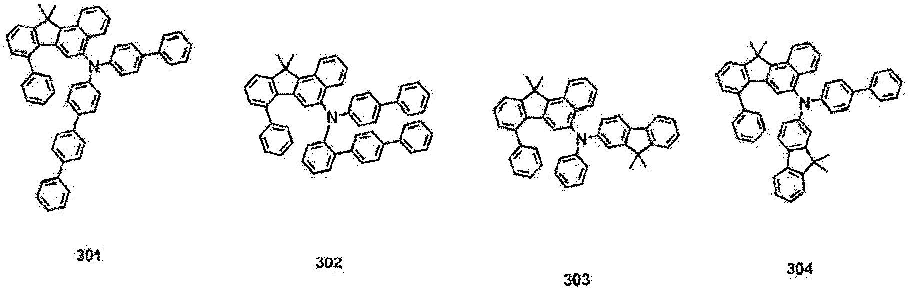

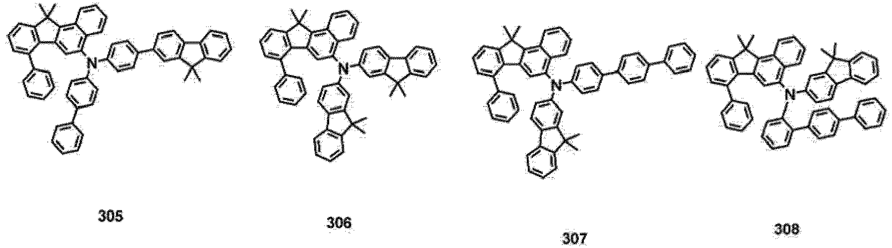

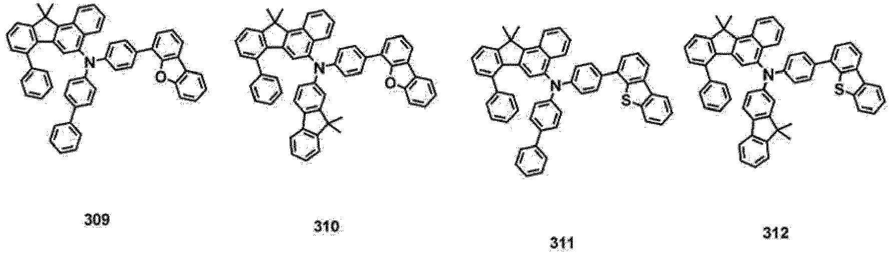

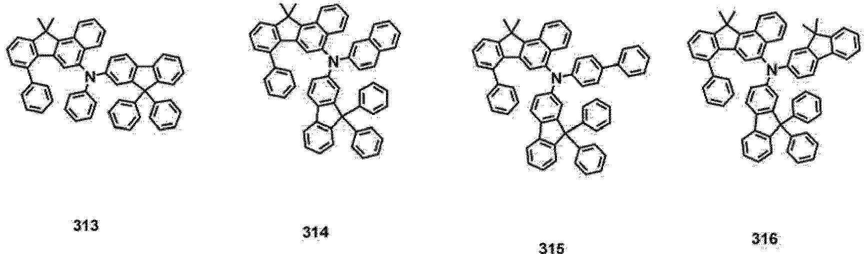

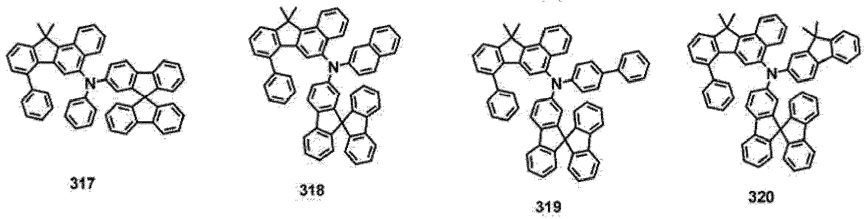

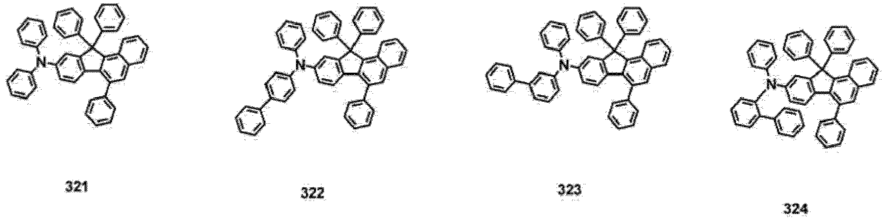

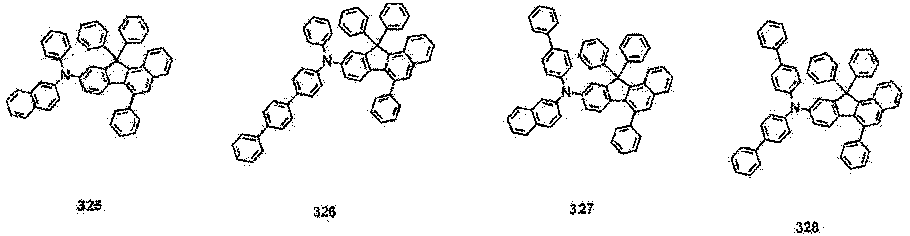

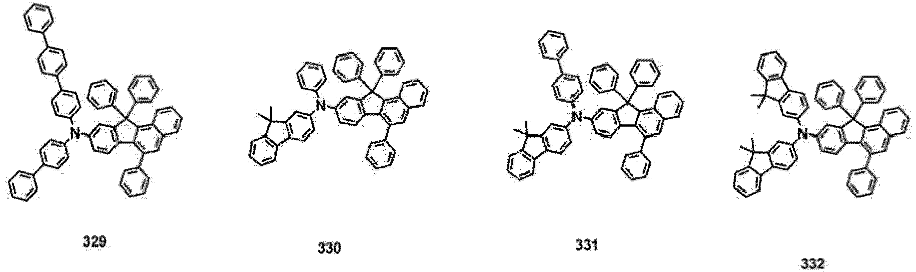

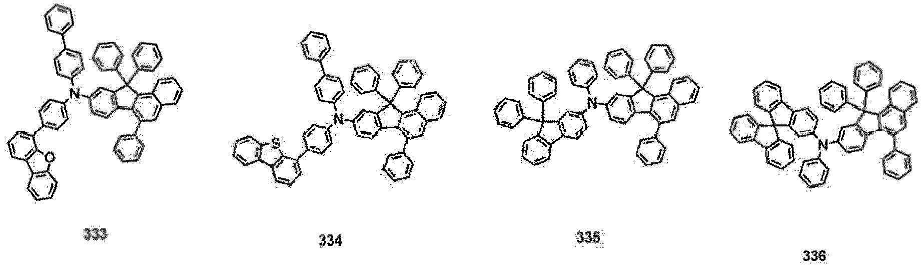

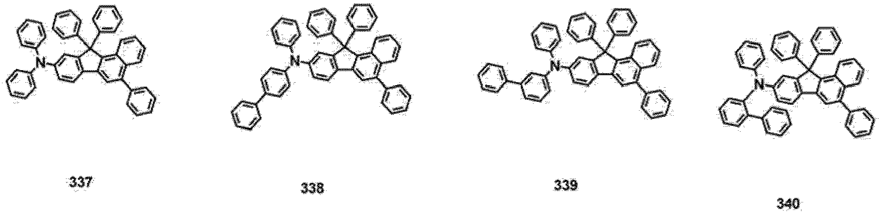

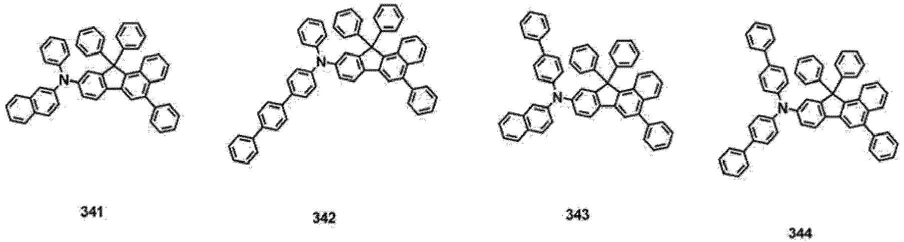

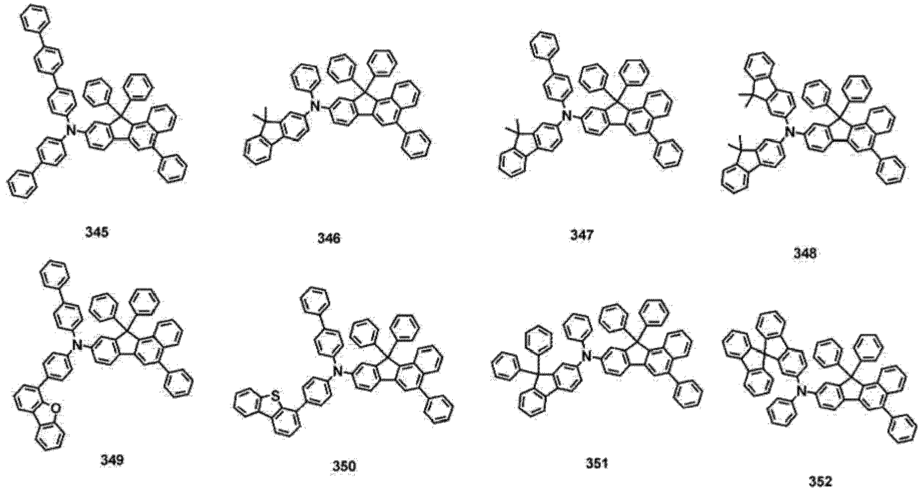

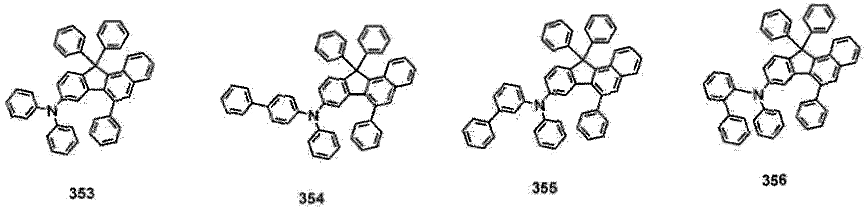

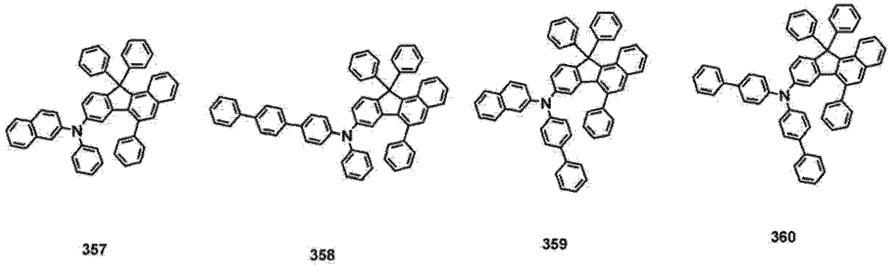

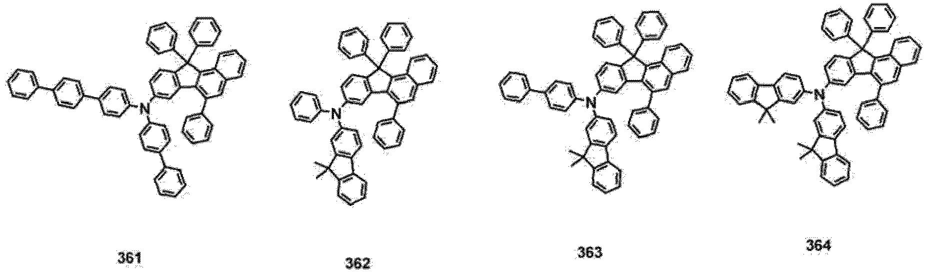

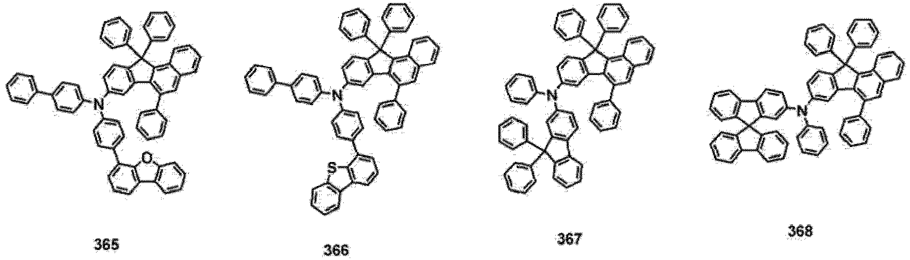

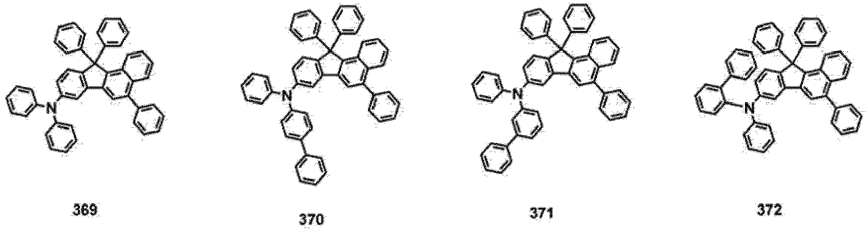

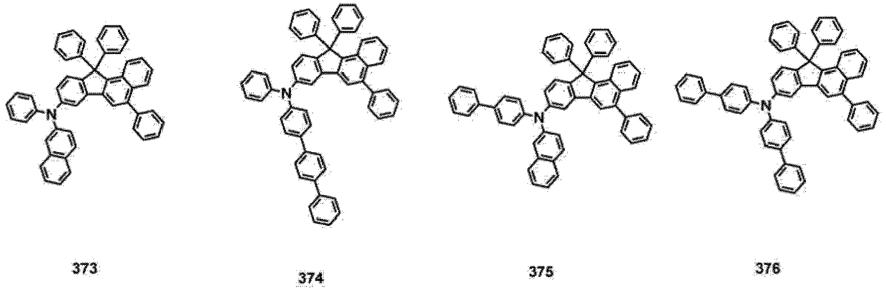

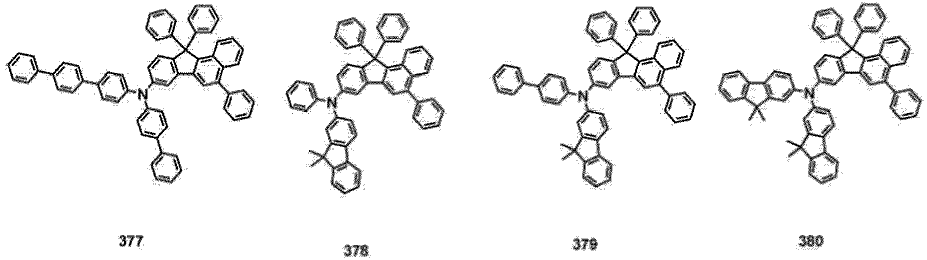

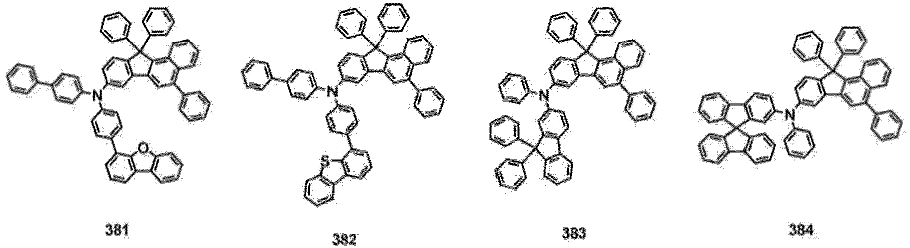

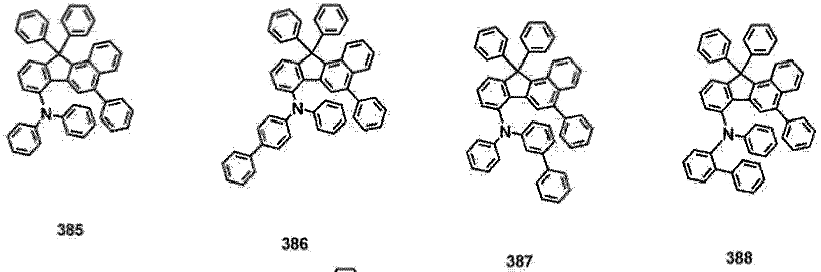

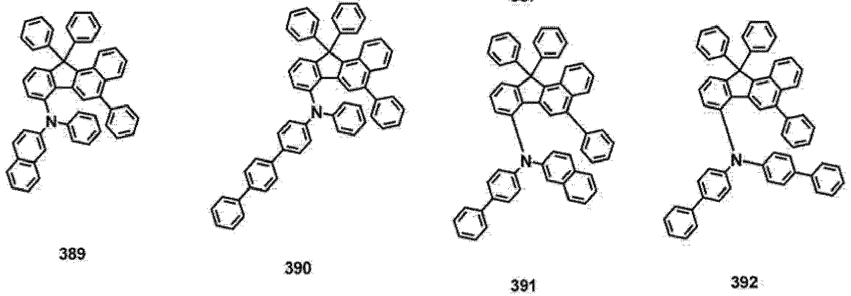

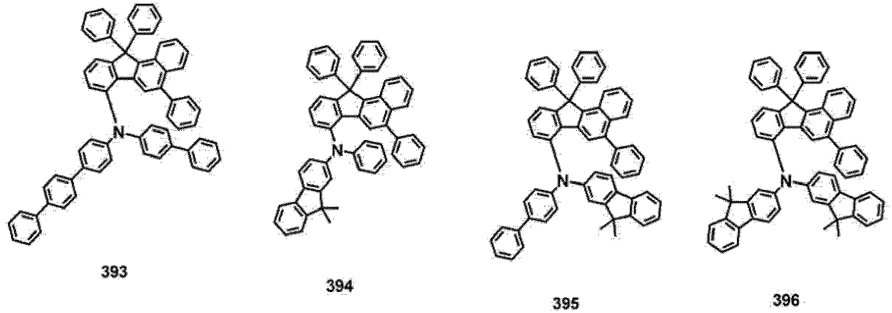

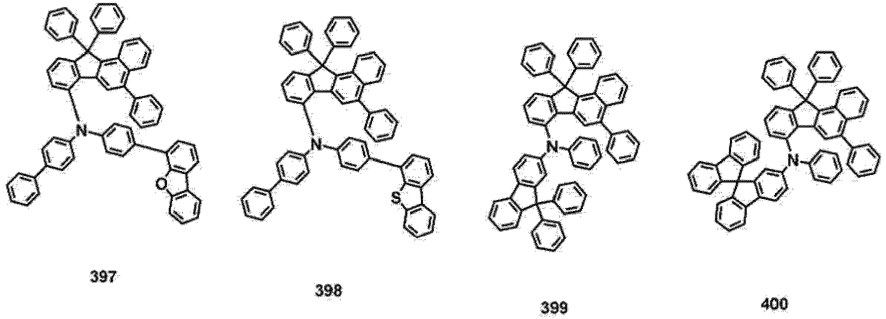

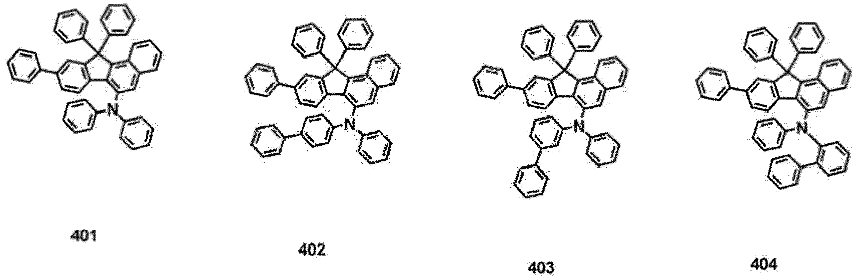

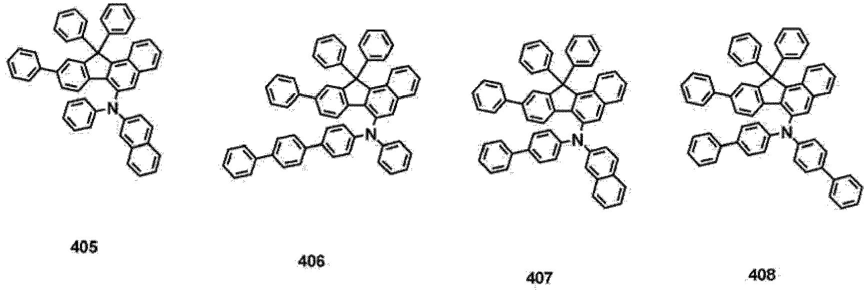

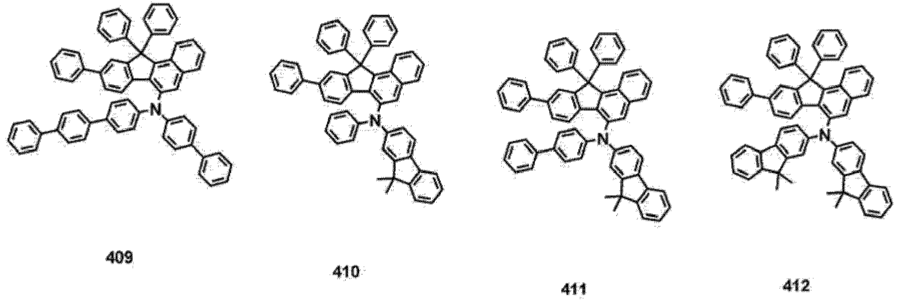

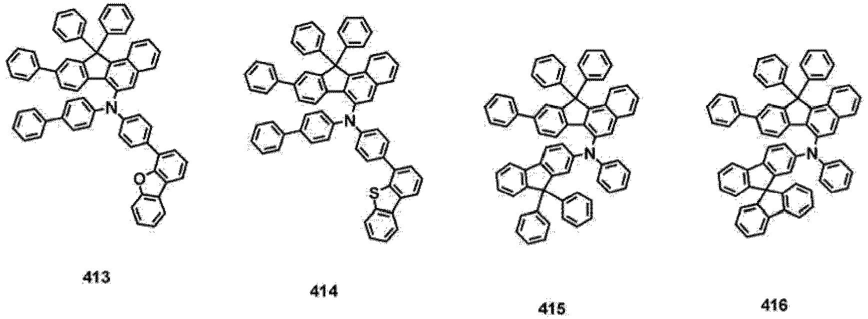

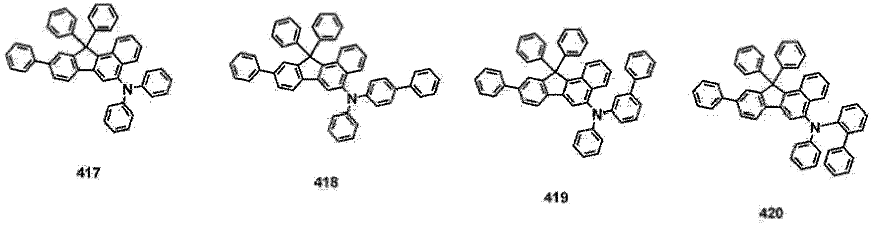

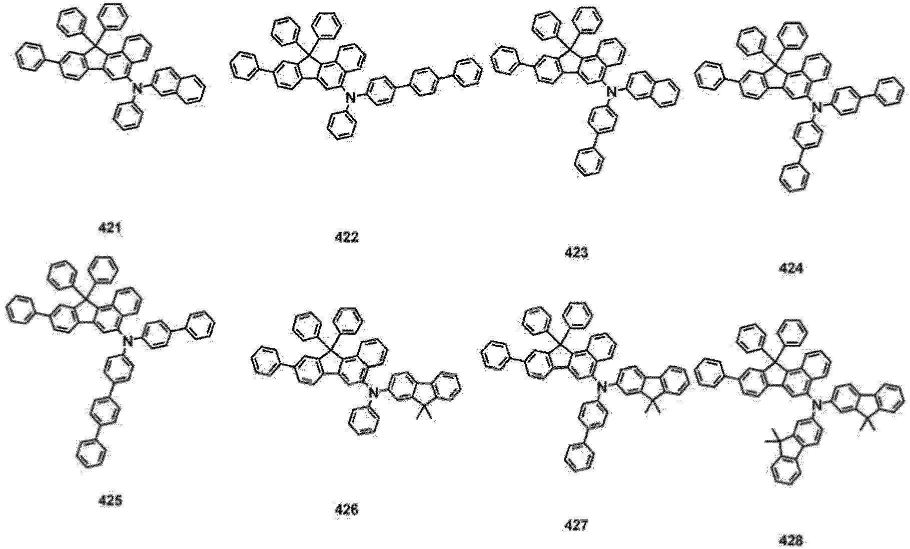

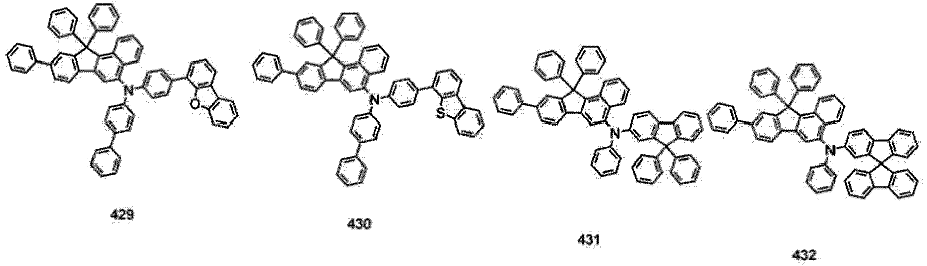

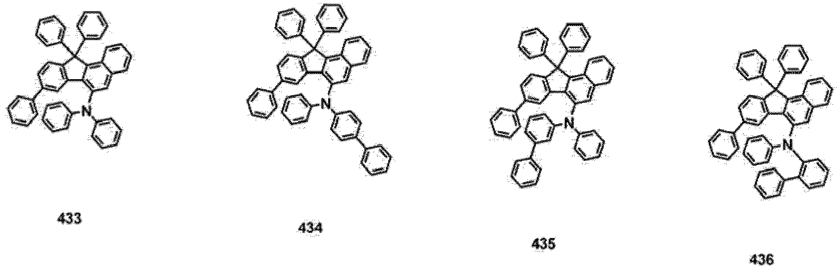

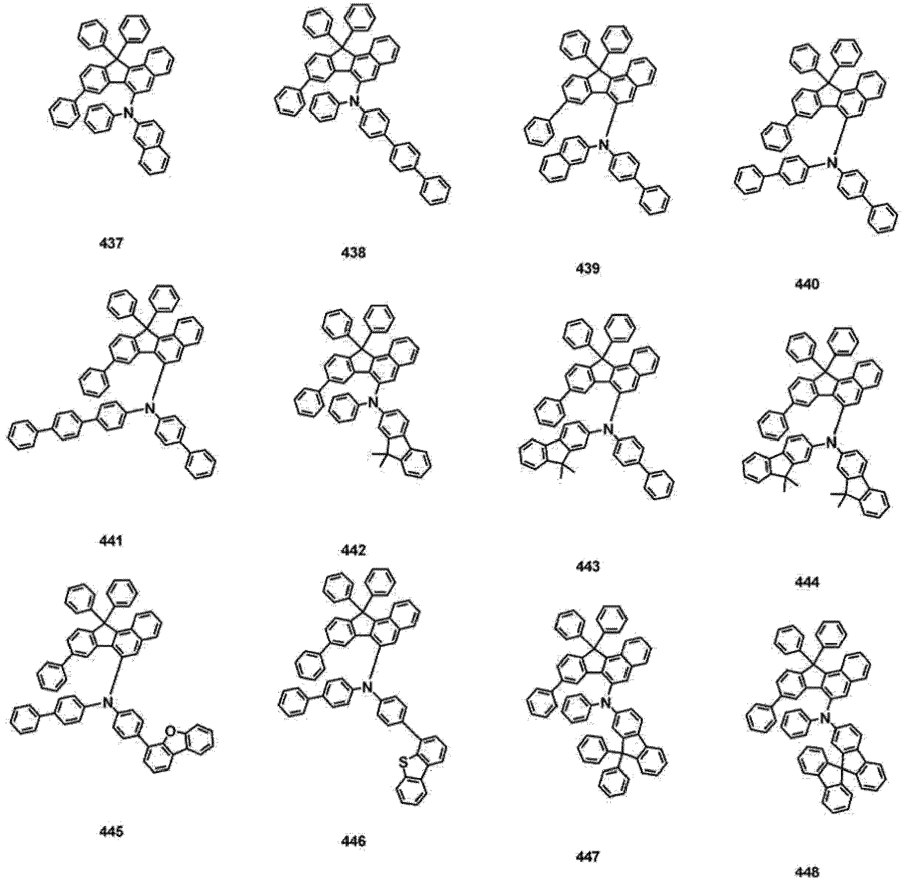

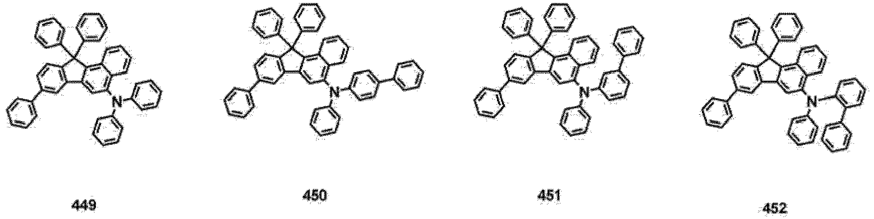

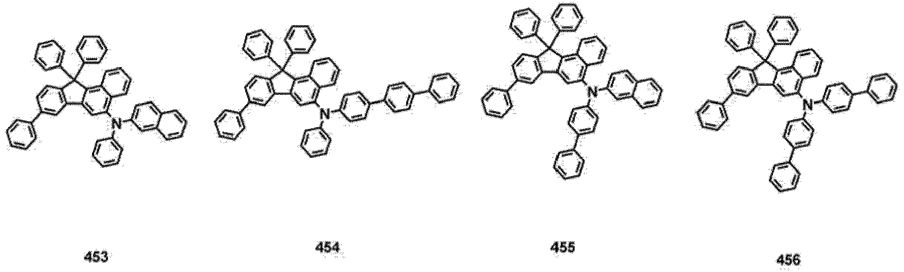

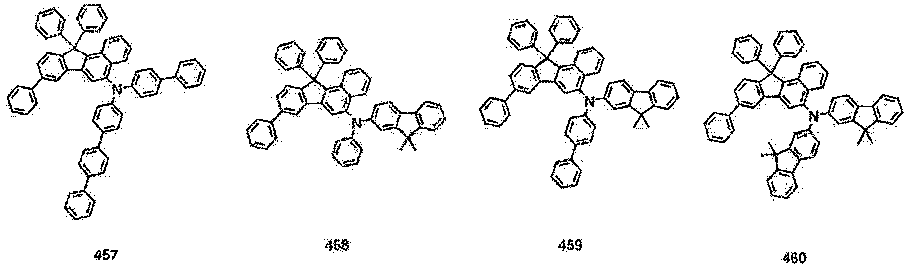

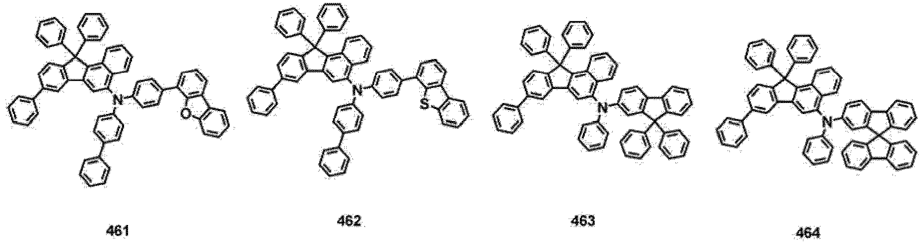

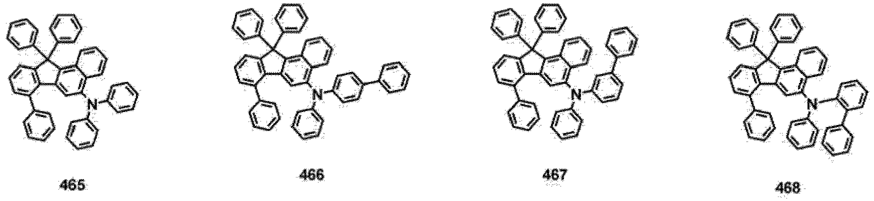

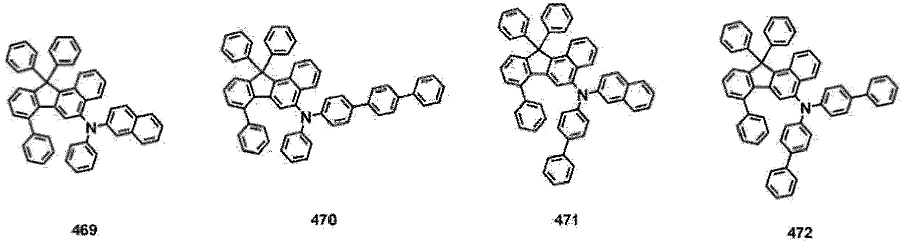

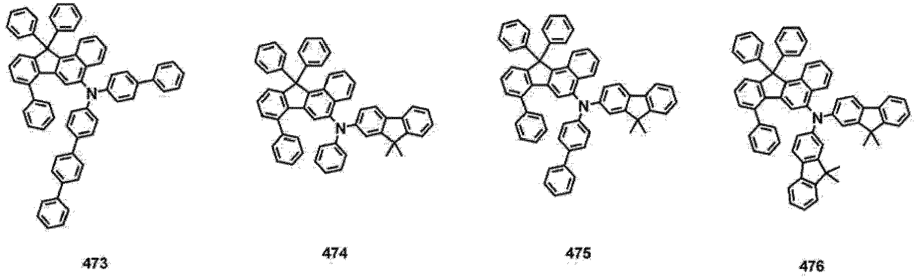

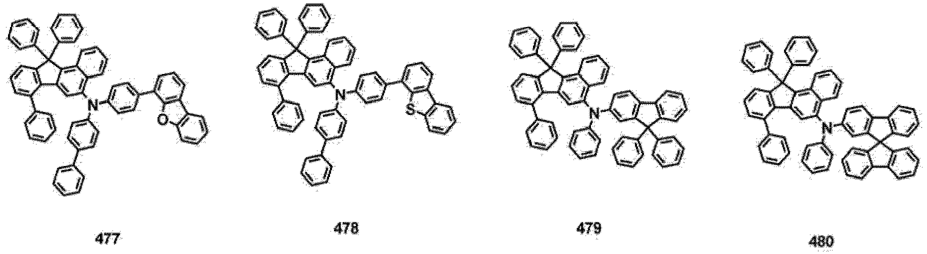

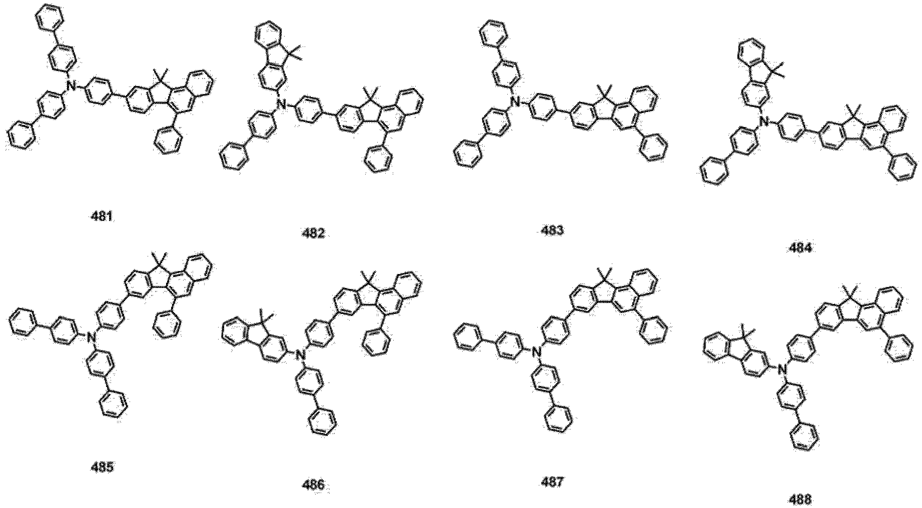

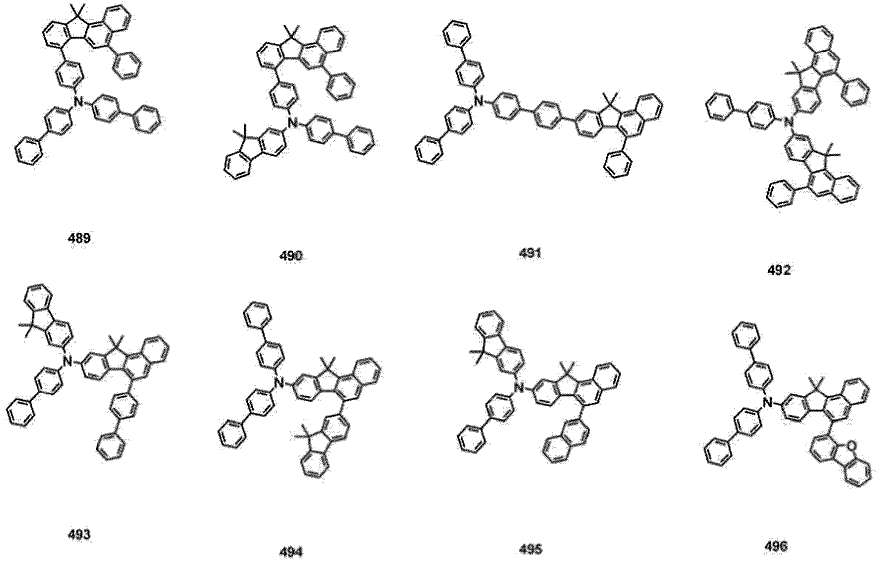

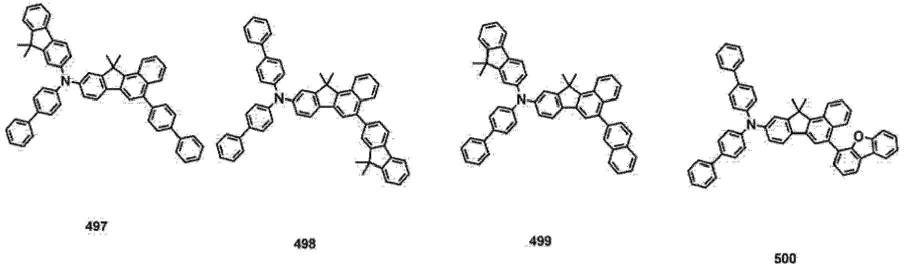

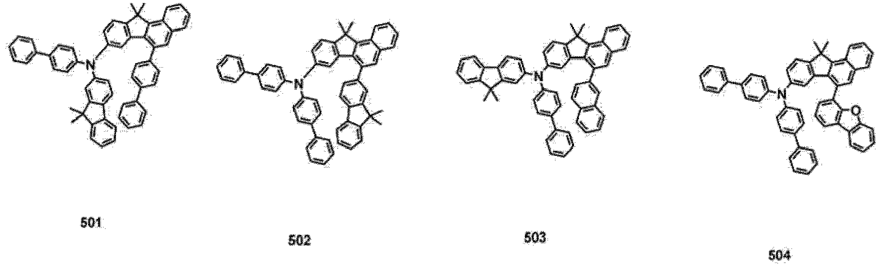

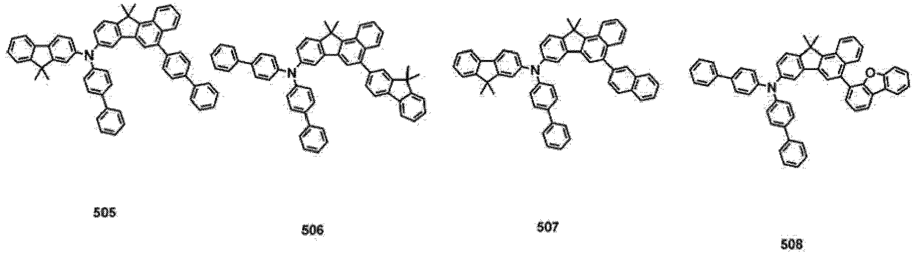

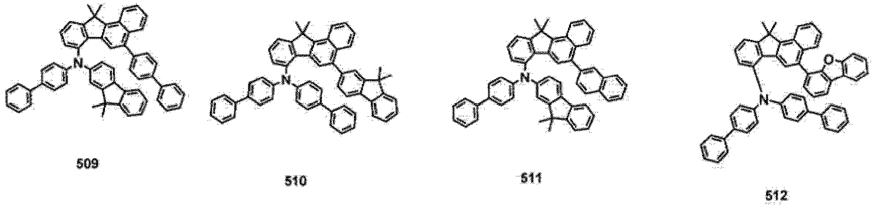

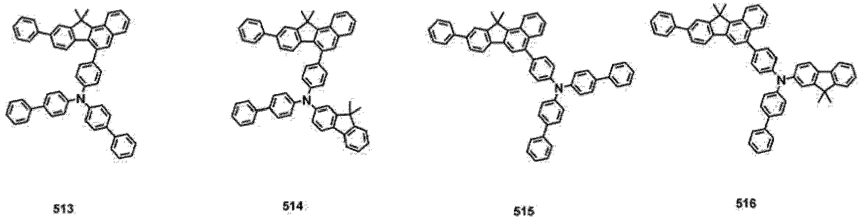

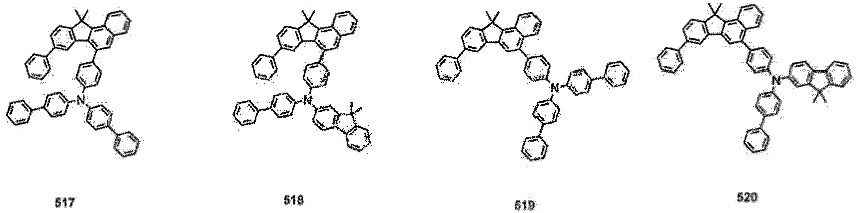

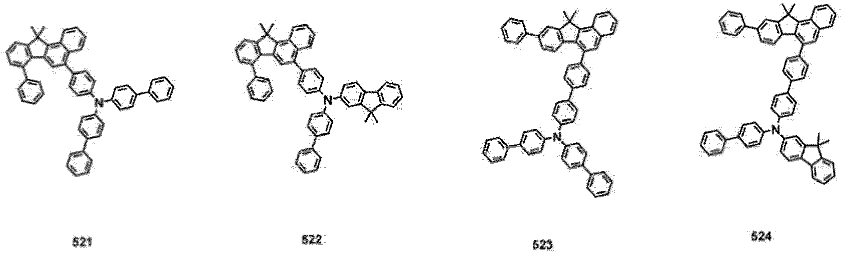

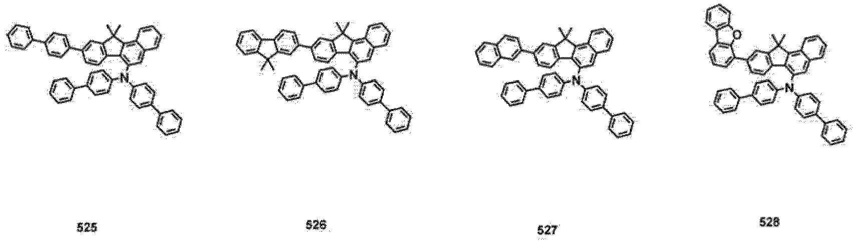

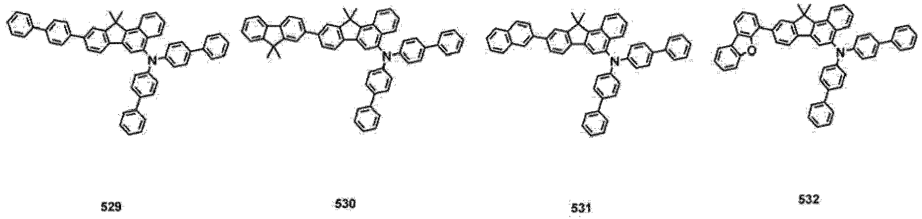

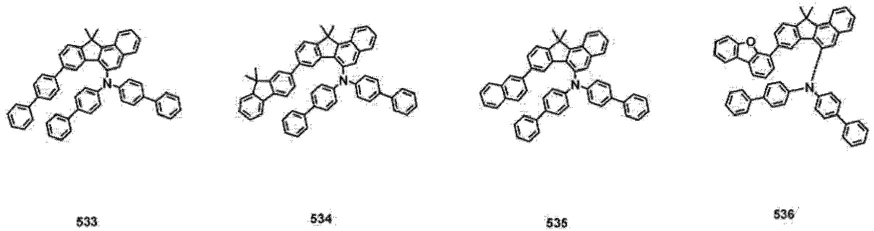

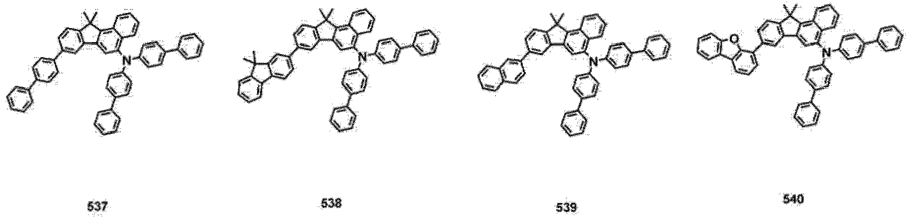

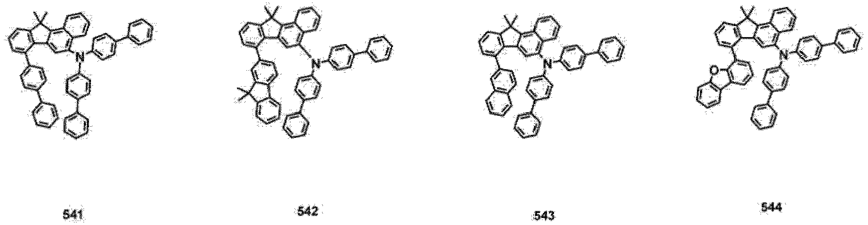

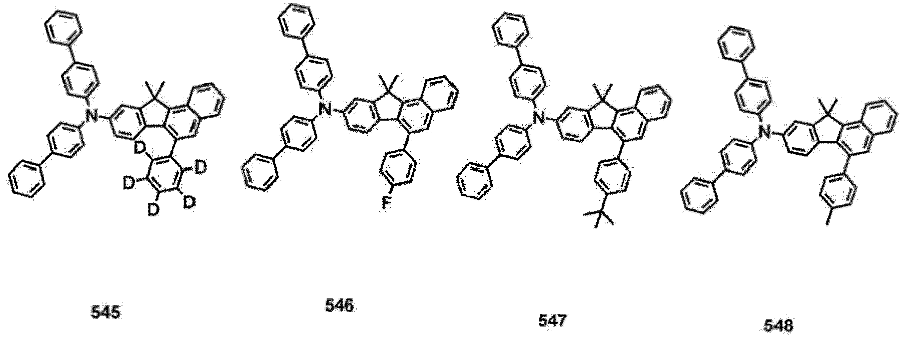

Further, various substituents may be introduced into the structure of Chemical Formula 1 to synthesize a compound having inherent characteristics of a substituent introduced. For example, it is possible to synthesize a material which satisfies conditions required for each organic material layer by introducing a substituent usually used for a hole injection layer material, a material for transporting holes, a light emitting layer material, an electron transport layer material, and a charge generation layer material, which are used for preparing an organic light emitting device, into the core structure.

In addition, it is possible to finely adjust an energy band-gap by introducing various substituents into the structure of Chemical Formula 1, and meanwhile, it is possible to improve characteristics at the interface between organic materials and diversify the use of the material.

Furthermore, in an exemplary embodiment of the present application, provided is an organic light emitting device including a first electrode; a second electrode provided to face the first electrode; and an organic material layer having one or more layers provided between the first electrode and the second electrode, in which one or more layers of the organic material layer include the heterocyclic compound according to Chemical Formula 1.

In another exemplary embodiment, provided is an organic light emitting device including: a first electrode; a second electrode provided to face the first electrode; and an organic material layer having one or more layers provided between the first electrode and the second electrode, in which one or more layers of the organic material layer include one heterocyclic compound according to Chemical Formula 1.

The specific content on the heterocyclic compound represented by Chemical Formula 1 is the same as that described above.

In an exemplary embodiment of the present application, the first electrode may be a positive electrode, and the second electrode may be a negative electrode.

In another exemplary embodiment, the first electrode may be a negative electrode, and the second electrode may be a positive electrode.

In an exemplary embodiment of the present application, the organic light emitting device may be a blue organic light emitting device, and the heterocyclic compound according to Chemical Formula 1 may be used as a material for the blue organic light emitting device. For example, the heterocyclic compound according to Chemical Formula 1 may be included in a host material of a blue light emitting layer of a blue organic light emitting device.

In an exemplary embodiment of the present application, the organic light emitting device may be a green organic light emitting device, and the heterocyclic compound according to Chemical Formula 1 may be used as a material for the green organic light emitting device. For example, the heterocyclic compound according to Chemical Formula 1 may be included in a host material of a green light emitting layer of a green organic light emitting device.

In an exemplary embodiment of the present application, the organic light emitting device may be a red organic light emitting device, and the heterocyclic compound according to Chemical Formula 1 may be used as a material for the red organic light emitting device. For example, the heterocyclic compound according to Chemical Formula 1 may be included in a host material of a red light emitting layer of a red organic light emitting device.

The organic light emitting device of the present invention may be manufactured using typical manufacturing methods and materials of an organic light emitting device, except that the above-described heterocyclic compound is used to form an organic material layer having one or more layers.

The heterocyclic compound may be formed as an organic material layer by not only a vacuum deposition method, but also a solution application method when an organic light emitting device is manufactured. Here, the solution application method means spin coating, dip coating, inkjet printing, screen printing, a spray method, roll coating, and the like, but is not limited thereto.

The organic material layer of the organic light emitting device of the present invention may be composed of a single-layered structure, but may be composed of a multilayered structure in which two or more organic material layers are stacked. For example, the organic light emitting device of the present invention may have a structure including a hole injection layer, a hole transport layer, a light emitting layer, an electron transport layer, an electron injection layer, and the like as organic material layers. However, the structure of the organic light emitting device is not limited thereto, and may include a fewer number of organic material layers.

In the organic light emitting device of the present invention, the organic material layer may include a light emitting layer, and the light emitting layer may include the heterocyclic compound.

In another organic light emitting device, the organic material layer includes a light emitting layer, the light emitting layer includes a host material, and the host material may include the heterocyclic compound.

As another example, the organic material layer including the heterocyclic compound includes the heterocyclic compound represented by Chemical Formula 1 as a host, and the heterocyclic compound may be used with an iridium-based dopant.

In the organic light emitting device of the present invention, the organic material layer includes an electron injection layer or an electron transport layer, and the electron transport layer or the electron injection layer may include the heterocyclic compound.

In another organic light emitting device, the organic material layer includes an electron blocking layer or a hole blocking layer, and the electron blocking layer or the hole blocking layer may include the heterocyclic compound.

In the organic light emitting device of the present invention, the organic material layer includes a hole transport layer, and the hole transport layer may include the heterocyclic compound.

The organic light emitting device of the present invention may further include one or two or more layers selected from the group consisting of a light emitting layer, a hole injection layer, a hole transport layer, an electron injection layer, an electron transport layer, an electron blocking layer, and a hole blocking layer.

FIGS. 1 to 3 exemplify the stacking sequence of the electrodes and the organic material layer of the organic light emitting device according to an exemplary embodiment of the present application. However, the scope of the present application is not intended to be limited by these drawings, and the structure of the organic light emitting device known in the art may also be applied to the present application.

According to FIG. 1, an organic light emitting device in which a positive electrode 200, an organic material layer 300, and a negative electrode 400 are sequentially stacked on a substrate 100 is illustrated. However, the organic light emitting device is not limited only to such a structure, and as in FIG. 2, an organic light emitting device in which a negative electrode, an organic material layer, and a positive electrode are sequentially stacked on a substrate may also be implemented.

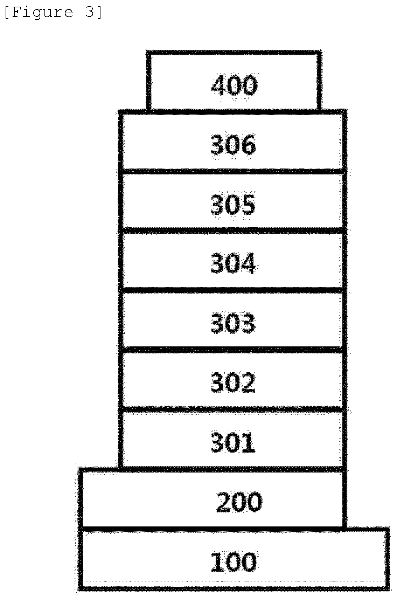

FIG. 3 exemplifies a case where an organic material layer is a multilayer. An organic light emitting device according to FIG. 3 includes a hole injection layer 301, a hole transport layer 302, a light emitting layer 303, a hole blocking layer 304, an electron transport layer 305, and an electron injection layer 306. However, the scope of the present application is not limited by the stacking structure as described above, and if necessary, the other layers except for the light emitting layer may be omitted, and another necessary functional layer may be further added.

An organic material layer including the compound of Chemical Formula 1 may additionally include other materials, if necessary.

In the organic light emitting device according to an exemplary embodiment of the present application, materials other than the heterocyclic compound of Chemical Formula 1 will be exemplified below, but these materials are illustrative only and are not for limiting the scope of the present application, and may be replaced with materials publicly known in the art.

As a positive electrode material, materials having a relatively high work function may be used, and a transparent conductive oxide, a metal or a conductive polymer, and the like may be used. Specific examples of the positive electrode material include: a metal such as vanadium, chromium, copper, zinc, and gold, or an alloy thereof; a metal oxide such as zinc oxide, indium oxide, indium tin oxide (ITO), and indium zinc oxide (IZO); a combination of a metal and an oxide, such as ZnO:Al or SnO₂:Sb; a conductive polymer such as poly(3-methylthiophene), poly[3,4-(ethylene-1,2-dioxy)thiophene] (PEDOT), polypyrrole, and polyaniline; and the like, but are not limited thereto.

As a negative electrode material, materials having a relatively low work function may be used, and a metal, a metal oxide, or a conductive polymer, and the like may be used. Specific examples of the negative electrode material include: a metal such as magnesium, calcium, sodium, potassium, titanium, indium, yttrium, lithium, gadolinium, aluminum, silver, tin, and lead, or an alloy thereof; a multi-layer structured material, such as LiF/Al or LiO₂/Al; and the like, but are not limited thereto.

As a hole injection material, a publicly-known hole injection material may also be used, and it is possible to use, for example, a phthalocyanine compound such as copper phthalocyanine disclosed in US Patent No. 4,356,429 or starburst-type amine derivatives described in the document [Advanced Material, 6, p. 677 (1994)], for example, tris(4-carbazoyl-9-ylphenyl)amine (TCTA), 4,4',4"-tri[phenyl(m-tolyl)amino]triphenylamine (m-MTDATA), 1,3,5-tris[4-(3-methylphenylphenylamino)phenyl]benzene (m-MTDAPB), polyaniline/dodecylbenzenesulfonic acid or poly(3,4-ethylenedioxythiophene)/poly(4-styrenesulfonate), which is a soluble conductive polymer, polyaniline/camphor sulfonic acid or polyaniline/poly(4-styrene-sulfonate), and the like.

As a hole transport material, a pyrazoline derivative, an arylamine-based derivative, a stilbene derivative, a triphenyldiamine derivative, and the like may be used, and a low-molecular weight or polymer material may also be used.

As an electron transport material, it is possible to use an oxadiazole derivative, anthraquinodimethane and a derivative thereof, benzoquinone and a derivative thereof, naphthoquinone and a derivative thereof, anthraquinone and a derivative thereof, tetracyanoanthraquinodimethane and a derivative thereof, a fluorenone derivative, diphenyldicyanoethylene and a derivative thereof, a diphenoquinone derivative, a metal complex of 8-hydroxyquinoline and a derivative thereof, and the like, and a low-molecular weight material and a polymer material may also be used.

As an electron injection material, for example, LiF is representatively used in the art, but the present application is not limited thereto.

As a light emitting material, a red, green, or blue light emitting material may be used, and if necessary, two or more light emitting materials may be mixed and used. In this case, two or more light emitting materials are deposited or used as an individual supply source, or premixed to be deposited and used as one supply source. Further, a fluorescent material may also be used as the light emitting material, but may also be used as a phosphorescent material. As the light emitting material, it is also possible to use alone a material which emits light by combining holes and electrons each injected from a positive electrode and a negative electrode, but materials in which a host material and a dopant material are involved in light emission together may also be used.

When hosts of the light emitting material are mixed and used, the same series of hosts may also be mixed and used, and different series of hosts may also be mixed and used. For example, two or more types of materials selected from n-type host materials or p-type host materials may be used as a host material for a light emitting layer.

The organic light emitting device according to an exemplary embodiment of the present application may be a top emission type, a bottom emission type, or a dual emission type according to the material to be used.

The heterocyclic compound according to an exemplary embodiment of the present application may act even in organic electronic devices including organic solar cells, organic photoconductors, organic transistors, and the like, based on the principle similar to those applied to organic light emitting devices.

### [Mode for Invention]

Hereinafter, the present specification will be described in more detail through Examples, but these Examples are provided only for exemplifying the present application, and are not intended to limit the scope of the present application.

### <Preparation Example 1> Preparation of Compound 2

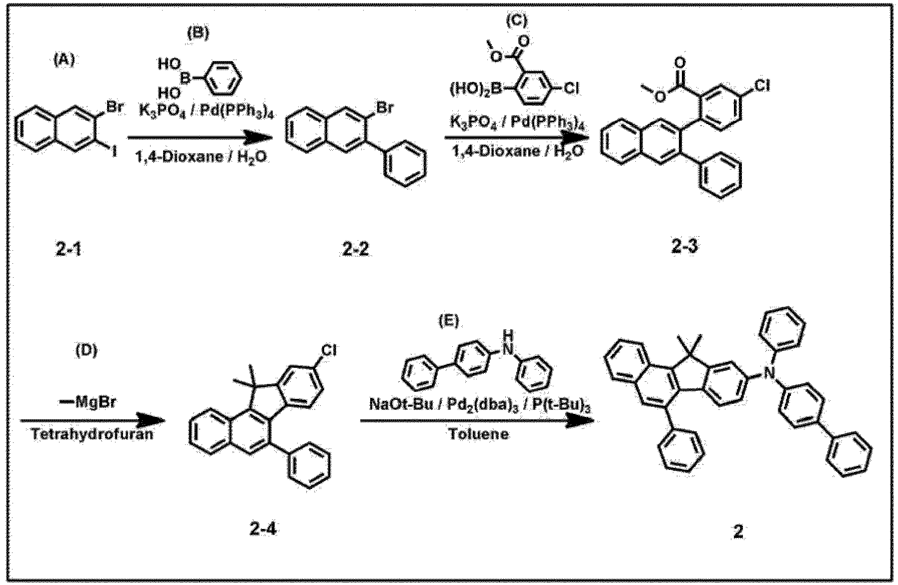

### Preparation of Compound 2-2

1,4-dioxane (400 ml) and H₂O (100 ml) were put into 2-bromo-3-iodonaphthalene (A) (20 g, 0.060 mol, 1 eq), phenylboronic acid (B) (8 g, 0.066 mol, 1.1 eq), K₃PO₄ (38 g, 0.18 mol, 3 eq), and Pd(PPh₃)₄ (3.4 g, 0.003 mol, 0.05 eq), and the resulting mixture was stirred at 100°C for 6 hours. After the reaction was terminated by adding water, extraction was performed using MC and water. Thereafter, moisture was removed with MgSO₄. The residue was separated by silica gel column to obtain 15 g of Compound 2-2 with a yield of 88%.

### Preparation of Compound 2-3

1,4-dioxane (300 ml) and H₂O (750 ml) were put into Compound 2-2 (15 g, 0.053 mol, 1 eq), (4-chloro-2-(methoxycarbonyl)phenyl)boronic acid (C) (12.5 g, 0.058 mol, 1.1 eq), K₃PO₄ (33.7 g, 0.159 mol, 3 eq), and Pd(PPh₃)₄ (3 g, 0.003 mol, 0.0026 eq), and the resulting mixture was stirred at 100°C for 6 hours. After the reaction was terminated by adding water, extraction was performed using MC and water. Thereafter, moisture was removed with MgSO₄. The residue was separated by silica gel column to obtain 15 g of Compound 2-3 with a yield of 76%.

### Preparation of Compound 2-4

After Compound 2-3 (15 g, 0.040 mol, 1 eq) was dissolved in tetrahydrofuran (150 ml), methylmagnesium bromide (3M solution in ether) (D) (40 ml, 0.120 mol, 3 eq) was slowly added thereto at 0°C, and then the resulting mixture was stirred at 60°C for hours. After the reaction was terminated by adding water, extraction was performed using MC and water. Thereafter, moisture was removed with MgSO₄. After boron trifluoride diethyl etherate was added to the reaction product dissolved in MC, the resulting mixture was stirred at RT for 4 hours. The mixture was separated by silica gel column to obtain 10 g of Compound 2-4 with a yield of 70%.

### Preparation of Compound 2

P(t-Bu)₃ (0.57 g, 0.0028 mol, 0.1 eq) and toluene (150 ml) were put into Compound 2-4 (10 g, 0.028 mol, 1 eq), N-phenyl-[1,1'-biphenyl]-4-amine (7.2 g, 0.029 mol, 1.05 eq), NaOt-Bu (4 g, 0.042 mol, 1.5 eq), and Pd(dba₂)₃ (1.3 g, 0.0014 mol, 0.05 eq), and the resulting mixture was stirred at 100°C for 8 hours. After the reaction was terminated by adding water, extraction was performed using MC and water. Thereafter, moisture was removed with MgSO₄. The residue was separated by silica gel column to obtain 11 g of Compound 2 with a yield of 69%.

Compound 2 was synthesized in the same manner as in the preparation of Preparation Example 1, except that Intermediates A, B, C, D, and E in the following Table 1 were used instead of 2-bromo-3-iodonaphthalene (A), phenylboronic acid (B), (4-chloro-2-(methoxycarbonyl)phenyl)boronic acid (C), methylmagnesium bromide (3M solution in ether) (D), and N-phenyl-[1,1'-biphenyl]-4-amine (E) in Preparation Example 1.

**[Table 1]**

| Com pound number | Intermediate A | IntermediateB | IntermediateC | IntermediateD | IntermediateE | Yield |
|---|---|---|---|---|---|---|
| 10 | 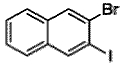 | 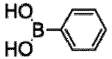 | 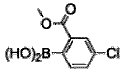 | -MgBr | 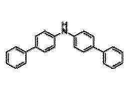 | 4 2% |
| 14 | 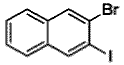 | 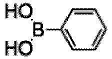 | 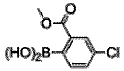 | -MgBr | 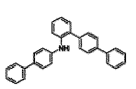 | 47% |
| 16 | 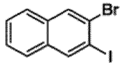 | 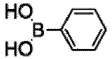 | 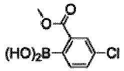 | -MgBr | 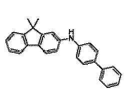 | 40% |
| 19 | 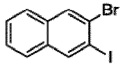 | 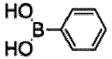 | 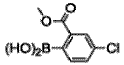 | -AAger | 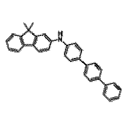 | 48% |
| 27 | 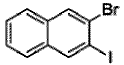 | 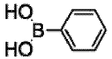 | 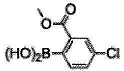 | -MgBr | 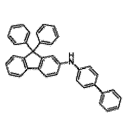 | 49% |
| 37 | 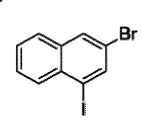 | 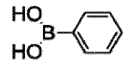 | 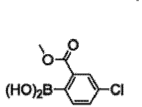 | -MgBr | 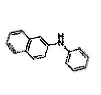 | 41 % |
| 49 | 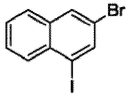 | 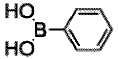 | 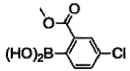 | -MgBr | 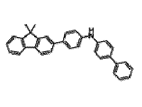 | 42% |
| 53 | 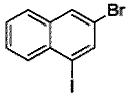 | 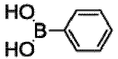 | 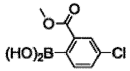 | -MgBr | 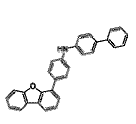 | 40% |
| 67 | 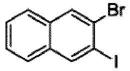 | 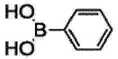 | 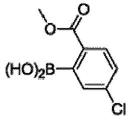 | -MgBr | 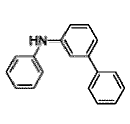 | 40% |
| 88 | 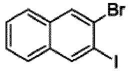 | 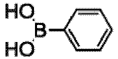 | 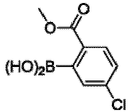 | -Mger | 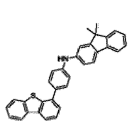 | 44% |
| 92 | 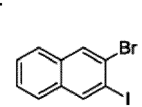 | 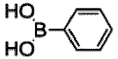 | 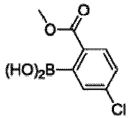 | -MgBr | 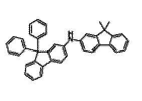 | 52% |
| 106 | 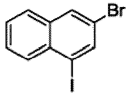 | 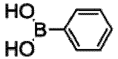 | 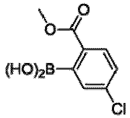 | -MgBr | 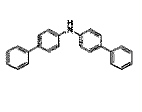 | 47% |
| 135 | 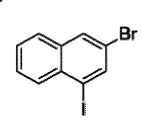 | 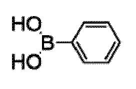 | 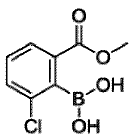 | -MgBr | 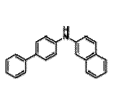 | 51 % |
| 142 | 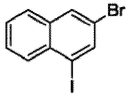 | 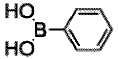 | 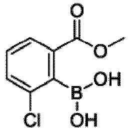 | -MgBr | 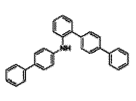 | 47% |
| 146 | 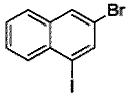 | 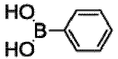 | 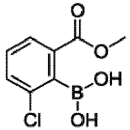 | -MgBr | 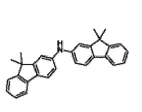 | 50% |
| 149 | 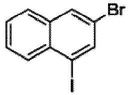 | 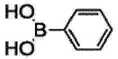 | 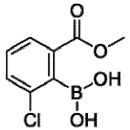 | -MgBr | 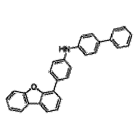 | 53% |
| 159 | 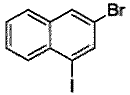 | 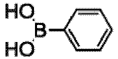 | 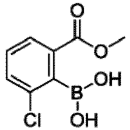 | -Mger | 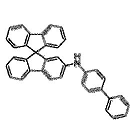 | 49% |
| 166 | 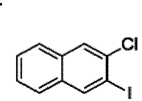 | 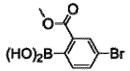 | 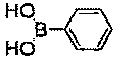 | -MgBr | 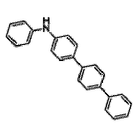 | 45% |
| 172 | 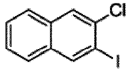 | 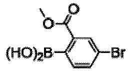 | 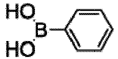 | -MgBr | 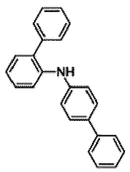 | 52% |
| 176 | 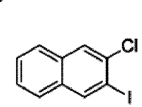 | 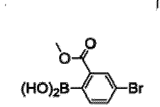 | 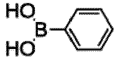 | -MgBr | 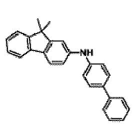 | 55% |
| 188 | 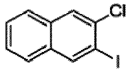 | 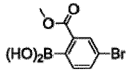 | 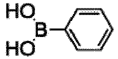 | -MgBr | 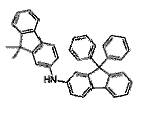 | 50% |
| 220 | 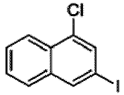 | 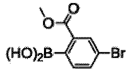 | 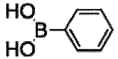 | -MgBr | 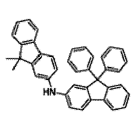 | 40% |
| 234 | 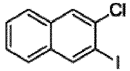 | 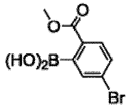 | 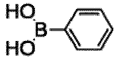 | -MgBr | 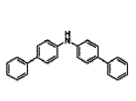 | 46% |
| 240 | 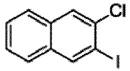 | 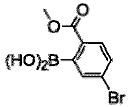 | 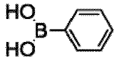 | -MgBr | 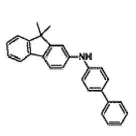 | 43% |
| 243 | 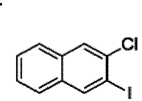 | 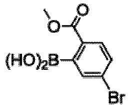 | 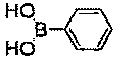 | -MgBr | 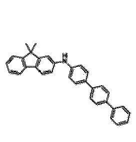 | 51% |
| 331 | 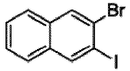 | 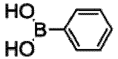 | 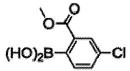 | 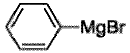 | 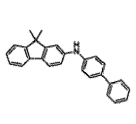 | 46% |
| 332 | 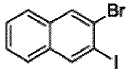 | 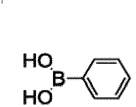 | 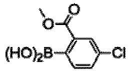 | 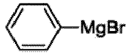 | 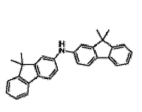 | 43% |
| 493 | 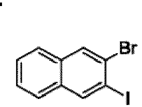 | 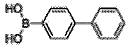 | 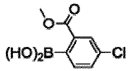 | -MgBr | 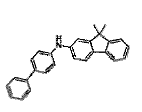 | 47% |
| 494 | 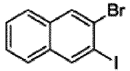 | 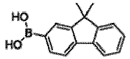 | 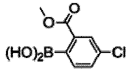 | -MgBr | 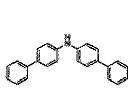 | 50% |

Heterocyclic compounds corresponding to Chemical Formula 1 other than the compounds described in Preparation Example 1 and Table 1 were also prepared in the same manner as in the methods described in the above-described preparation examples.

The synthetic confirmation data of the compounds prepared above are as shown in the following [Table 2] and [Table 3]. Table 2 shows the measured values of ¹H NMR(CDCl₃, 200 Mz), and Table 3 shows the measured values of field desorption mass spectrometry (FD-MS).

**[Table 2]**

| Compound | ¹H NMR(CDCl₃, 200Mz) |
|---|---|
| 2 | δ= 8.18(1H, d), 8.05(1H, d), 7.79(4H, m), 7.51(12H, m), 7.20 (2H, m), 6.81(2H, m), 6.63(5H, m) 1.85(6H, s) |
| 10 | δ= 8.18(1H, d), 8.05(1H, d), 7.79(4H, m), 7.51(19H, m), 6.81(1H, s), 6.69(5H, m) 1.85(6H, s) |
| 14 | δ= 8.18(1H, d), 8.05(1H, d), 7.79(4H, m), 7.51(18H, m), 7.25(4H, m), 7.16(1H, t), 6.87(1H, t), 6.81(1H, s), 6.69(4H, m), 1.85(6H, s) |
| 16 | δ= 8.18(1H, d), 8.05(1H, d), 7.79(5H, m), 7.51(15H, m), 7.28(1H, t), 6.81(1H, s), 6.75(1H, s), 6.69(4H, m), 1.85(6H, s), 1.72(6H, s) |
| 19 | δ= 8.18(1H, d), 8.05(1H, d), 7.79(5H, m), 7.51(15H, m), 7.28(1H, t), 7.25(4H, m), 6.81(1H, s), 6.75(1H, s), 6.69(4H, m), 1.85(6H, s), 1.72(6H, s) |
| 27 | δ= 8.18(1H, d), 8.05(1H, d), 7.79(5H, m), 7.54~7.26(22H, m), 7.11(4H, m), 6.81(1H, s), 6.75(1H, s), 6.69(4H, m), 1.85(6H, s), |
| 37 | δ= 8.52(1H, d), 8.18(1H, d), 7.84(7H, m), 7.63(1H, s), 7.51(8H, m), 7.20(2H, m), 6.81(2H, m), 6.63(3H, m), 1.85(6H, s), |
| 49 | δ= 8.52(1H, d), 8.18(1H, d), 7.84(6H, m), 7.63(2H, m), 7.51(16H, m), 7.28(1H, t), 6.81(1H, s), 6.69(5H, m), 1.85(6H, s), 1.72(6H, s) |
| 53 | δ= 8.52(1H, d), 8.18(1H, d), 7.84(6H, m), 7.66(1H, m), 7.63(1H, s), 7.52~7.32(17H, m), 6.81(1H, s), 6.69(5H, m), 1.85(6H, s) |
| 67 | δ= 8.18(1H, d), 8.05(1H, d), 7.79(3H, m), 7.52(12H, m), 7.26(1H, s), 7.20(2H, m), 6.88(2H, m), 6.81(1H, t), 6.63(3H, m), 6.44(1H, d), 1.85(6H, s), |
| 88 | δ= 8.45(2H, m), 8.20(2H, m), 8.05(2H, m), 7.87(1H, d), 7.79(3H, m), 7.54~7.28(16H, m), 6.75(1H, s), 6.69(2H, m), 6.58(1H, d), 6.44(1H, d), 1.85(6H, s), 1.72(6H, s) |
| 92 | δ= 8.18(1H, d), 8.05(1H, d), 7.87(2H, m), 7.79(3H, m), 7.55(8H, m), 7.38(13H, m), 7.11(4H, m), 6.75(2H, m), 6.58(2H, m), 6.44(1H, d), 1.85(6H, s), 1.72(6H, s) |
| 106 | δ= 8.52(1H, d), 8.18(1H, d), 7.79(2H, m), 7.63(1H, s), 7.54(20H, m), 7.26(1H, s), 6.69(4H, m), 6.44(1H, d), 1.85(6H, s) |
| 135 | δ= 8.52(1H, d), 8.18(1H, d), 7.77(6H, m), 7.63(1H, s), 7.54(15H, m), 6.99(2H, m), 6.69(3H, m), 1.85(6H, s) |
| 142 | δ= 8.52(1H, d), 8.18(1H, d), 7.79(2H, m), 7.63(1H, s), 7.54(18H, m), 7.25(4H, m), 7.15(1H, t), 6.99(2H, m), 6.87(1H, t), 6.64 (4H, m), 1.85(6H, s) |
| 146 | δ= 8.52(1H, d), 8.18(1H, d), 7.87(2H, m), 7.79(2H, m), 7.55(9H, m), 7.38(3H, m), 7.28(2H, m), 6.99(2H, m), 6.75(2H, s), 6.58(3H, m), 1.85(6H, s), 1.72(12H, s) |
| 149 | δ= 8.52(1H, d), 8.18(1H, d), 7.85(5H, m), 7.66(1H, d), 7.63(1H, s), 7.51(17H, m), 6.99(2H, m), 6.64(5H, m), 1.85(6H, s) |
| 159 | δ= 8.52(1H, d), 8.18(1H, d), 7.87(2H, m), 7.79(3H, m), 7.63(1H, s), 7.54~7.28(20H, m), 7.19(2H, m), 6.99(2H, m), 6.64(3H, m), 6.55(1H, s), 6.39(1H, d), 1.85(6H, s) |
| 166 | δ= 8.15(1H, d), 8.06(1H, d), 7.82(2H, m), 7.69(1H, d), 7.51(13H, m), 7.31(7H, m), 6.81(1H, t), 6.69(5H, m), 1.85(6H, s) |
| 172 | δ= 8.15(1H, d), 8.06(1H, d), 7.82(2H, m), 7.69(1H, d), 7.51(17H, m), 7.31(1H, t), 7.08(3H, m), 6.87(1H, t), 6.69(4H, m), 1.85(6H, s) |
| 176 | δ= 8.15(1H, d), 8.06(1H, d), 7.87(3H, m), 7.69(1H, d), 7.62(1H, d), 7.51(17H, m), 6.70(4H, m), 6.58(1H, d), 1.85(6H, s), 1.72(6H, s) |
| 188 | δ= 8.15(1H, d), 8.06(1H, d), 7.83(4H, m), 7.55~7.26(22H, m), 7.11(4H, m), 6.75(3H, m), 6.58(2H, m), 1.85(6H, s), 1.72(6H, s) |
| 220 | δ= 8.12(2H, m), 8.04(1H, d), 7.88(9H, m), 7.52~7.16(20H, m), 6.65(1H, s), 6.55(1H, s), 6.39(1H, d), 1.85(6H, s), |
| 234 | δ= 8.28(1H, s), 8.06(1H, d), 7.82(1H, d), 7.67(1H, d), 7.52(21H, m), 7.31(1H, t), 6.70(5H, m), 1.85(6H, s), |
| 240 | δ= 8.28(1H, s), 8.06(1H, d), 7.82(2H, m), 7.67(2H, m), 7.52(18H, m), 6.69(4H, m), 6.58(1H, d), 1.85(6H, s), 1.72(6H, s), |
| 243 | δ= 8.28(1H, s), 8.06(1H, d), 7.82(2H, m), 7.67(2H, m), 7.54~7.25(22H, m), 6.69(4H, m), 6.58(1H, d), 1.85(6H, s), 1.72(6H, s), |
| 331 | δ= 8.18(1H, d), 8.05(1H, d), 7.79(5H, m), 7.54~7.28(22H, m), 7.11(4H, m), 6.81(1H, s), 6.75(1H, s), 6.69(4H, m), 1.72(6H, s), |
| 332 | δ= 8.18(1H, d), 8.05(1H, d), 7.87(6H, m), 7.52(8H, m), 7.33(11H, m), 7.11(4H, m), 6.81(1H, s), 6.75(2H, s), 6.58(3H, m), 1. 72 (12H, s), |
| 493 | δ= 8.18(1H, d), 8.05(1H, d), 7.84(3H, m), 7.54(17H, m), 7.25(5H, m), 6.81(1H, s), 6.71(1H, s), 6.69(4H, m), 1.85(6H, s), 1.72(6H, s), |
| 494 | δ= 8.18(1H, d), 8.05(1H, d), 7.77(5H, m), 7.63(1H, d), 7.52(18H, m), 7.28(1H, t), 6.81(1H, s), 6.69(5H, m), 1.85(6H, s), 1.72(6H, s), |

**[Table 3]**

| Compound | FD-MS | Compound | FD-MS |
|---|---|---|---|
| 2 | m/z= 563.73 | 10 | m/z= 639.82 |
| | (C43H33N=563.26) | | (C49H37N=639.29) |
| 14 | m/z= 715.92 | 16 | m/z= 679.89 |
| | (C55H41N=715.32) | | (C52H41N=679.32) |
| 19 | m/z= 755.98 | 27 | m/z= 804.03 |
| | (C58H45N=755.36) | | (C62H45N=803.36) |
| 37 | m/z= 537.69 | 49 | m/z= 755.98 |
| | (C41H31N=537.25) | | (C58H45N=755.36) |
| 53 | m/z= 729.90 | 67 | m/z= 563.73 |
| | (C55H39NO=729.30) | | (C43H33N=563.26) |
| 88 | m/z= 786.03 | 92 | m/z= 844.09 |
| | (C58H43NS=785.31) | | (C65H49N=843.39) |
| 106 | m/z= 639.82 | 135 | m/z= 613.79 |
| | (C49H37N=639.29) | | (C47H35N=613.28) |
| 142 | m/z= 715.92 | 146 | m/z= 719.95 |
| | (C55H41N=715.32) | | (C55H45N=719.36) |
| 149 | m/z= 729.90 | 159 | m/z= 786.03 |
| | (C55H39NO=729.30) | | (C58H43NS=785.31) |
| 166 | m/z= 639.82 | 172 | m/z= 639.82 |
| | (C49H37N=639.29) | | (C49H37N=639.29) |
| 176 | m/z= 679.89 | 188 | m/z= 844.09 |
| | (C52H41N=679.32) | | (C65H49N=843.39) |
| 220 | m/z= 775.97 | 234 | m/z= 639.82 |
| | (C60H41N=775.32) | | (C49H37N=639.29) |
| 240 | m/z= 679.89 | 243 | m/z= 755.98 |
| | (C52H41N=679.32) | | (C58H45N=755.36) |
| 331 | m/z= 804.03 | 332 | m/z= 844.09 |
| | (C62H45N=803.36) | | (C65H49N=843.39) |
| 493 | m/z= 755.98(C58H45N=755.36) | 494 | m/z= 755.98 |
| | | | (C58H45N=755.36) |

### <Experimental Example 1> - Manufacture of organic light emitting device

### 1) Manufacture of Organic Light Emitting Device

Trichloroethylene, acetone, ethanol, and distilled water were each sequentially used to ultrasonically wash a transparent electrode indium tin oxide (ITO) thin film obtained from glass for OLED (manufactured by Samsung-Corning Co., Ltd.) for 5 minutes, and then the ITO thin film was placed in isopropanol, stored, and then used. Next, the ITO substrate was disposed in a substrate folder of a vacuum deposition apparatus, and the following

4,4',4"-tris(N,N-(2-naphthyl)-phenylamino)triphenyl amine (2-TNATA) was placed in a cell in the vacuum deposition apparatus.

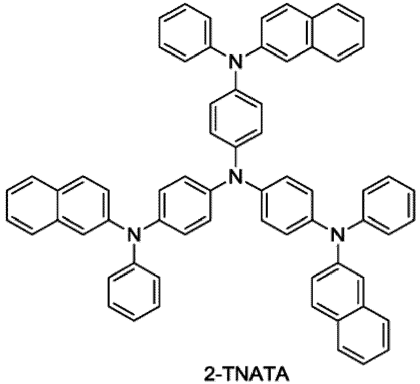

Subsequently, air in the chamber was evacuated until the degree of vacuum in the chamber reached 10⁻⁶ torr, and then a hole injection layer having a thickness of 600 Å was deposited on the ITO substrate by applying current to the cell to evaporate 2-TNATA. A hole transport layer having a thickness of 300 Å was deposited on the hole injection layer by placing the following N,N'-bis(α-naphthyl)-N,N'-diphenyl-4,4'-diamine (NPB) in another cell in the vacuum deposition apparatus and applying current to the cell to evaporate NPB.

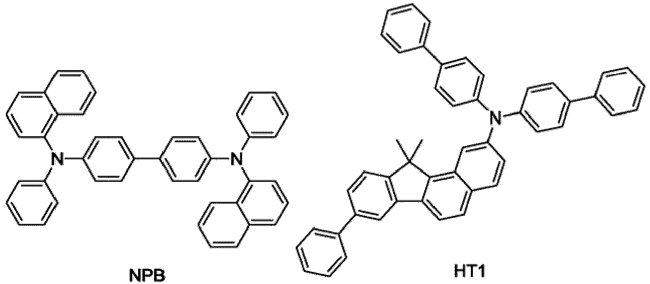

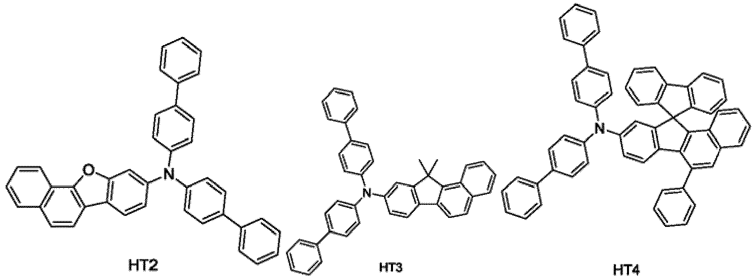

The hole injection layer and the hole transport layer were formed as described above, and then a blue light emitting material having the following structure as a light emitting layer was deposited thereon. Specifically, a blue light emitting host material H1 was vacuum deposited to have a thickness of 200 Å on one cell in the vacuum deposition apparatus, and a blue light emitting dopant material D1 was vacuum deposited thereon in an amount of 5% based on the host material.

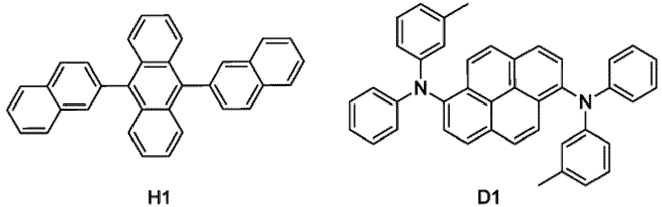

Subsequently, a compound having the following structural formula E1 as an electron transport layer was deposited to have a thickness of 300 Å.

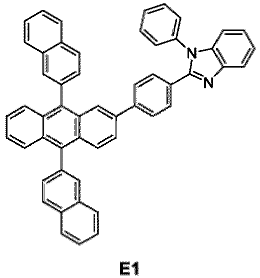

An OLED device was manufactured by depositing lithium fluoride (LiF) as an electron injection layer to have a thickness of 10 Å and allowing the Al negative electrode to have a thickness of 1,000 Å. Meanwhile, all the organic compounds required for manufacturing an OLED device were subjected to vacuum sublimed purification under 10⁻⁶ to 10⁻⁸ torr for each material, and used for the manufacture of OLED. An organic electroluminescence device was manufactured in the same manner as in Experimental Example 1, except that the compound in the following Table 4 was used instead of NPB used when a hole transport layer was formed in Experimental Example 1.

The results of measuring the driving voltage, light emitting efficiency, color coordinate (CIE) and service life of the blue organic light emitting device manufactured according to the present invention are shown in the following Table 4.

**[Table 4]**

| | Compound | Driving voltage (V) | Light emitting efficiency (cd/A) | CIE (x, y) | Service life (T95) |
|---|---|---|---|---|---|
| Example 1 | 1 | 4.81 | 6.73 | (0.134, 0.100) | 47 |
| Example 2 | 10 | 4.80 | 6.69 | (0.134, 0.100) | 48 |
| Example 3 | 14 | 4.78 | 6.75 | (0.134, 0.100) | 44 |
| Example 4 | 16 | 4.80 | 6.66 | (0.134, 0.100) | 47 |
| Example 5 | 19 | 4.81 | 6.71 | (0.134, 0.101) | 49 |
| Example 6 | 27 | 4.79 | 6.74 | (0.134, 0.100) | 46 |
| Example 7 | 37 | 4.80 | 6.70 | (0.134, 0.100) | 45 |
| Example 8 | 49 | 4.88 | 6.79 | (0.134, 0.100) | 46 |
| Example 9 | 53 | 4.87 | 6.69 | (0.134, 0.101) | 43 |
| Example 10 | 67 | 4.79 | 6.71 | (0.134, 0.100) | 43 |
| Example 11 | 88 | 4.81 | 6.70 | (0.134, 0.100) | 44 |
| Example 12 | 92 | 4.88 | 6.73 | (0.134, 0.101) | 51 |
| Example 13 | 106 | 4.83 | 6.66 | (0.134, 0.101) | 48 |
| Example 14 | 135 | 4.80 | 6.71 | (0.134, 0.100) | 47 |
| Example 15 | 142 | 4.90 | 6.73 | (0.134, 0.100) | 48 |
| Example 16 | 146 | 4.84 | 6.69 | (0.134, 0.101) | 51 |
| Example 17 | 149 | 4.88 | 6.60 | (0.134, 0.101) | 50 |
| Example 18 | 159 | 4.88 | 6.61 | (0.134, 0.100) | 44 |
| Example 19 | 166 | 4.87 | 6.63 | (0.134, 0.100) | 46 |
| Example 20 | 172 | 4.81 | 6.76 | (0.134, 0.101) | 46 |
| Example 21 | 176 | 4.84 | 6.73 | (0.134, 0.100) | 49 |
| Example 22 | 188 | 4.90 | 6.61 | (0.134, 0.101) | 50 |
| Example 23 | 220 | 4.91 | 6.63 | (0.134, 0.100) | 43 |
| Example 24 | 234 | 4.76 | 6.65 | (0.134, 0.101) | 45 |
| Example 25 | 240 | 4.74 | 6.66 | (0.134, 0.100) | 46 |
| Example 26 | 243 | 4.84 | 6.60 | (0.134, 0.100) | 48 |
| Example 27 | 331 | 4.80 | 6.70 | (0.134, 0.101) | 47 |
| Example 28 | 332 | 4.84 | 6.71 | (0.134, 0.100) | 47 |
| Example 29 | 493 | 4.87 | 6.72 | (0.134, 0.100) | 45 |
| Example 30 | 494 | 4.90 | 6.66 | (0.134, 0.100) | 48 |
| Comparative Example 1 | NPB | 5.51 | 6.06 | (0.134, 0.101) | 35 |
| Comparative Example 2 | HT1 | 5.36 | 6.15 | (0.134, 0.100) | 34 |
| Comparative Example 3 | HT2 | 5.38 | 6.17 | (0.134, 0.100) | 34 |
| Comparative Example 4 | HT3 | 5.44 | 6.11 | (0.134, 0.101) | 37 |
| Comparative Example 5 | HT4 | 5.38 | 6.08 | (0.134, 0.101) | 36 |

As can be seen from the results in Table 4, the organic light emitting device using a hole transport layer material of the blue organic light emitting device of the present invention has a low driving voltage and remarkably improved light emitting efficiency and service life compared to Comparative Examples 1 to 5.

Comparing the comparative examples in Table 4 with the compounds of the present invention, they are similar in having an arylamine group, but differ in that the fluorene group is substituted. A fluorene group with a substituent attached suppresses the pi-pi stacking of aromatic rings, and thus has a characteristic of preventing the device characteristics from deteriorating because the driving voltage of the organic light emitting device is increased. Therefore, it can be seen that the compound of the present invention using these derivatives enhances hole transport characteristics or stability to bring excellence in terms of all the aspects in driving, efficiency, and service life.

### <Experimental Example 2> - Manufacture of organic light emitting device

### 1) Manufacture of Organic Light Emitting Device

Trichloroethylene, acetone, ethanol, and distilled water were each sequentially used to ultrasonically wash a transparent electrode indium tin oxide (ITO) thin film obtained from glass for OLED (manufactured by Samsung-Corning Co., Ltd.) for 5 minutes, and then the ITO thin film was placed in isopropanol, stored, and then used. Next, the ITO substrate was disposed in a substrate folder of a vacuum deposition apparatus, and the following

4,4',4"-tris(N,N-(2-naphthyl)-phenylamino)triphenyl amine (2-TNATA) was placed in a cell in the vacuum deposition apparatus.

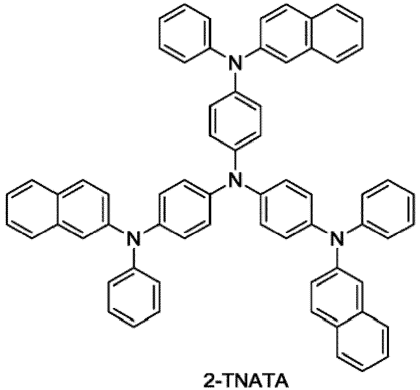

Subsequently, air in the chamber was evacuated until the degree of vacuum in the chamber reached 10⁻⁶ torr, and then a hole injection layer having a thickness of 600 Å was deposited on the ITO substrate by applying current to the cell to evaporate 2-TNATA. A hole transport layer having a thickness of 300 Å was deposited on the hole injection layer by placing the following N,N'-bis(α-naphthyl)-N,N'-diphenyl-4,4'-diamine (NPB) in another cell in the vacuum deposition apparatus and applying current to the cell to evaporate NPB.

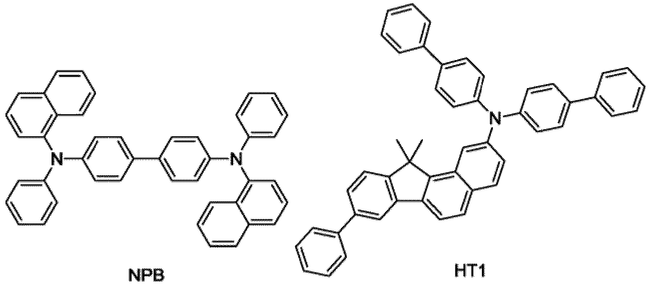

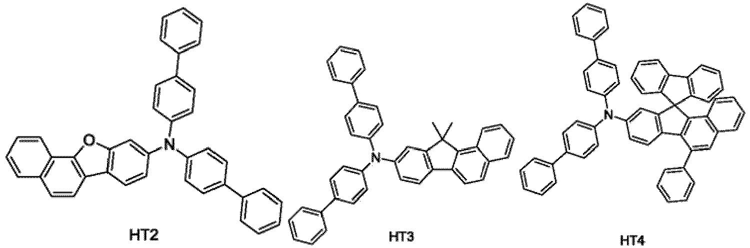

The hole injection layer and the hole transport layer were formed as described above, and then a blue light emitting material having the following structure as a light emitting layer was deposited thereon. Specifically, a blue light emitting host material H1 was vacuum deposited to have a thickness of 200 Å on one cell in the vacuum deposition apparatus, and a blue light emitting dopant material D1 was vacuum deposited thereon in an amount of 5% based on the host material.

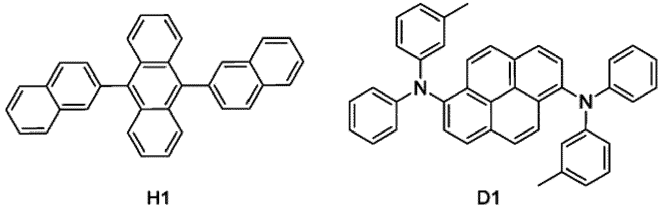

Subsequently, a compound having the following structural formula E1 as an electron transport layer was deposited to have a thickness of 300 Å.

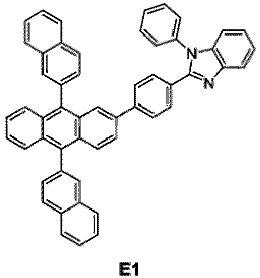

An OLED device was manufactured by depositing lithium fluoride (LiF) as an electron injection layer to have a thickness of 10 Å and allowing the Al negative electrode to have a thickness of 1,000 Å. Meanwhile, all the organic compounds required for manufacturing an OLED device were subjected to vacuum sublimed purification under 10⁻⁶ to 10⁻⁸ torr for each material, and used for the manufacture of OLED. An organic electroluminescence device was manufactured in the same manner as in Example 2, except that a hole transport layer NPB was formed to have a thickness of 250 Å, and then an electron blocking layer was formed to have a thickness of 50 Å from the compound denoted in the following Table 5 on the upper portion of the hole transport layer in Example 2. The results of measuring the driving voltage, light emitting efficiency, color coordinate (CIE) and service life of the blue organic light emitting device manufactured according to the present invention are shown in the following Table 5.

**[Table 5]**

| | Compound | Driving voltage (V) | Light emitting efficiency (cd/A) | CIE (x, y) | Service life (T95) |
|---|---|---|---|---|---|
| Example 31 | 1 | 4.81 | 6.73 | (0.134, 0.100) | 49 |
| Example 32 | 10 | 4.80 | 6.69 | (0.134, 0.100) | 48 |
| Example 33 | 14 | 4.78 | 6.75 | (0.134, 0.100) | 45 |
| Example 34 | 16 | 4.80 | 6.66 | (0.134, 0.100) | 47 |
| Example 35 | 19 | 4.81 | 6.71 | (0.134, 0.101) | 48 |
| Example 36 | 27 | 4.79 | 6.74 | (0.134, 0.100) | 48 |
| Example 37 | 37 | 4.80 | 6.70 | (0.134, 0.100) | 45 |
| Example 38 | 49 | 4.88 | 6.79 | (0.134, 0.100) | 42 |
| Example 39 | 53 | 4.87 | 6.69 | (0.134, 0.101) | 43 |
| Example 40 | 67 | 4.79 | 6.71 | (0.134, 0.100) | 46 |
| Example 41 | 88 | 4.81 | 6.70 | (0.134, 0.100) | 46 |
| Example 42 | 92 | 4.88 | 6.73 | (0.134, 0.101) | 51 |
| Example 43 | 106 | 4.83 | 6.66 | (0.134, 0.101) | 48 |
| Example 44 | 135 | 4.80 | 6.71 | (0.134, 0.100) | 47 |
| Example 45 | 142 | 4.90 | 6.73 | (0.134, 0.100) | 48 |
| Example 46 | 146 | 4.84 | 6.69 | (0.134, 0.101) | 51 |
| Example 47 | 149 | 4.88 | 6.60 | (0.134, 0.101) | 47 |
| Example 48 | 159 | 4.88 | 6.61 | (0.134, 0.100) | 43 |
| Example 49 | 166 | 4.87 | 6.63 | (0.134, 0.100) | 47 |
| Example 50 | 172 | 4.81 | 6.76 | (0.134, 0.101) | 44 |
| Example 51 | 176 | 4.84 | 6.73 | (0.134, 0.100) | 44 |
| Example 52 | 188 | 4.90 | 6.61 | (0.134, 0.101) | 50 |
| Example 53 | 220 | 4.91 | 6.63 | (0.134, 0.100) | 43 |
| Example 54 | 234 | 4.76 | 6.65 | (0.134, 0.101) | 49 |
| Example 55 | 240 | 4.74 | 6.66 | (0.134, 0.100) | 47 |
| Example 56 | 243 | 4.84 | 6.60 | (0.134, 0.100) | 48 |
| Example 57 | 331 | 4.80 | 6.70 | (0.134, 0.101) | 43 |
| Example 58 | 332 | 4.84 | 6.71 | (0.134, 0.100) | 46 |
| Example 59 | 493 | 4.87 | 6.72 | (0.134, 0.100) | 43 |
| Example 60 | 494 | 4.90 | 6.66 | (0.134, 0.100) | 45 |
| Comparative Example 6 | NPB | 5.51 | 6.06 | (0.134, 0.101) | 34 |
| Comparative Example 7 | HT1 | 5.36 | 6.15 | (0.134, 0.100) | 36 |
| Comparative Example 8 | HT2 | 5.38 | 6.17 | (0.134, 0.100) | 36 |
| Comparative Example 9 | HT3 | 5.44 | 6.11 | (0.134, 0.101) | 37 |
| Comparative Example 10 | HT4 | 5.38 | 6.08 | (0.134, 0.101) | 37 |

As can be seen from the results in Table 5, the organic light emitting device using an electron blocking layer material of the blue organic light emitting device of the present invention has a low driving voltage and remarkably improved light emitting efficiency and service life compared to the Comparative Examples. When the electrons are not combined in the light emitting layer and pass through the hole transport layer and go to the positive electrode, there occurs a phenomenon in which the efficiency and service life of the OLED device are reduced.

When a compound with a high LUMO level is used as an electron blocking layer to prevent such a phenomenon, electrons trying to pass through the light emitting layer to the positive electrode are blocked by the energy barrier of the electron blocking layer. Therefore, it could be seen that the probability of holes and electrons forming excitons is increased, and the excitons are highly likely to be emitted from the light-emitting layer as light, and thus the compounds of the present invention brought excellence in terms of all the aspects in driving, efficiency, and service life.

In particular, for the compound of Chemical Formula 1 of the present invention, it could be confirmed that when an amine derivative was used as a hole transport layer, the unshared electron pair of amine could enhance the flow of holes and improve the hole transfer capacity of the hole transport layer, and when the amine derivative was used as an electron blocking layer, the deterioration of the hole transport material caused by electrons penetrating into the hole transport layer could be suppressed, and the binding of the amine moiety to the substituent with enhanced hole characteristics also enhanced the planarity and glass transition temperature of the amine derivative to enhance the thermal stability of the compound.

Furthermore, since the bandgap and T1 values are adjusted to improve the hole transfer capacity and improve the stability of the molecule, it could be confirmed that the driving voltage of the device was lowered, the light efficiency of the device was improved, and service life characteristics of the device were improved by the thermal stability of the compound.

## Claims

1. A heterocyclic compound represented by the following Chemical Formula 1: 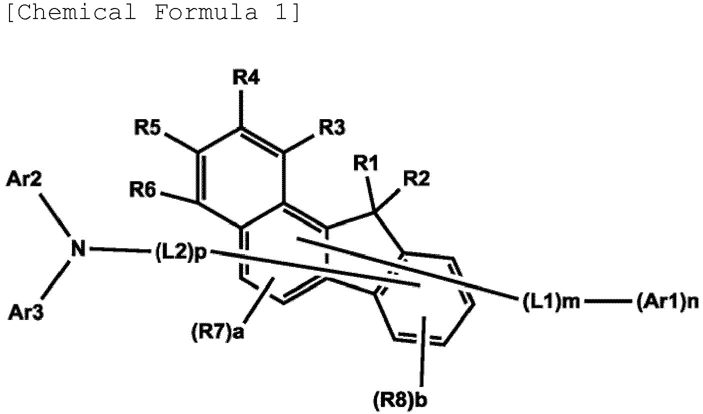 wherein, in Chemical Formula 1,
R1 and R2 are the same as or different from each other, and are each independently a substituted or unsubstituted C1 to C60 alkyl group; a substituted or unsubstituted C6 to C60 aryl group; or a substituted or unsubstituted C2 to C60 heteroaryl group,
R3 to R8 are the same as or different from each other, and are each independently selected from the group consisting of hydrogen; deuterium; a halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)RR'; -SiRR'R" and -NRR', or two or more adjacent groups are bonded to each other to form a substituted or unsubstituted C6 to C60 aliphatic or aromatic hydrocarbon ring, or a substituted or unsubstituted C2 to C60 aliphatic or aromatic hetero ring,
L1 and L2 are the same as or different from each other, and are each independently a direct bond; a substituted or unsubstituted C6 to C60 arylene group; or a substituted or unsubstituted C2 to C60 heteroarylene group,
Ar1 to Ar3 are the same as or different from each other, and are each independently a substituted or unsubstituted C1 to C60 alkyl group; a substituted or unsubstituted C6 to C60 aryl group; or a substituted or unsubstituted C2 to C60 heteroaryl group,
p and m are an integer from 0 to 4, n is an integer from 1 to 6, a is an integer of 0 or 1, and b is an integer from 0 to 2, and
R, R' and R" are the same as or different from each other, and are each independently a substituted or unsubstituted C1 to C60 alkyl group; a substituted or unsubstituted C6 to C60 aryl group; or a substituted or unsubstituted C2 to C60 heteroaryl group.

2. The heterocyclic compound of claim 1, wherein Chemical Formula 1 is represented by the following Chemical Formula 2 or 3: 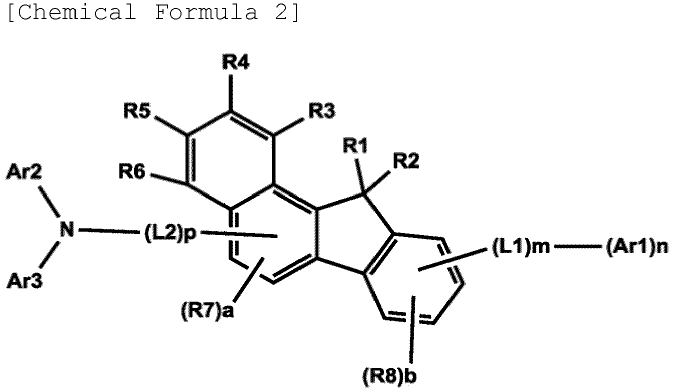 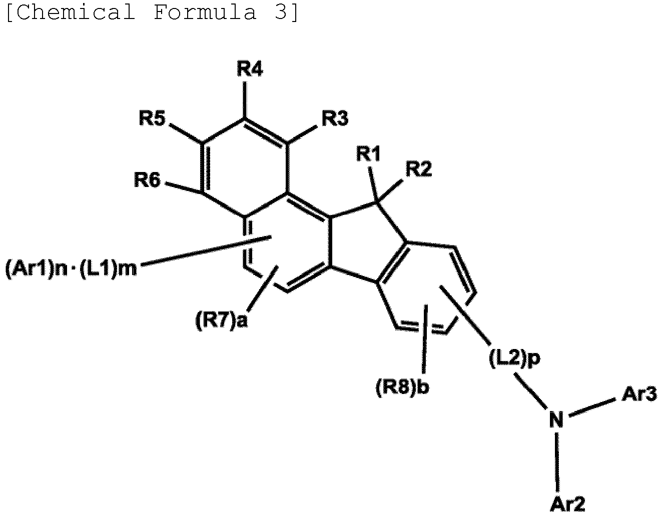 in Chemical Formulae 2 and 3, the definitions of R1 to R8, L1, L2, Ar1 to Ar3, p, m, n, a and b are the same as the definitions in Chemical Formula 1.

3. The heterocyclic compound of claim 1, wherein 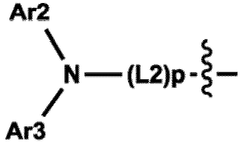 of Chemical Formula 1 is represented by the following Chemical Formula A-1 or A-2: 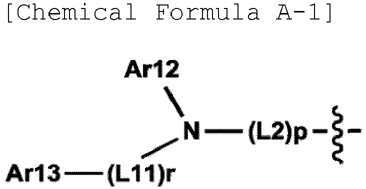 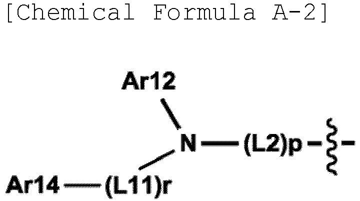 in Chemical Formulae A-1 and A-2,
the definitions of L2 and p are the same as the definitions in Chemical Formula 1, 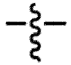 means a position linked to Chemical Formula 1, L11 is a direct bond; or a substituted or unsubstituted C6 to C60 arylene group,
Ar12 and Ar13 are the same as or different from each other, and are each independently a substituted or unsubstituted C6 to C60 aryl group,
Ar14 is a substituted or unsubstituted C2 to C60 heteroaryl group, and
r is an integer from 0 to 3.

4. The heterocyclic compound of claim 1, wherein R1 and R2 are the same as or different from each other, and are each independently a C1 to C20 alkyl group; or a C6 to C20 aryl group.

5. The heterocyclic compound of claim 1, wherein R3 to R8 are hydrogen.

6. The heterocyclic compound of claim 1, wherein Chemical Formula 1 is represented by any one of the following compounds:

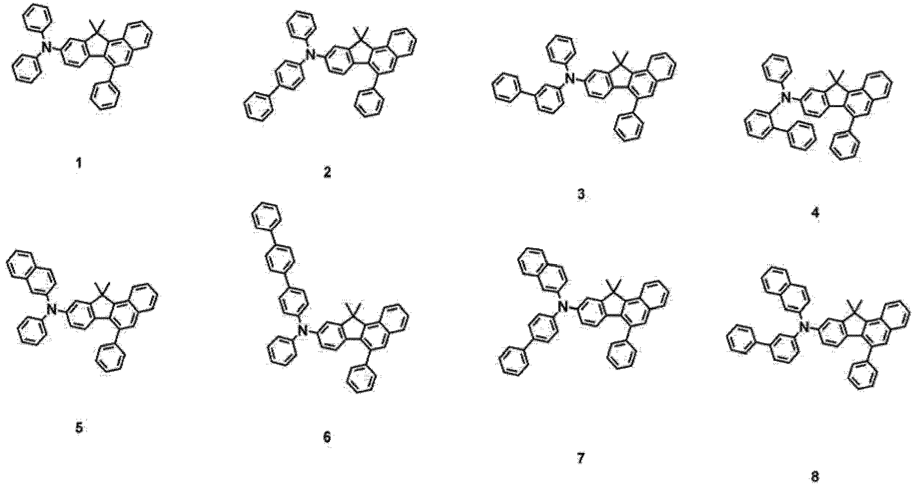

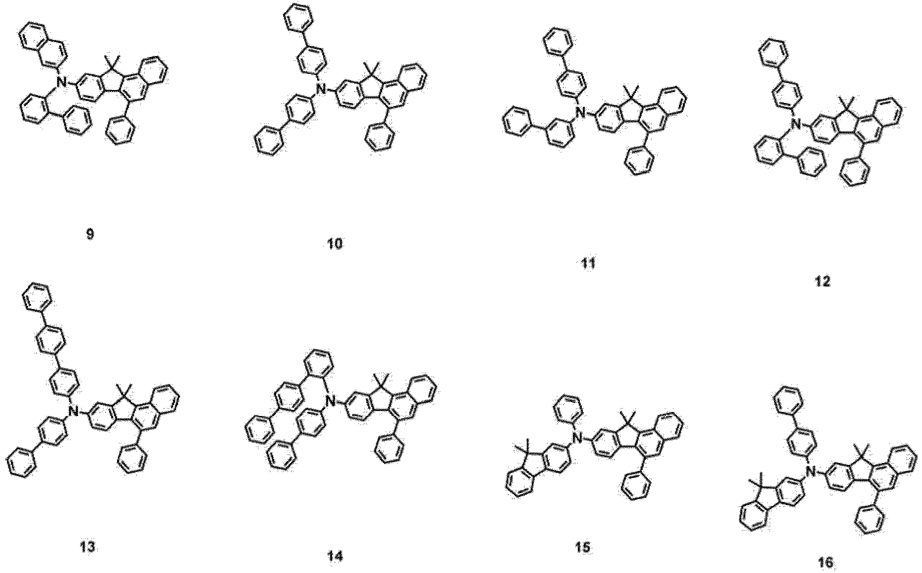

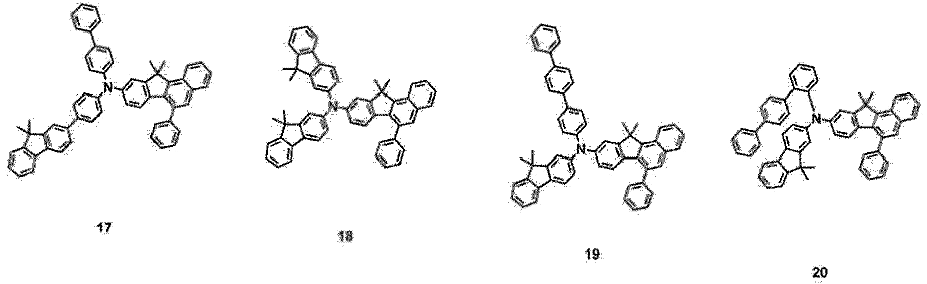

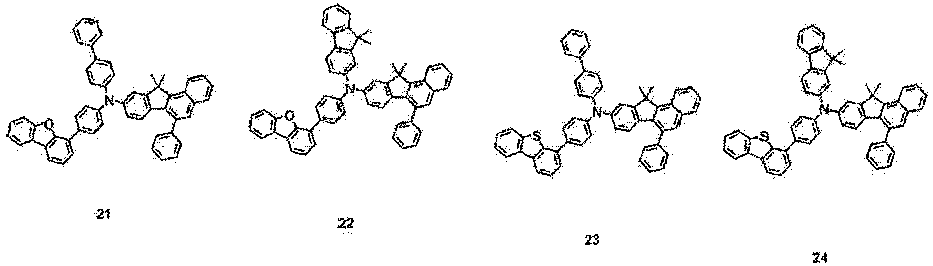

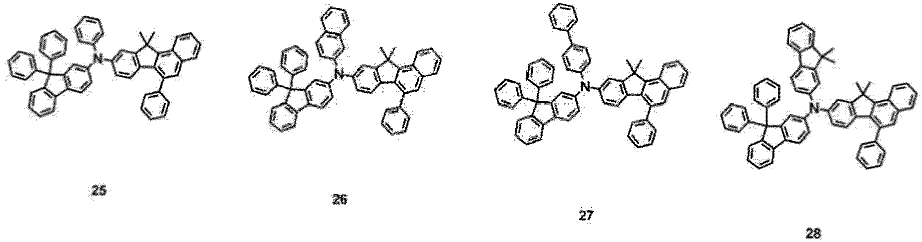

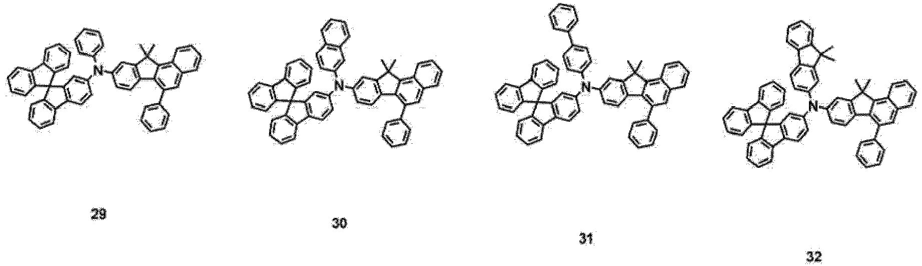

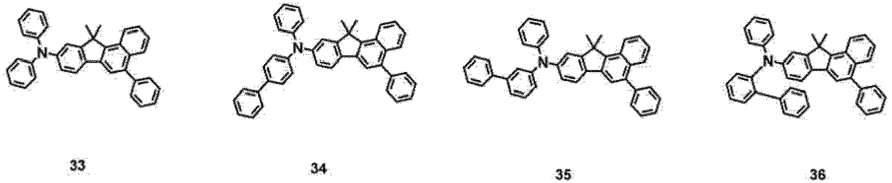

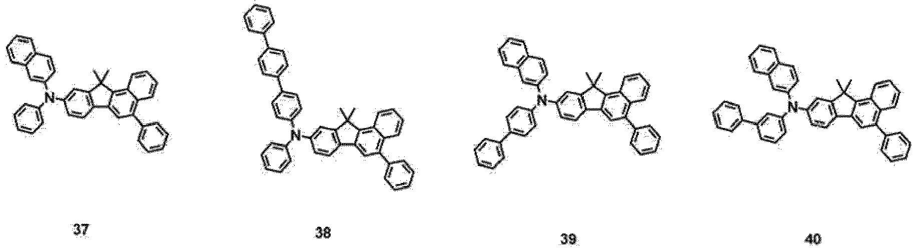

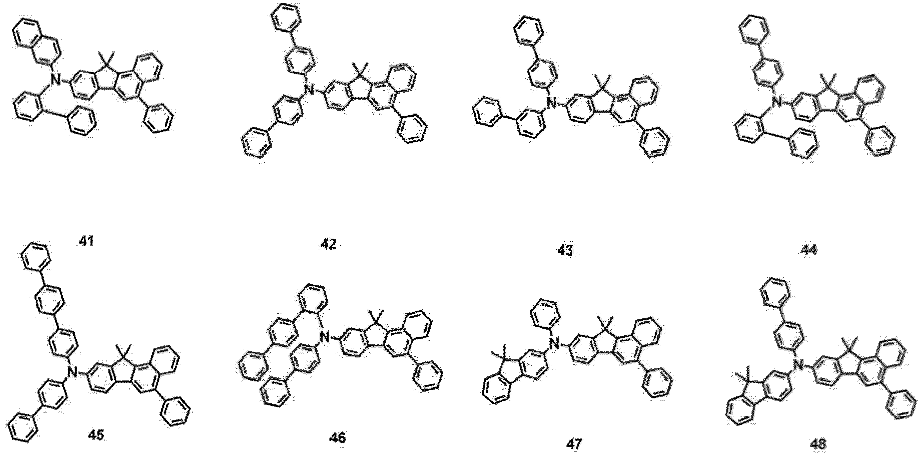

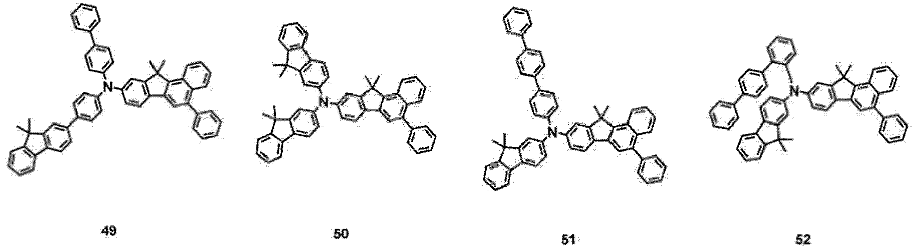

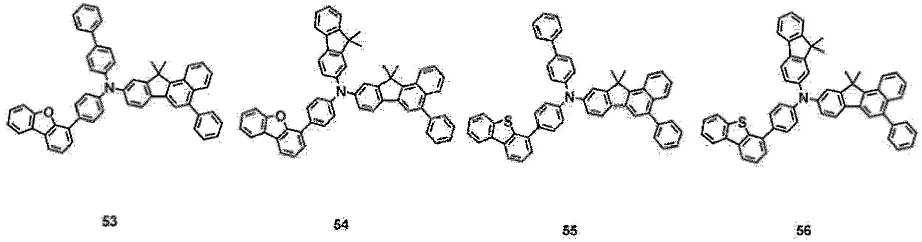

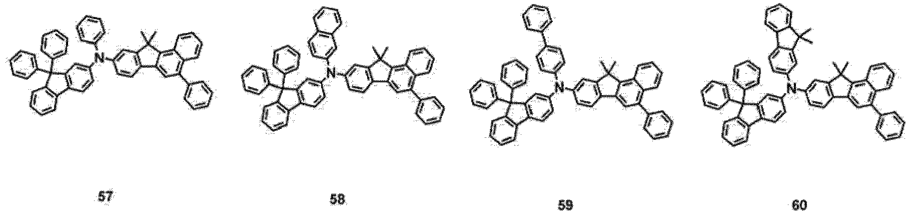

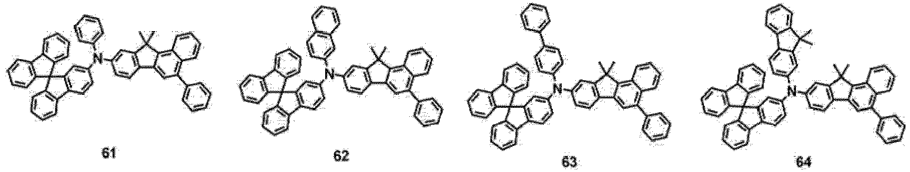

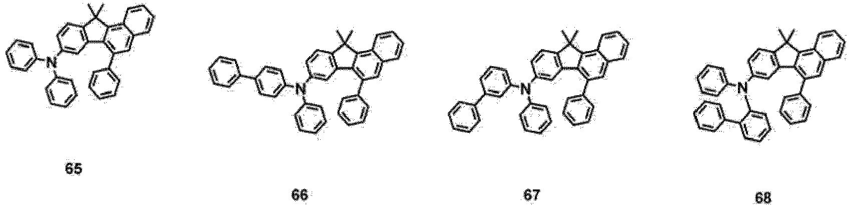

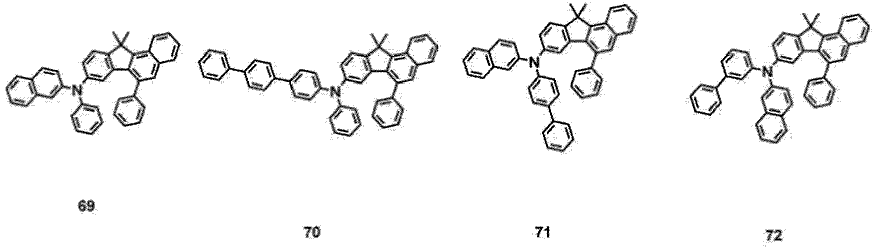

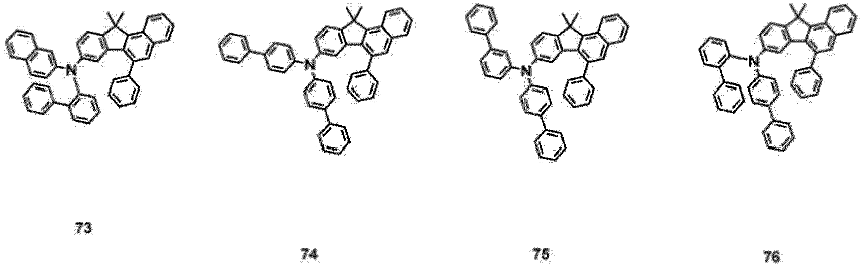

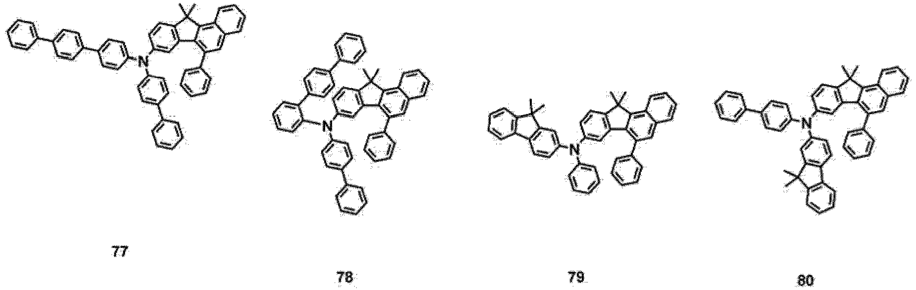

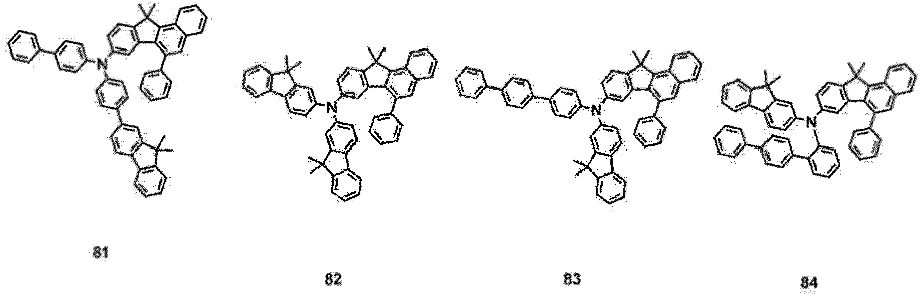

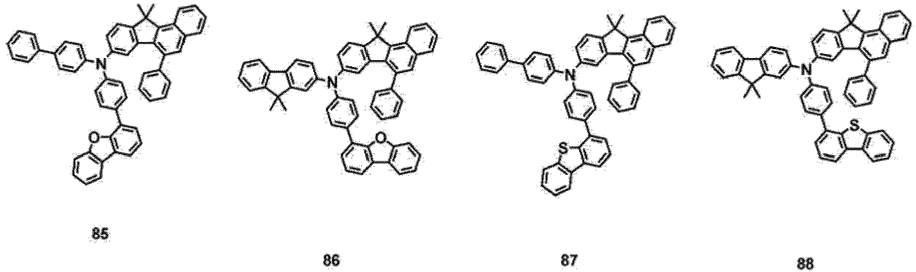

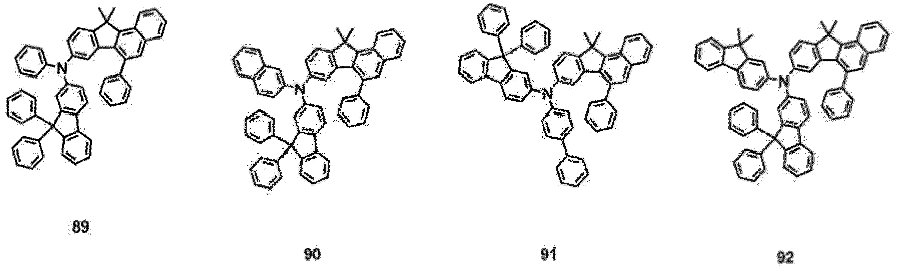

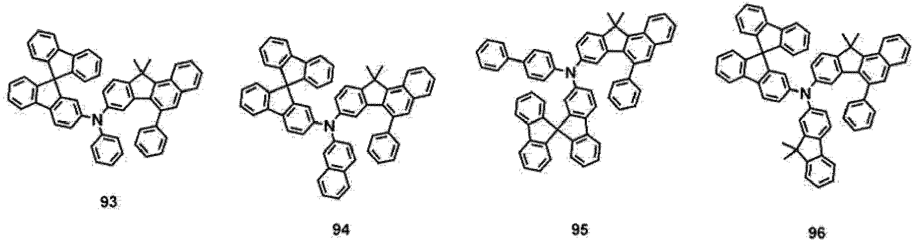

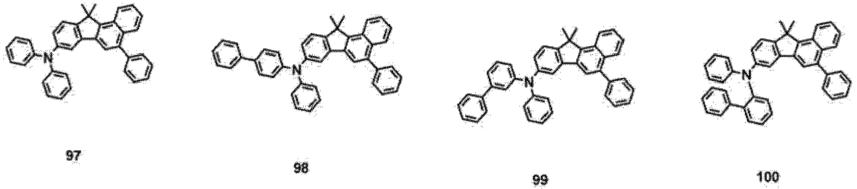

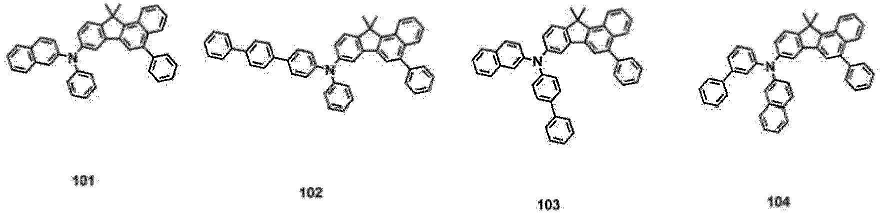

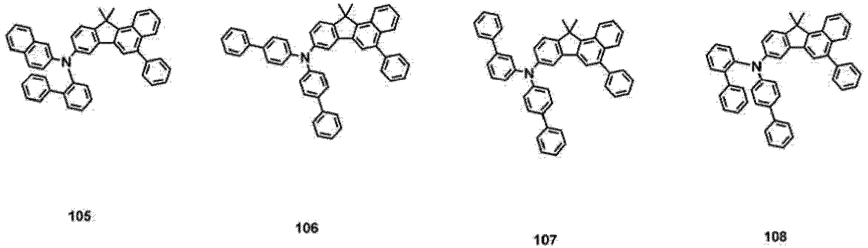

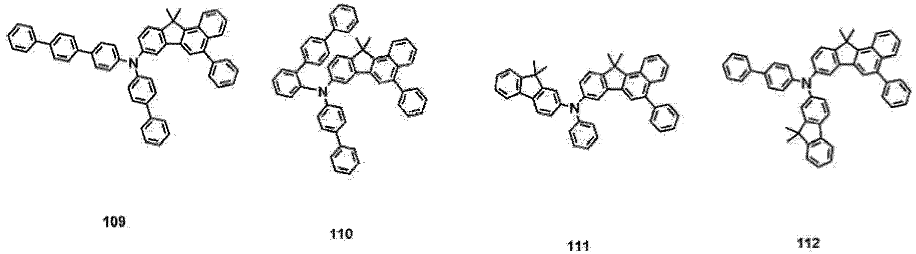

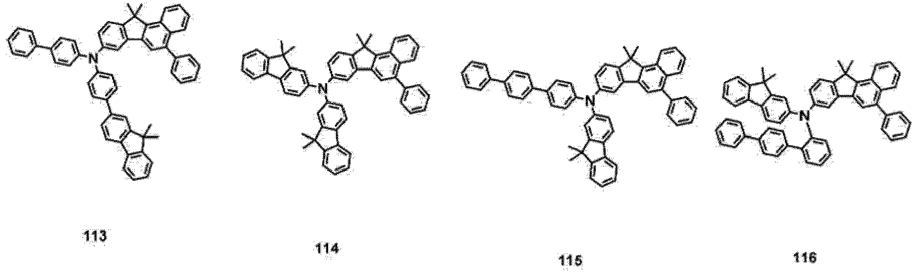

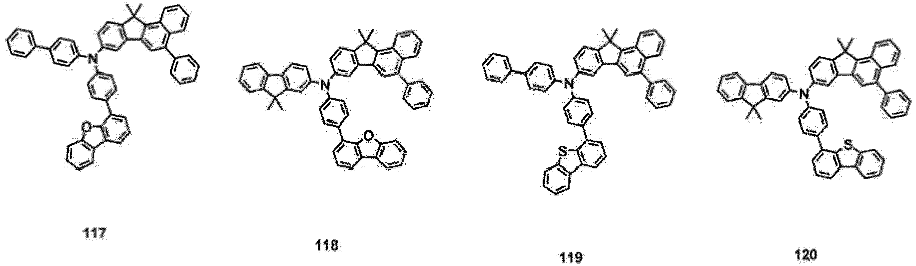

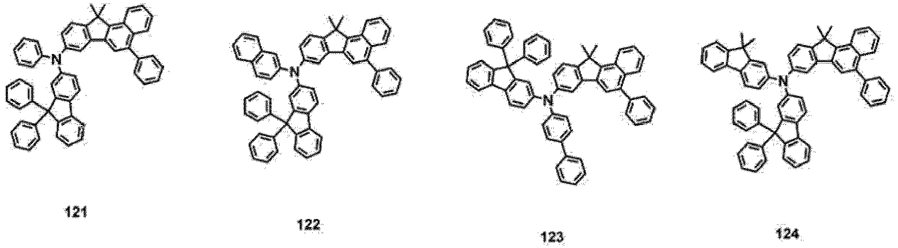

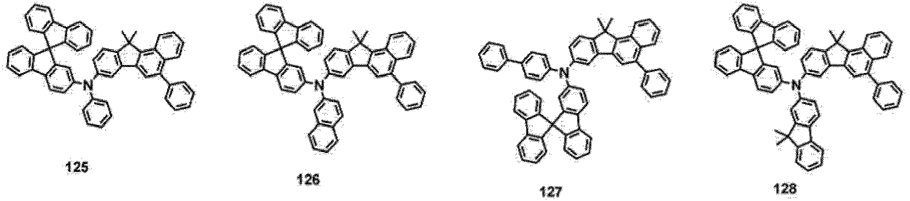

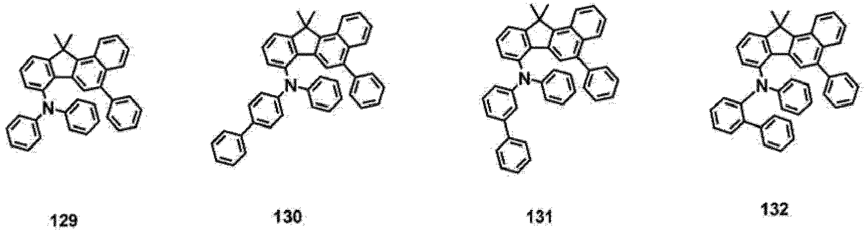

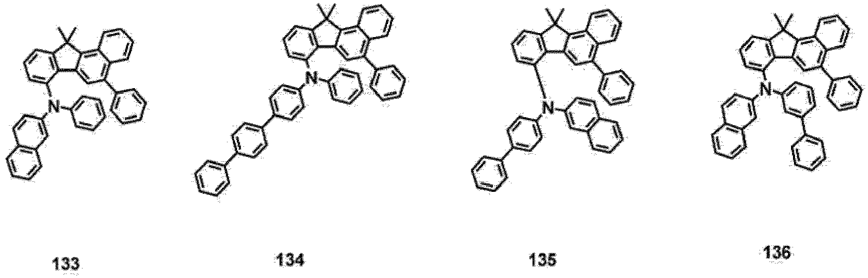

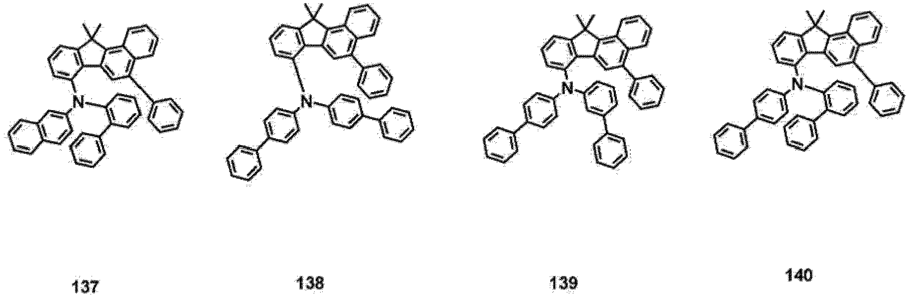

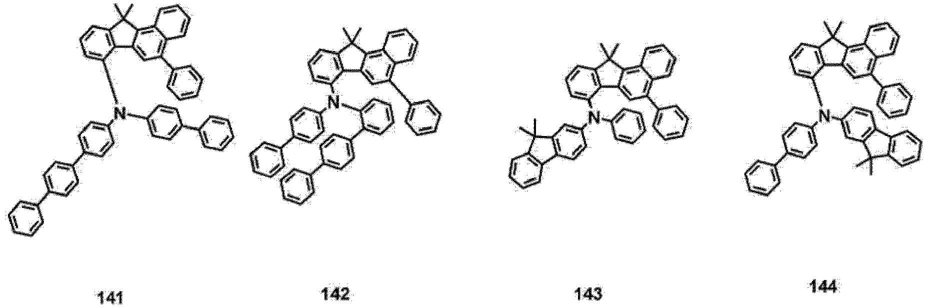

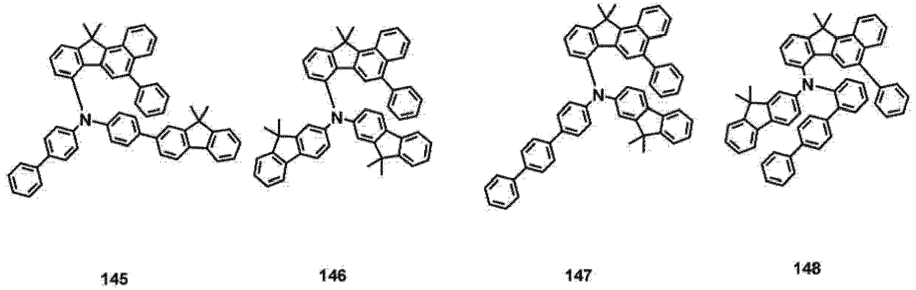

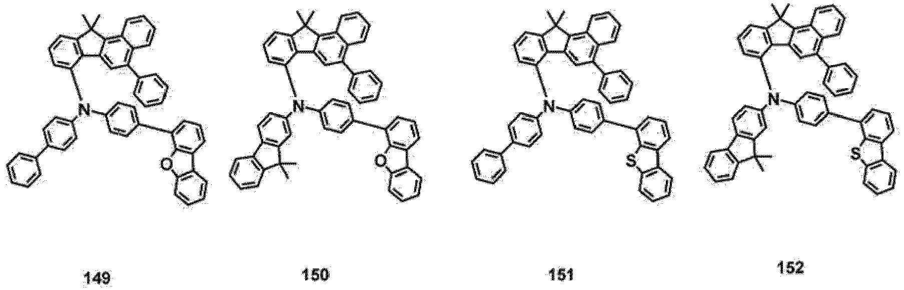

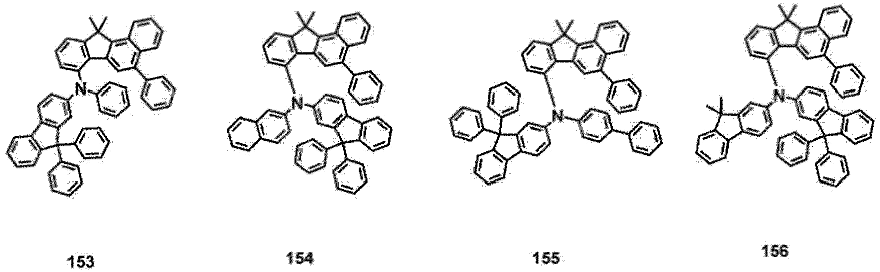

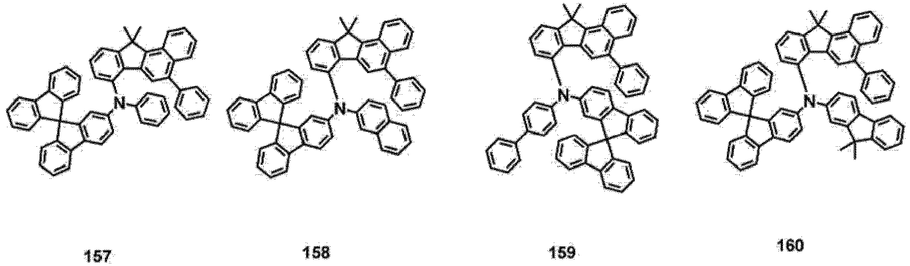

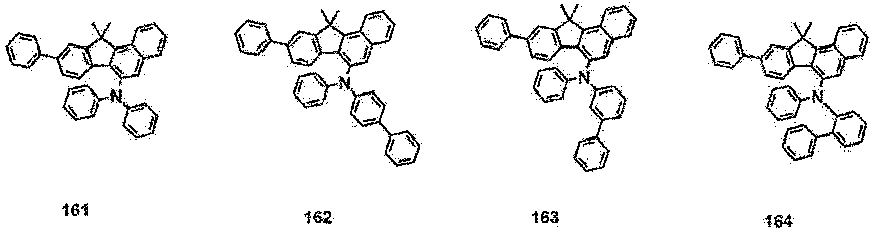

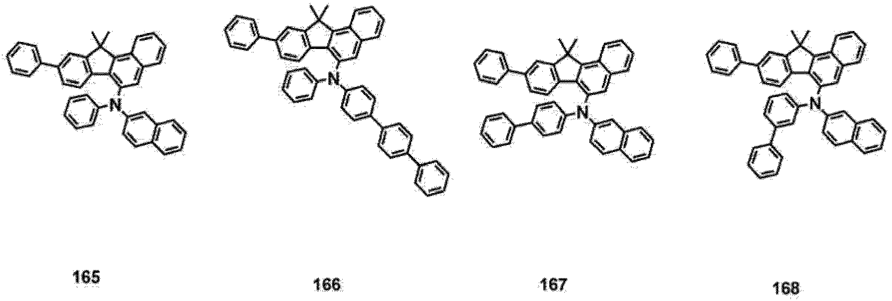

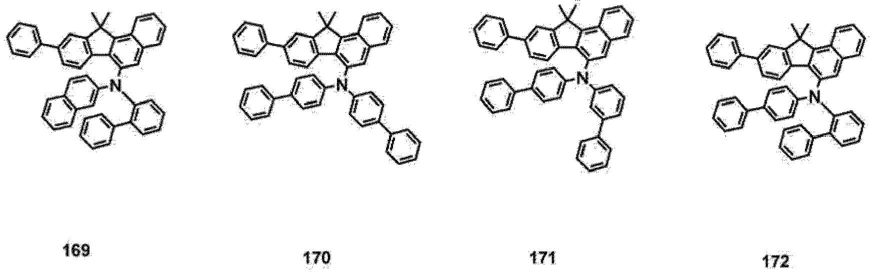

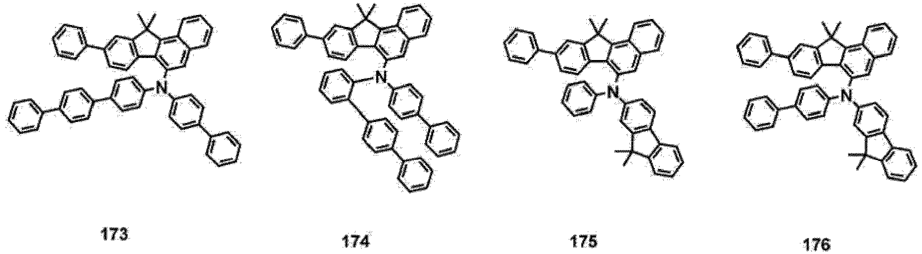

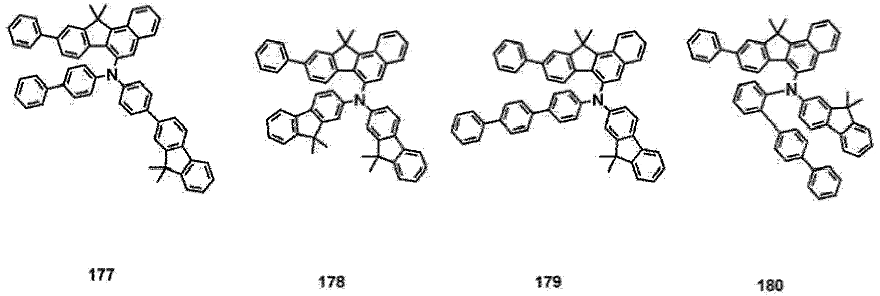

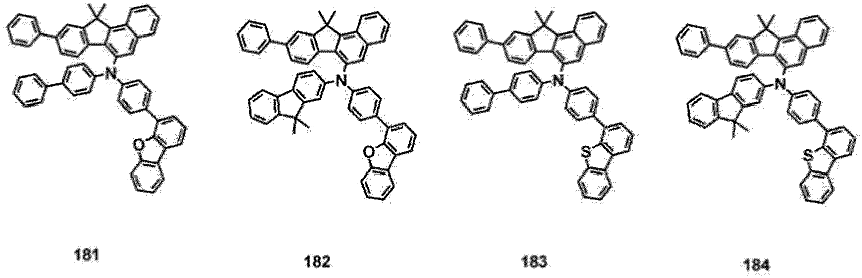

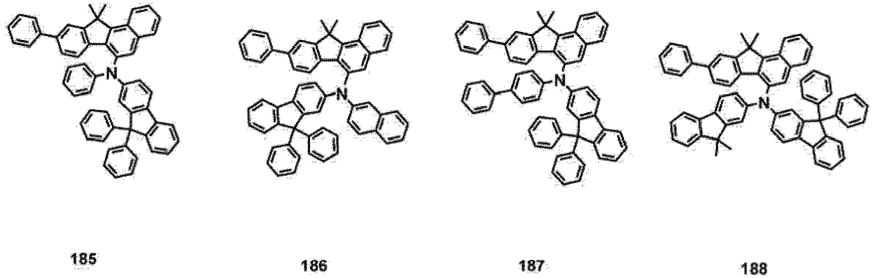

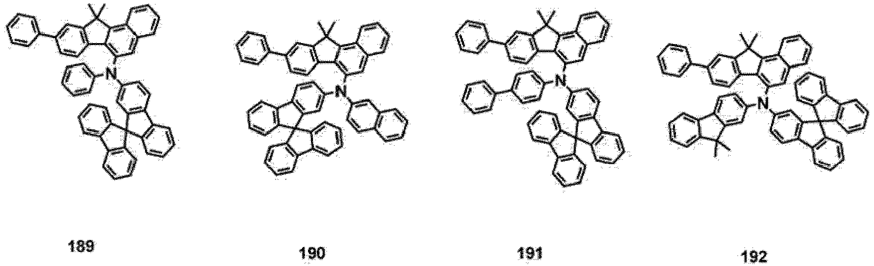

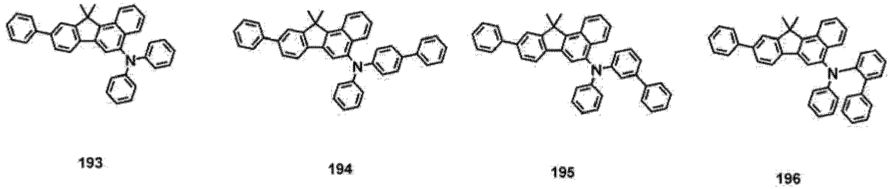

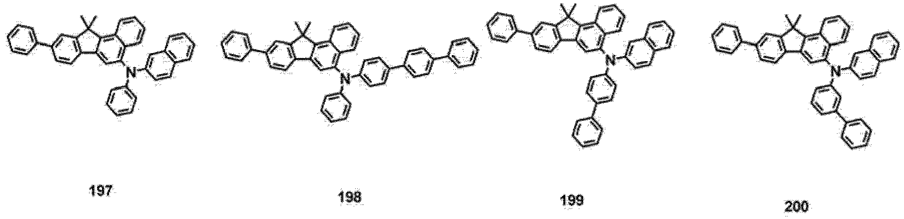

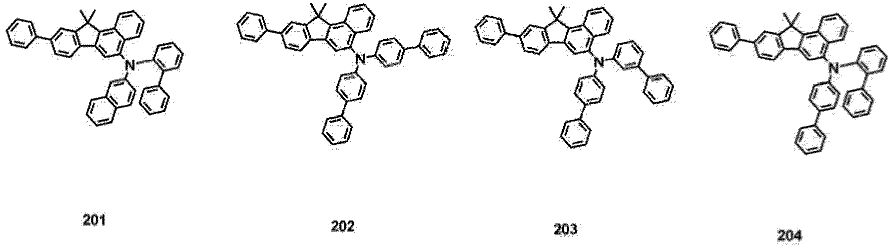

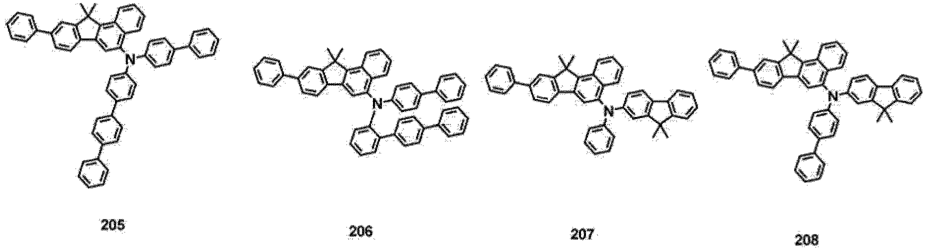

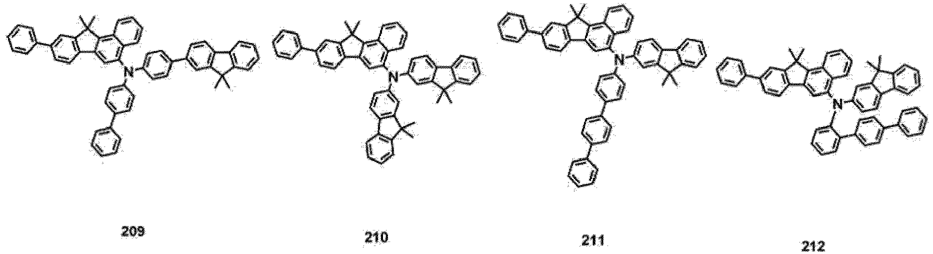

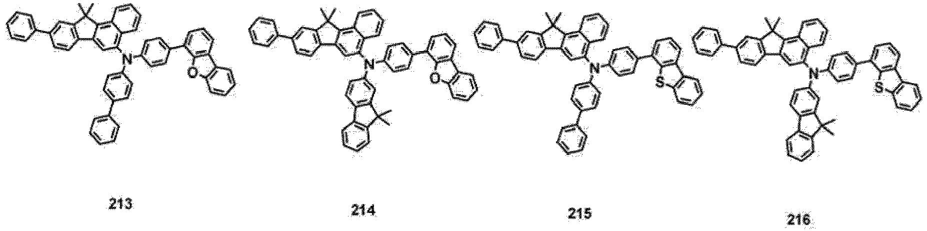

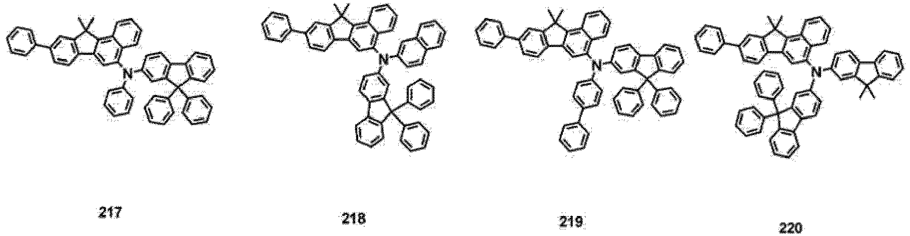

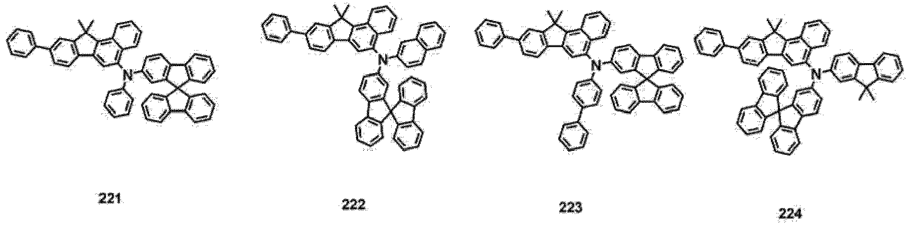

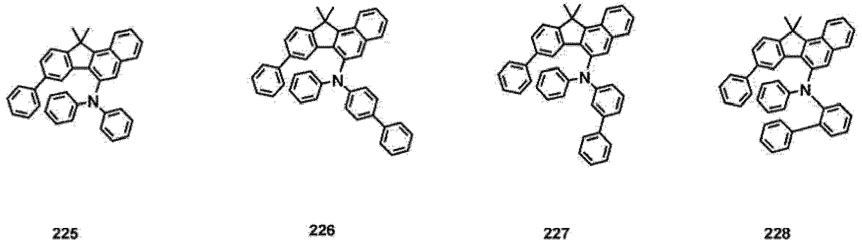

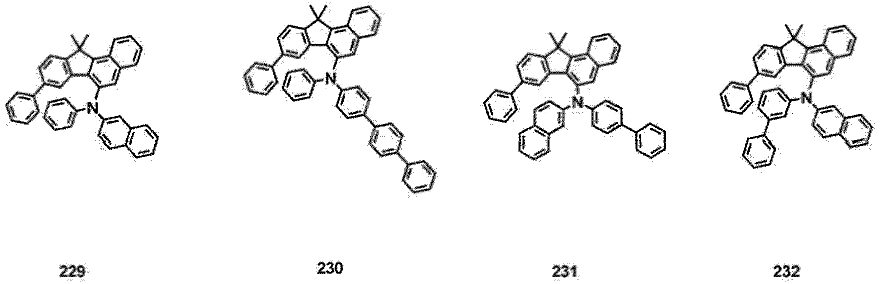

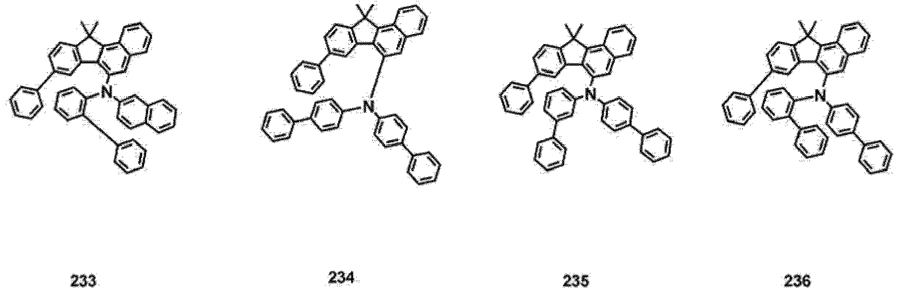

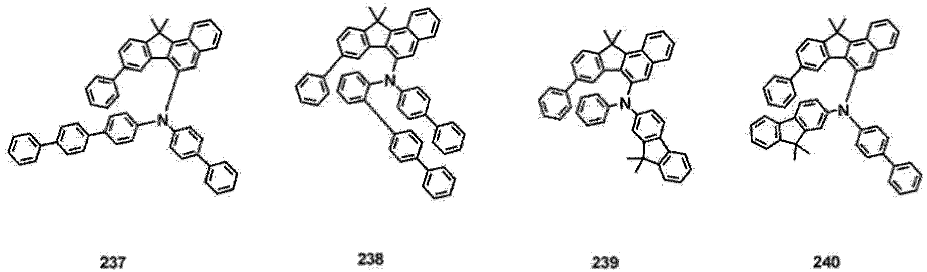

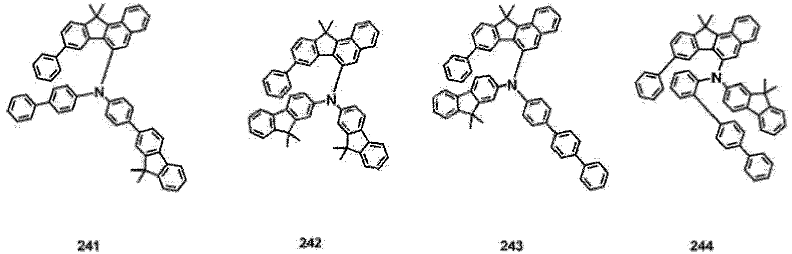

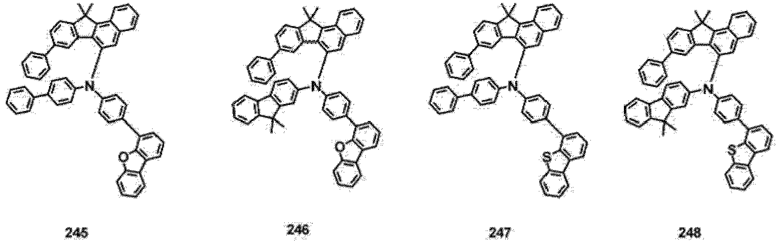

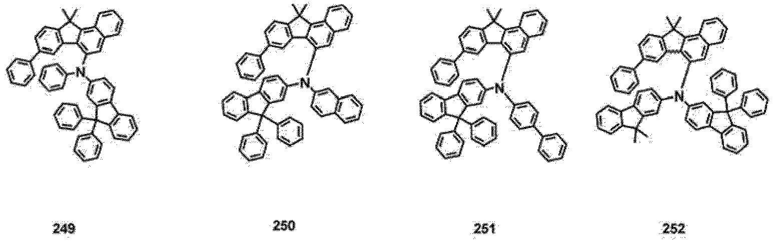

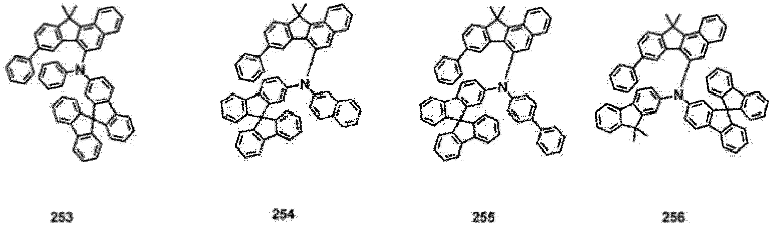

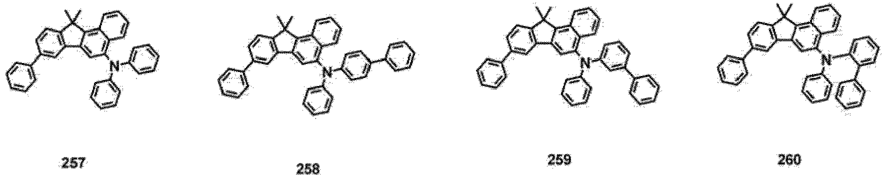

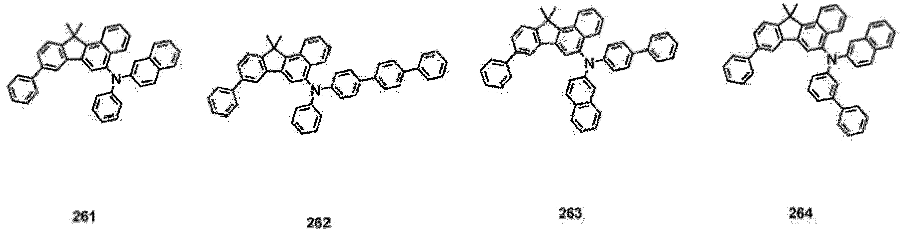

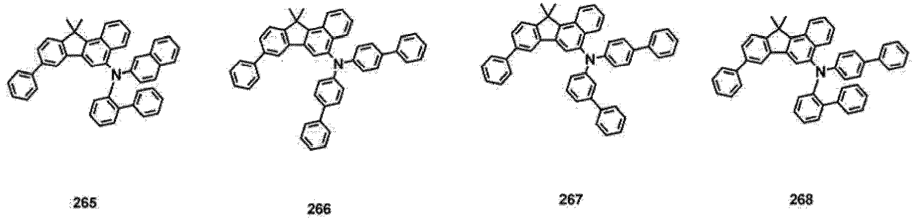

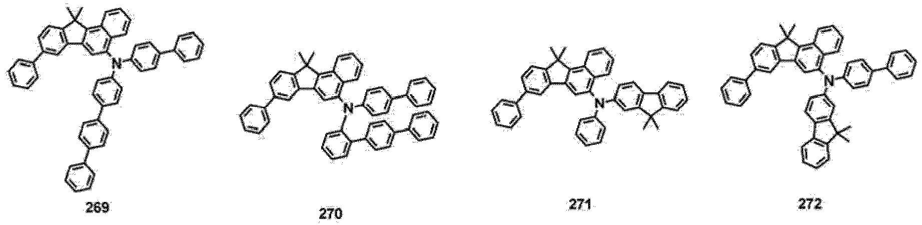

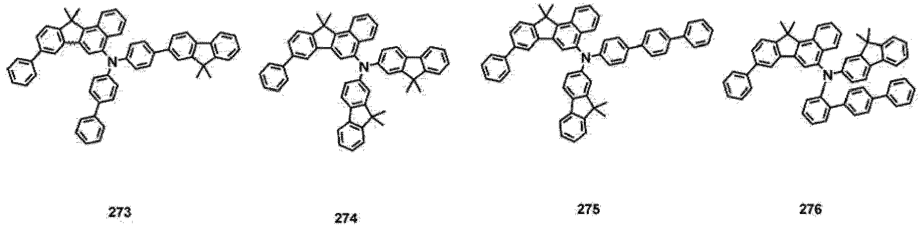

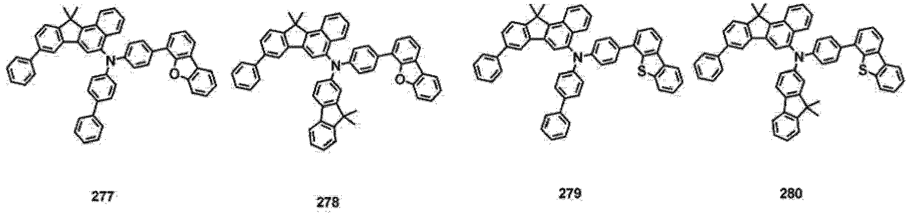

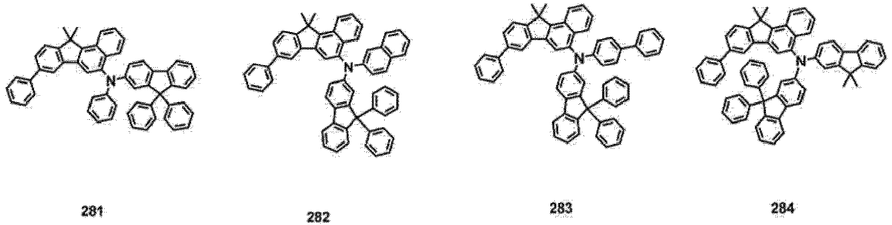

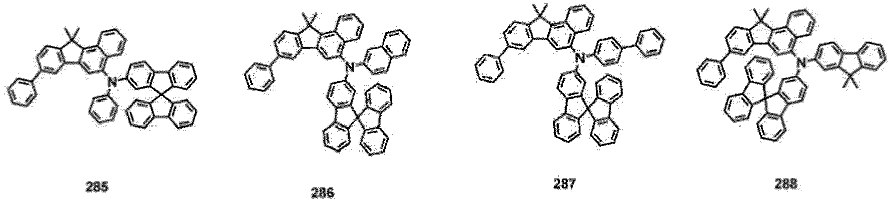

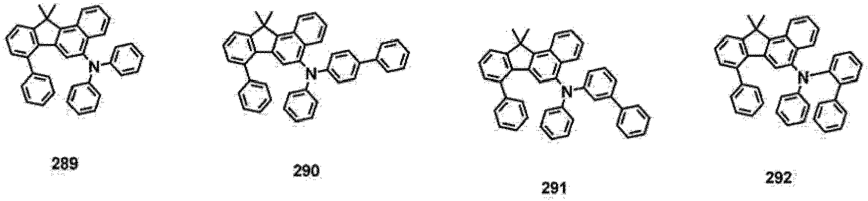

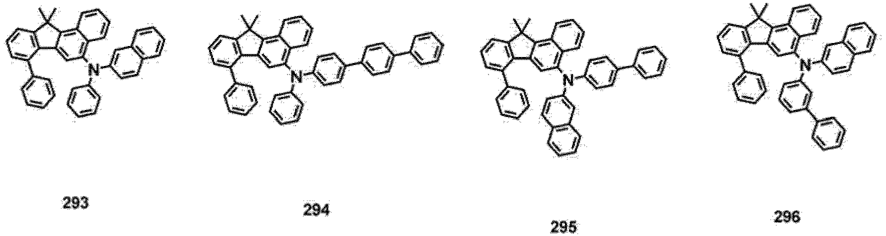

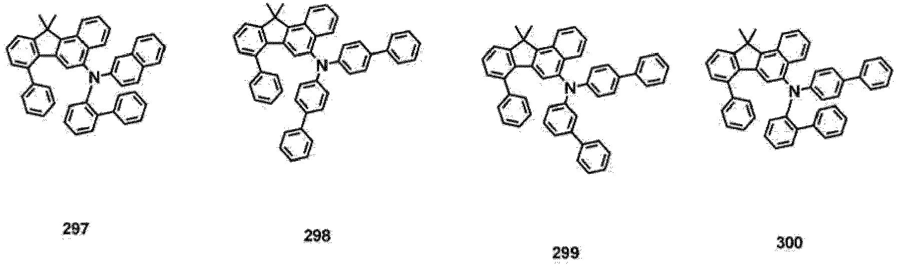

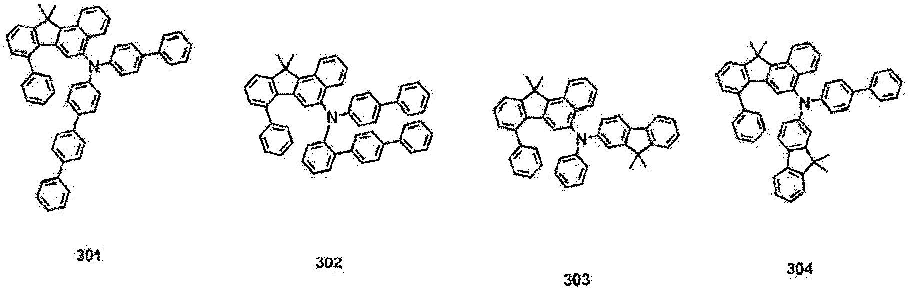

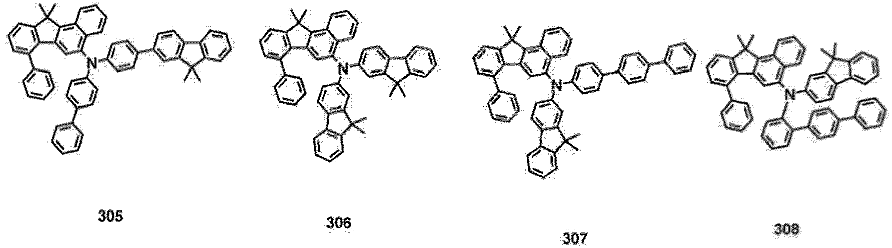

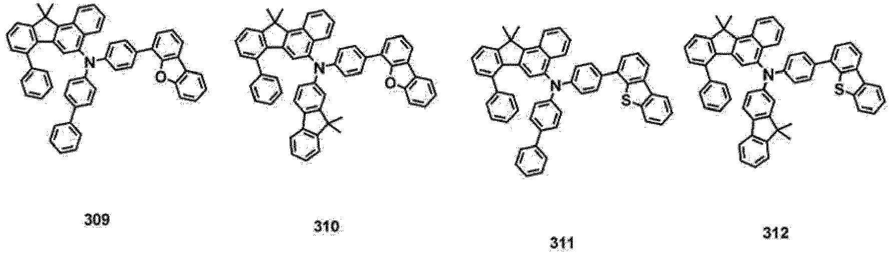

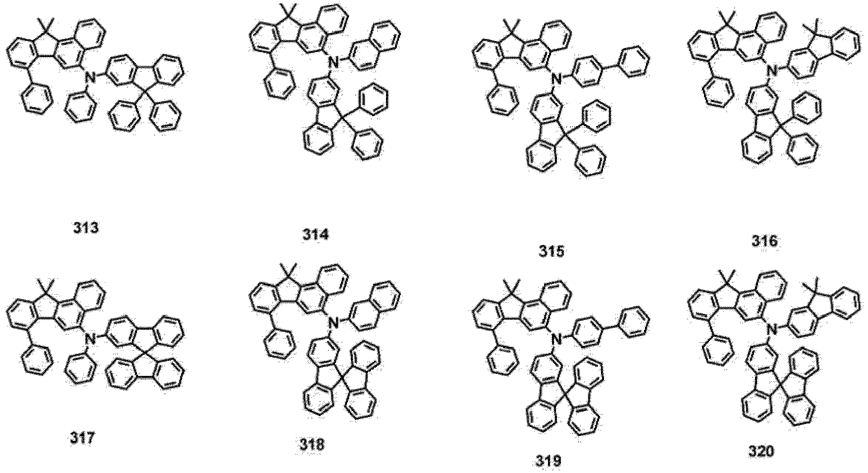

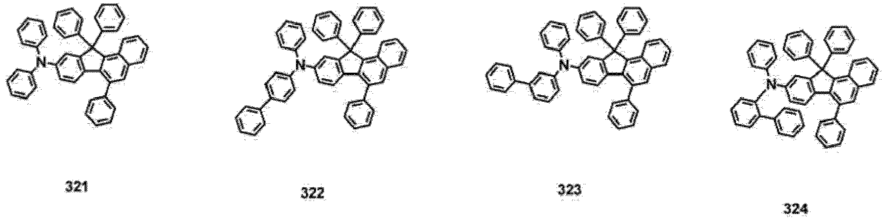

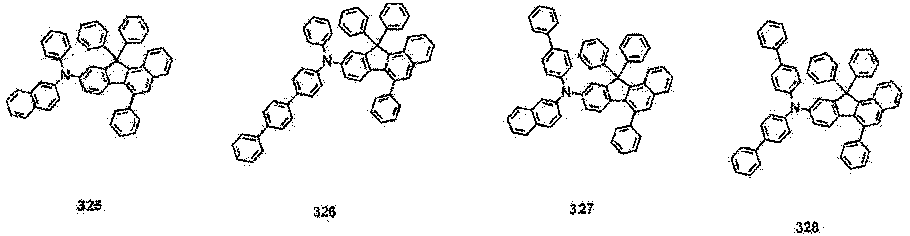

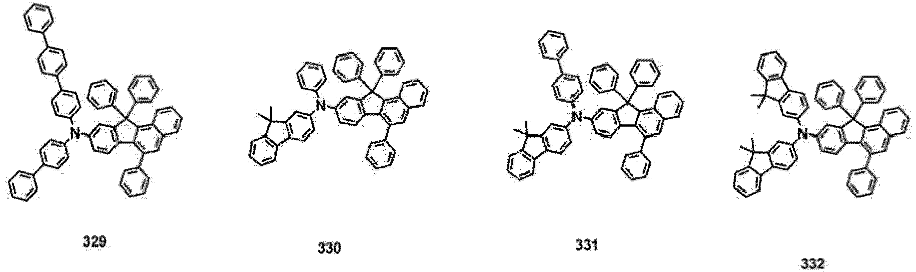

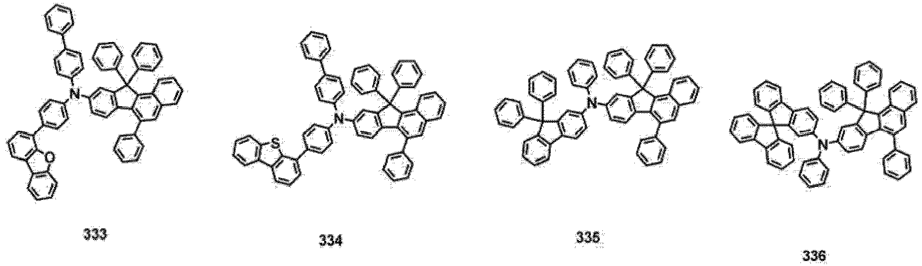

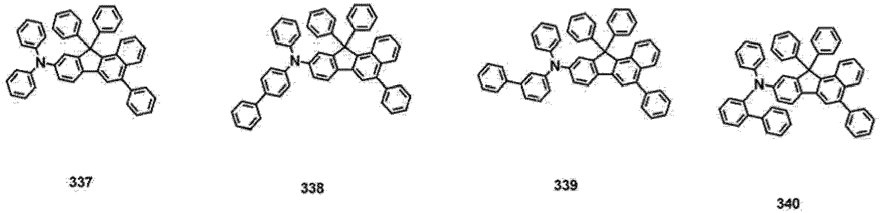

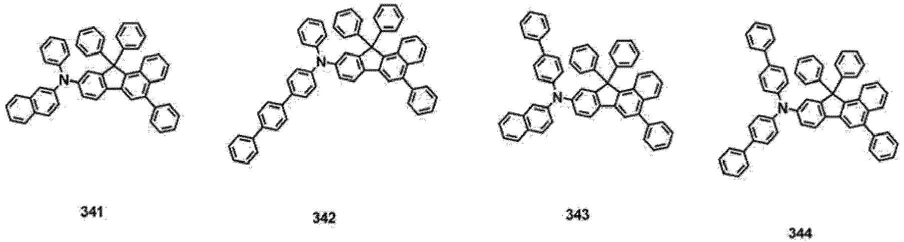

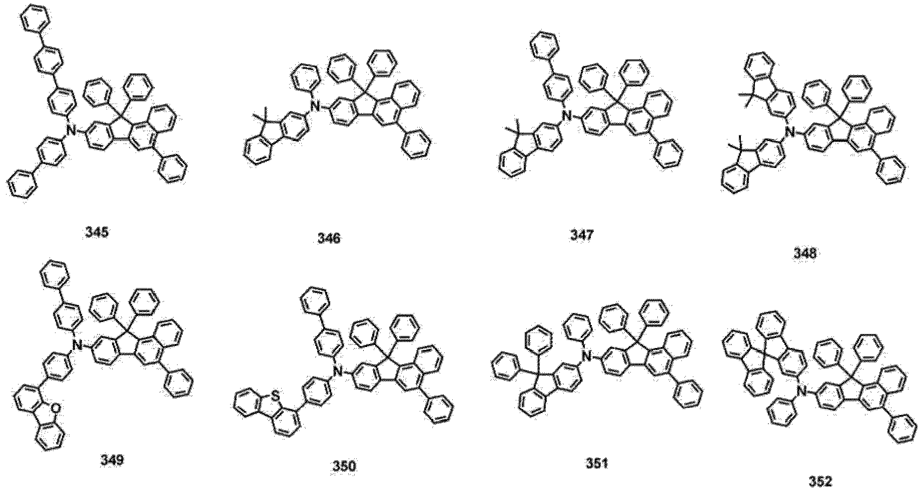

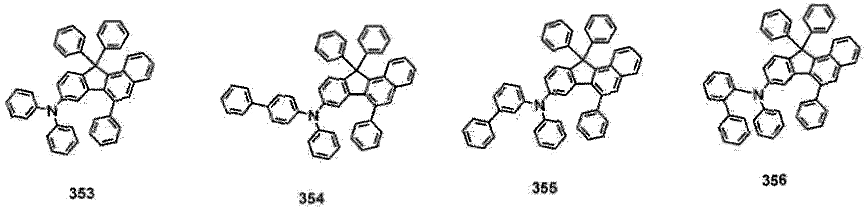

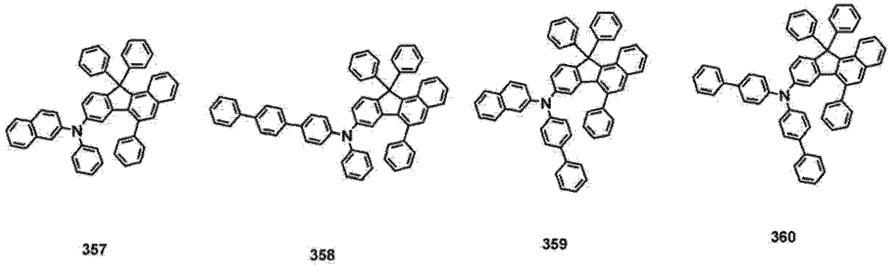

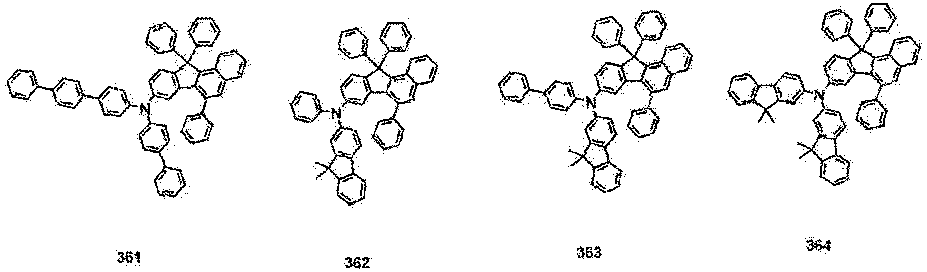

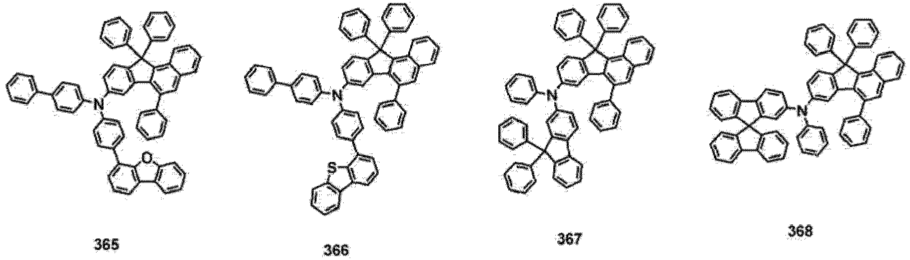

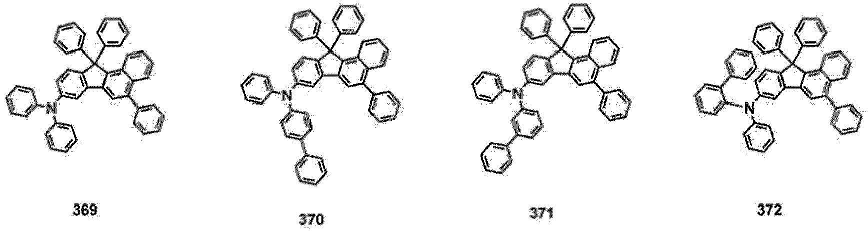

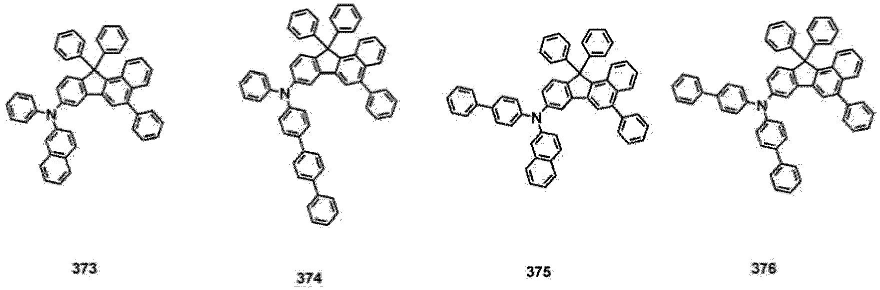

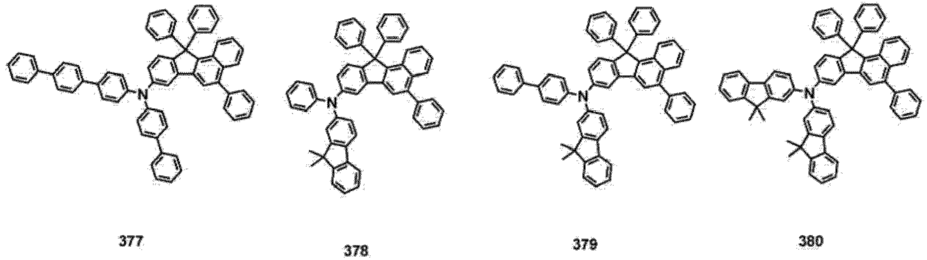

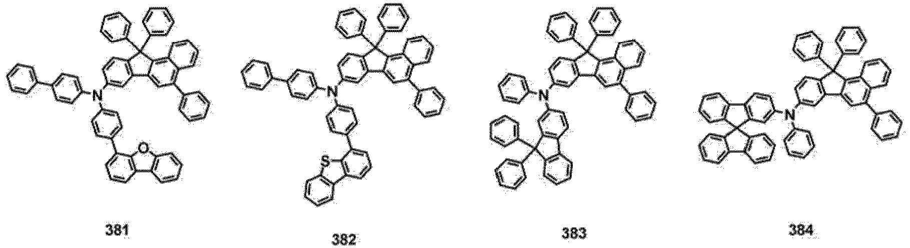

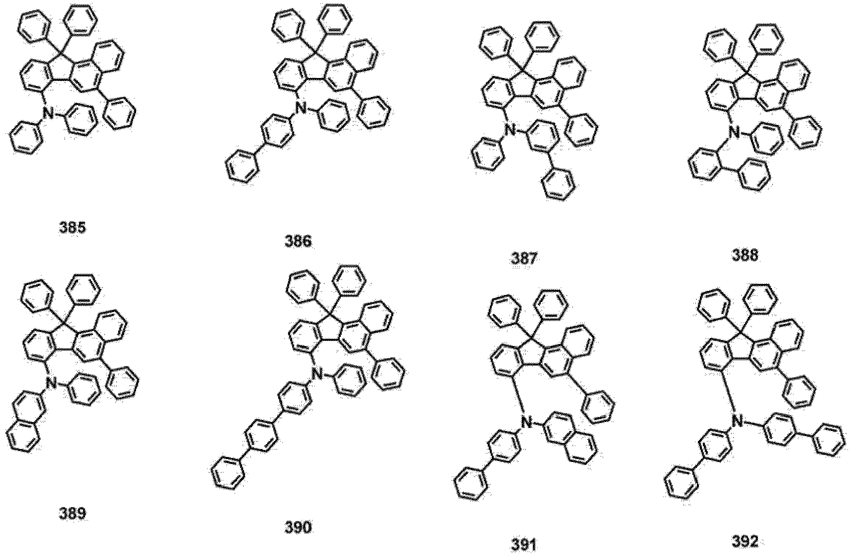

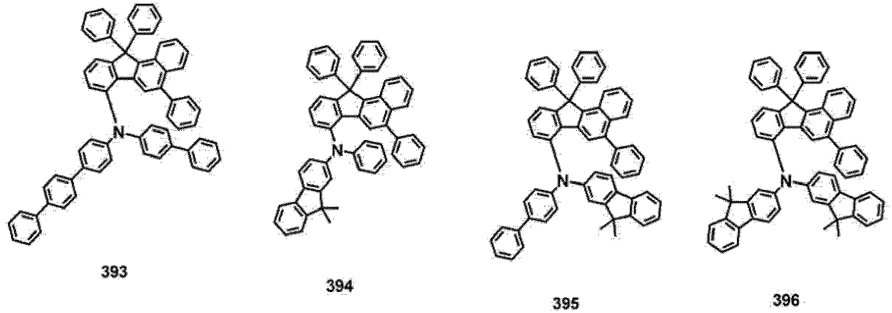

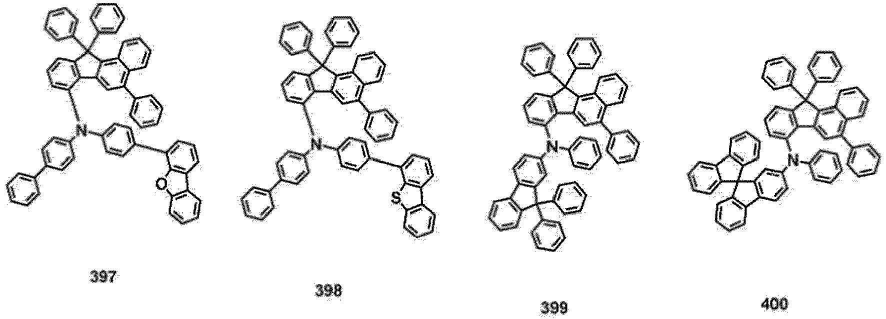

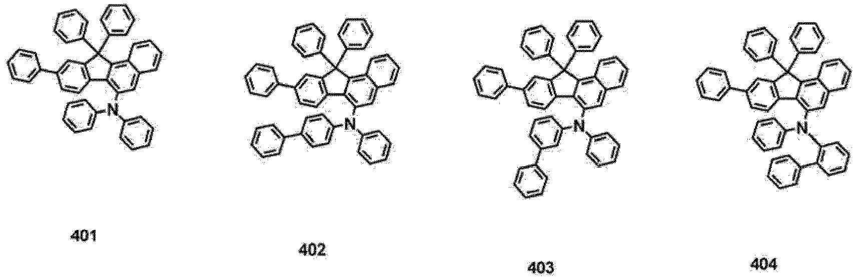

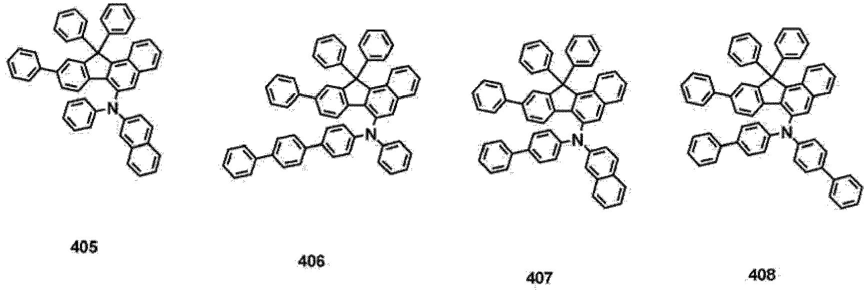

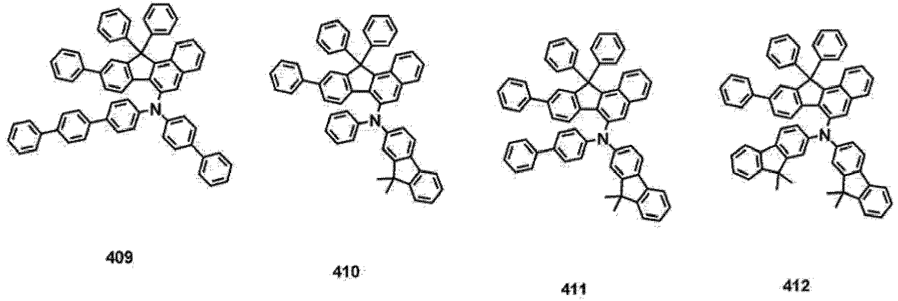

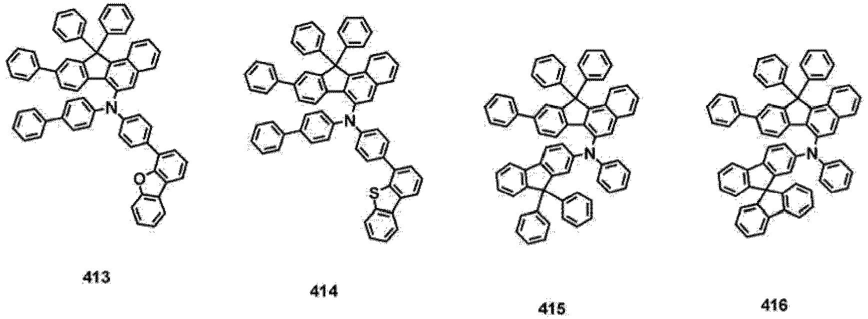

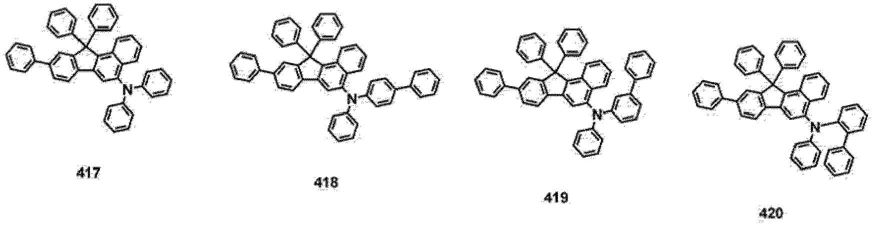

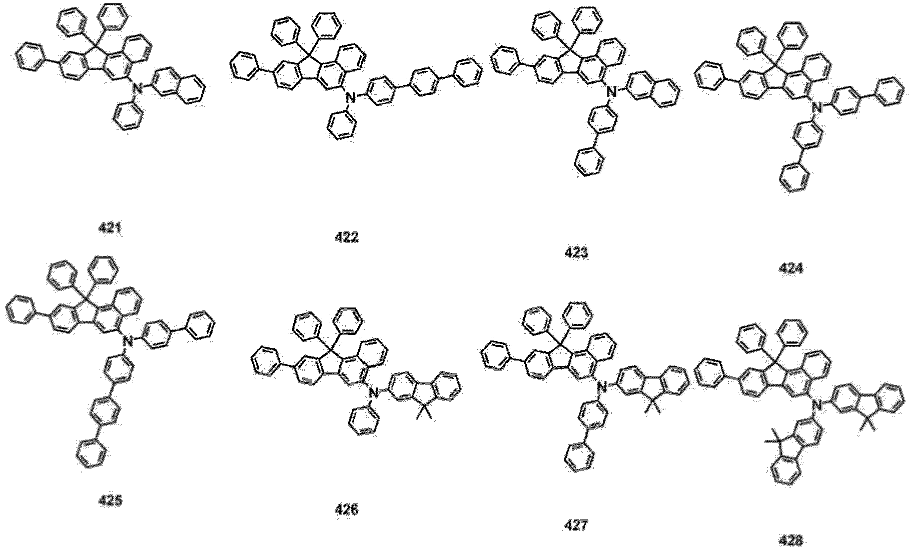

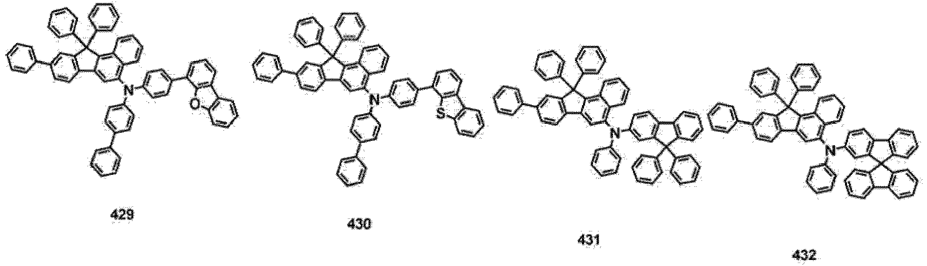

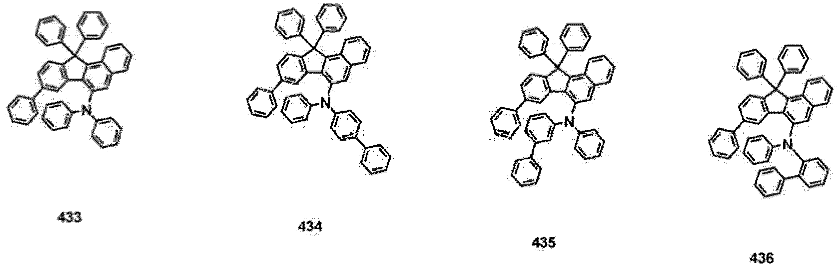

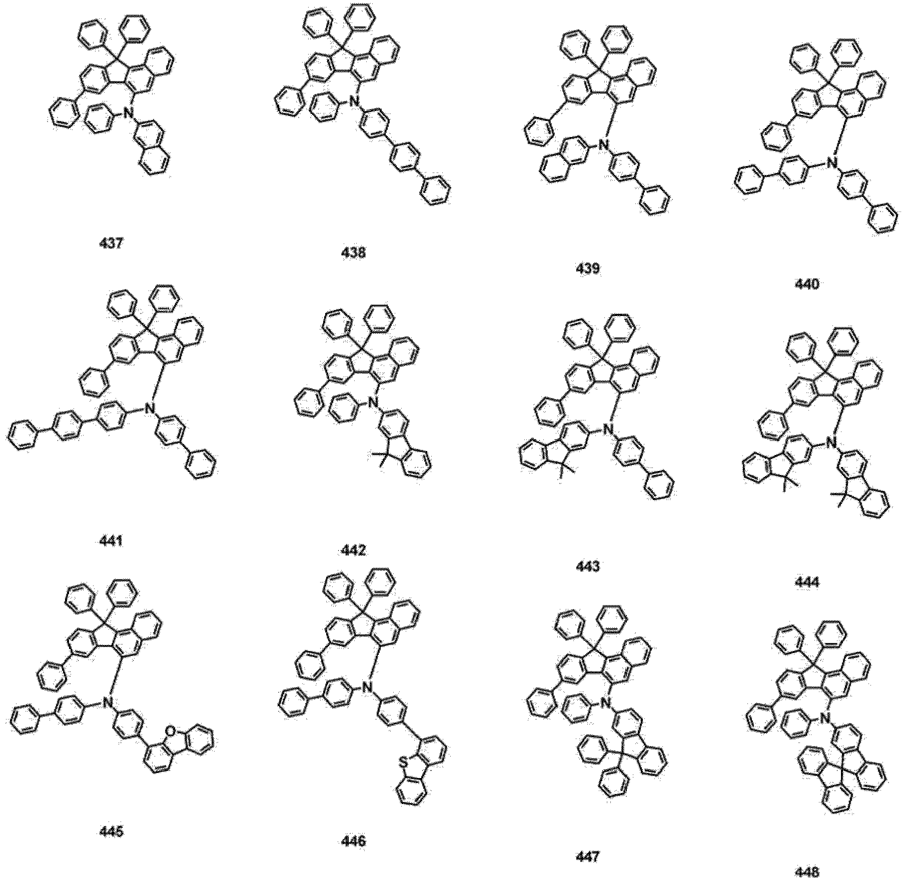

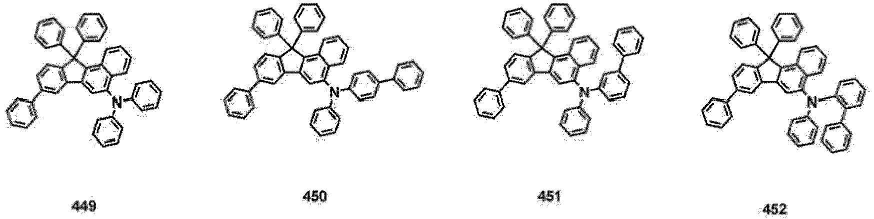

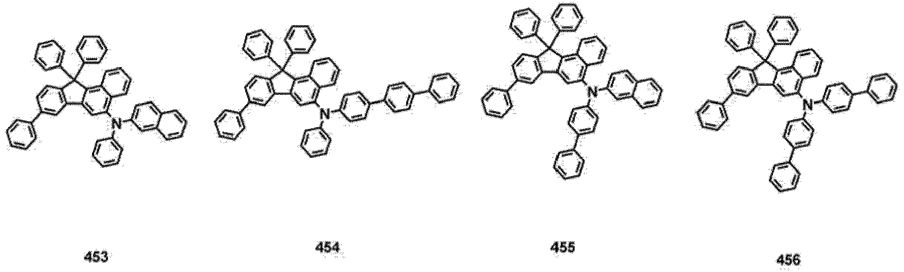

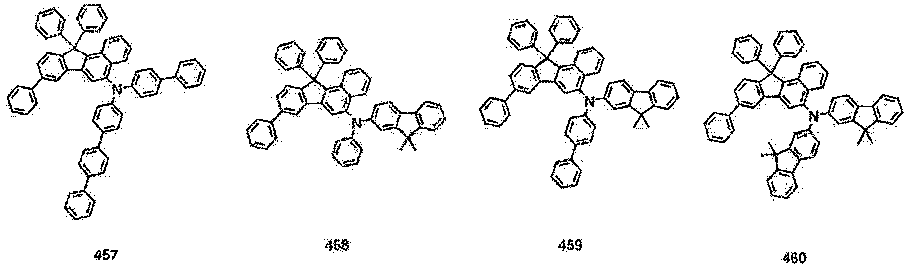

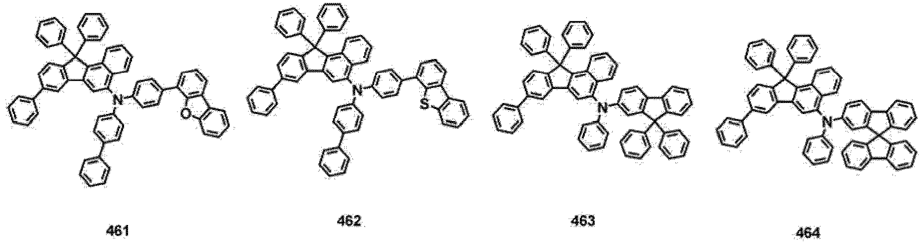

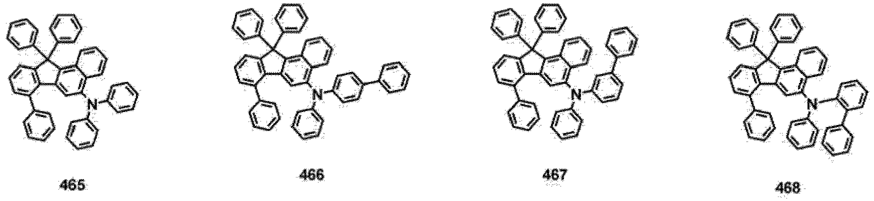

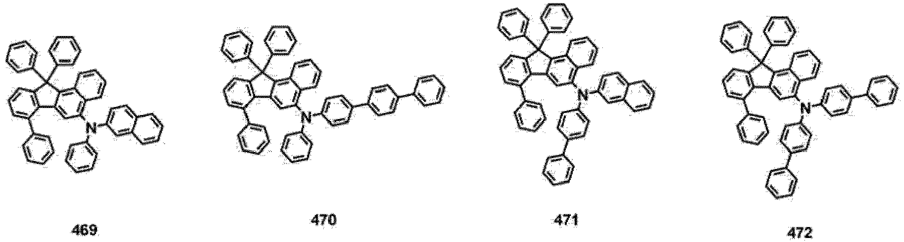

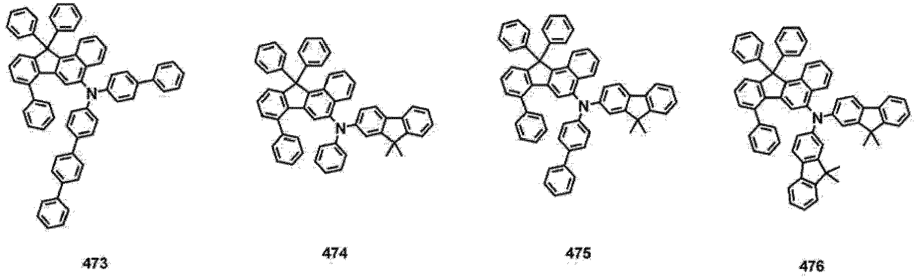

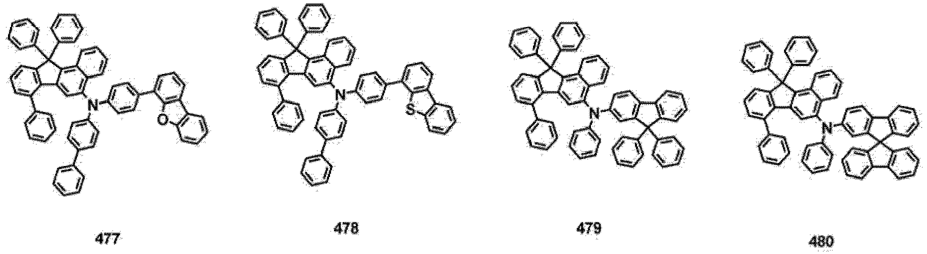

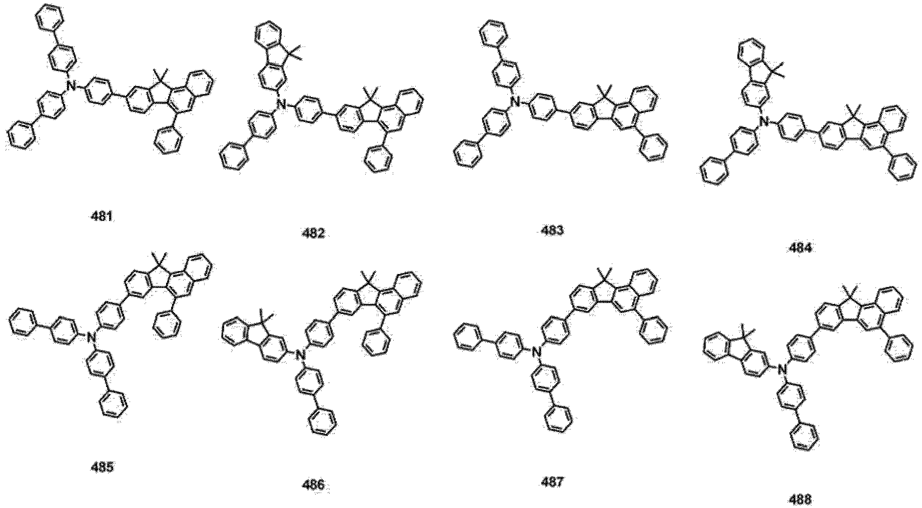

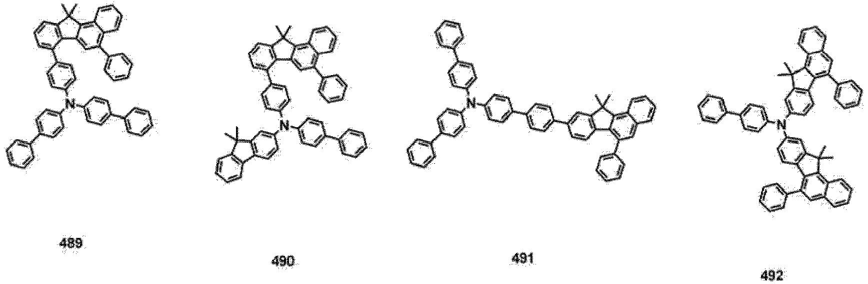

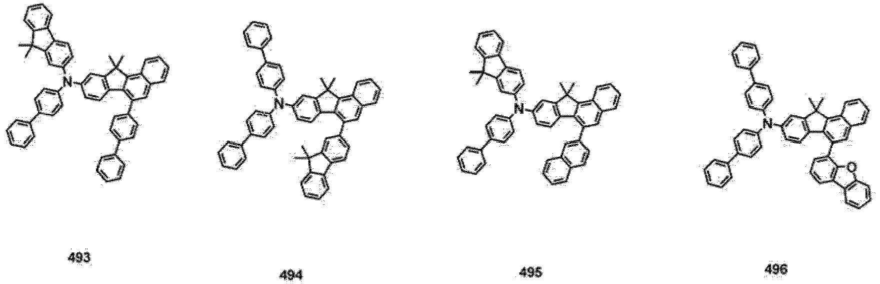

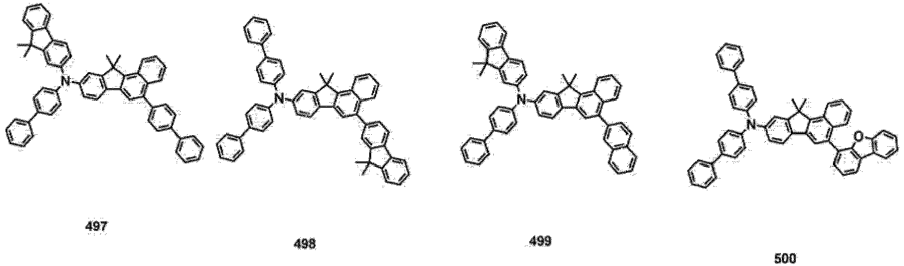

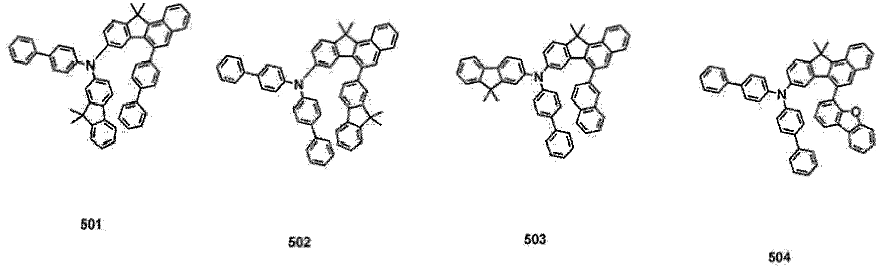

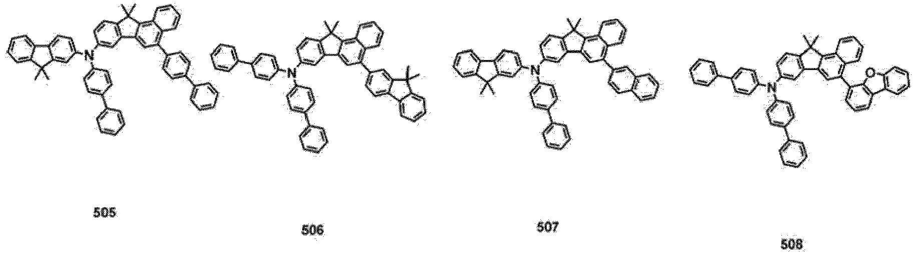

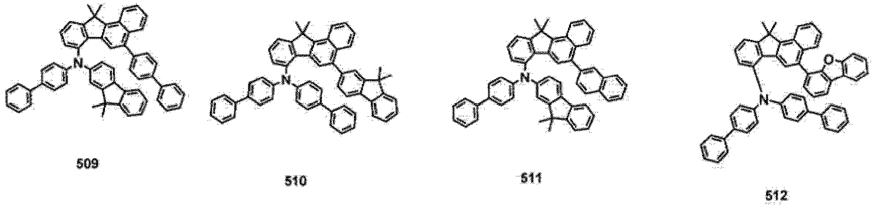

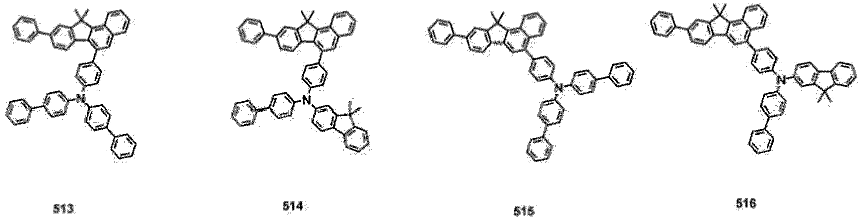

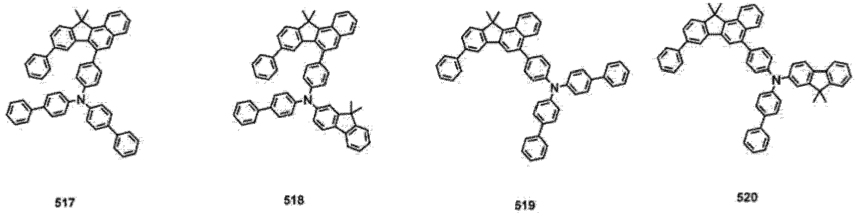

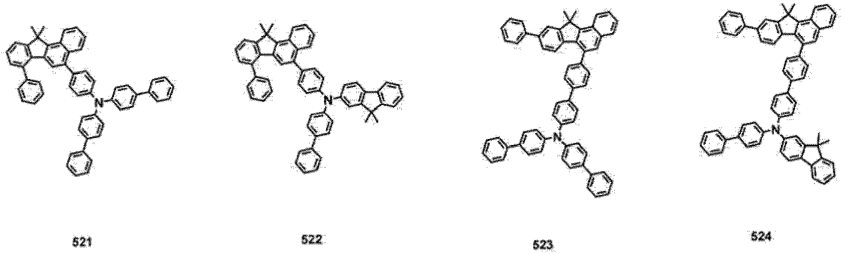

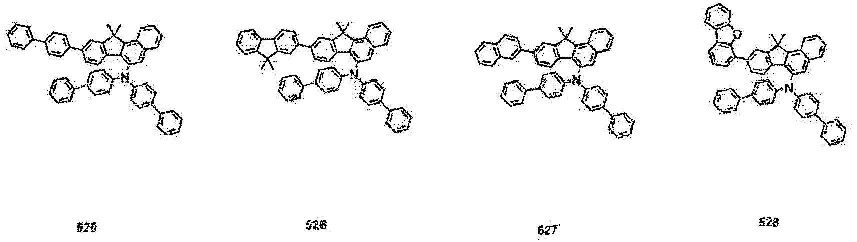

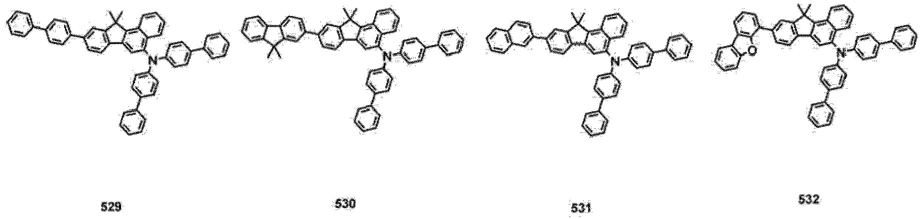

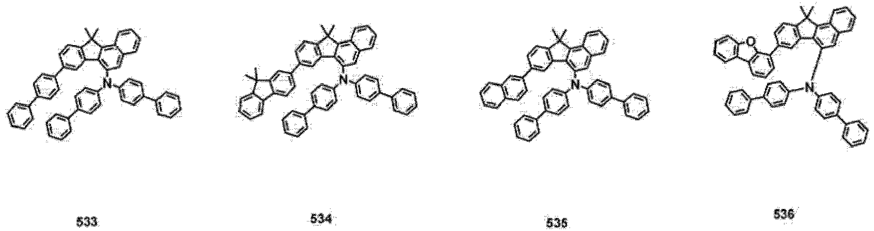

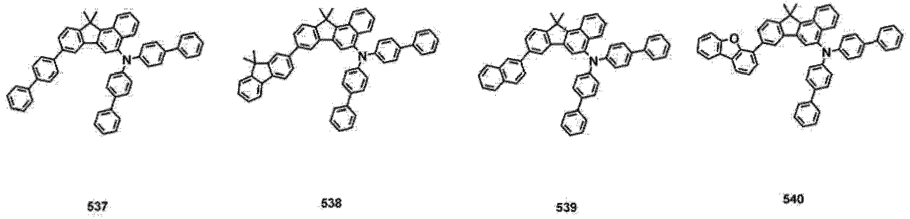

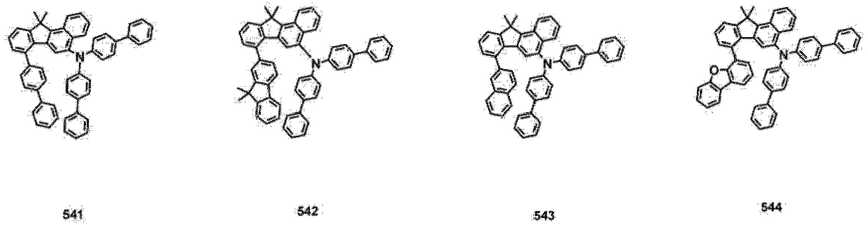

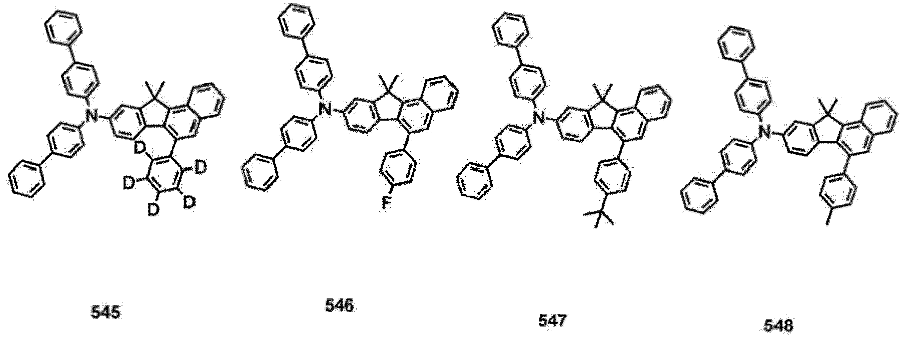

7. An organic light emitting device comprising:
a first electrode;
a second electrode provided to face the first electrode; and
an organic material layer having one or more layers provided between the first electrode and the second electrode,
wherein one or more layers of the organic material layer comprise the heterocyclic compound according to any one of claims 1 to 6.

8. The organic light emitting device of claim 7, wherein the organic material layer comprises a light emitting layer, and the light emitting layer comprises the heterocyclic compound.

9. The organic light emitting device of claim 7, wherein the organic material layer comprises an electron injection layer or an electron transport layer, and the electron transport layer or the electron injection layer comprises the heterocyclic compound.

10. The organic light emitting device of claim 7, wherein the organic material layer comprises an electron blocking layer or a hole blocking layer, and the electron blocking layer or the hole blocking layer comprises the heterocyclic compound.

11. The organic light emitting device of claim 7, wherein the organic material layer comprises a hole transport layer, and the hole transport layer comprises the heterocyclic compound.

12. The organic light emitting device of claim 7, further comprising one or two or more layers selected from the group consisting of a light emitting layer, a hole injection layer, a hole transport layer, an electron injection layer, an electron transport layer, an electron blocking layer and a hole blocking layer.
